# EUROPÄISCHE PATENTSCHRIFT

(11) **EP 2 053 063 B1**
(45) Veröffentlichungstag und Bekanntmachung des Hinweises auf die Patenterteilung: **19.06.2013**
(21) Anmeldenummer: 08171923.9
(22) Anmeldetag: 23.01.2004
(51) Int. Cl.: C07K 16/30, A61K 47/48, C12N 15/13, C12N 15/70, C12N 15/62, C12N 15/79, C12N 1/21, C12N 5/10, C12N 15/86, A01K 67/027, A61K 39/395, A61K 51/10, G01N 33/574, G01N 33/577

(54) **Erkennungsmoleküle zur Behandlung und Detektion von Tumoren**
Recognition molecules for treatment and detection of tumours
Molécules de reconnaissance pour le traitment et la détection de tumeurs

(30) Priorität: 23.01.2003 DE 10303664
(43) Veröffentlichungstag der Anmeldung: 29.04.2009
(62) Teilanmeldung aus: 04704545.5
(73) Patentinhaber: Glycotope GmbH, 13125 Berlin (DE)
(72) Erfinder: Goletz, Steffen, 16548 Glienicke-Nordbahn (DE); Danielczyk, Antje, 16341 Panketal (DE); Stahn, Renate, 13125 Berlin (DE); Karsten, Uwe, 16341 Panketal (DE)
(74) Vertreter: Schiweck, Weinzierl & Koch

(56) Entgegenhaltungen:
- WO-A-01/12217
- WO-A-02/44217
- PRICE M ET AL: "Summary report on the ISOBM TD-4 workshop: Analysis of 56 monoclonal antibodies against the MUC1 mucin" TUMOR BIOLOGY, KARGER, BASEL, CH, Bd. 19, Nr. SUPPL 1, Dezember 1998 (1998-12), Seiten 1-20, XP002112482 ISSN: 1010-4283
- GOLETZ S ET AL: "Binding patterns of 33 TD-4 (MUC1) antibodies towards single-chain fragments and peptides mimicking the conformation of the MUC1 PDTRP epitope" TUMOR BIOLOGY, Bd. 21, Nr. Supplement 1, September 2000 (2000-09), Seite 142, XP008034905 & 28TH MEETING OF THE INTERNATIONAL SOCIETY FOR ONCODEVELOPMENTAL BIOLOGY AND MEDICINE; MUNICH, GERMANY; SEPTEMBER 08-13, 2000 ISSN: 1010-4283
- YANG WEI-PING ET AL: "CDR walking mutagenesis of the affinity maturation of a potent human anti-HIV-1 antibody into the picomolar range", JOURNAL OF MOLECULAR BIOLOGY, vol. 254, no. 3, 1995, pages 392-403, ISSN: 0022-2836
- FURUKAWA KOJI ET AL: "A role of the third complementarity-determining region in the affinity maturation of an antibody", JOURNAL OF BIOLOGICAL CHEMISTRY, vol. 276, no. 29, 20 July 2001 (2001-07-20), pages 27622-27628, ISSN: 0021-9258

### SEQUENZPROTOKOLL

<110> Glycotope GmbH
<120> Erkennungsmoleküle zur Behandlung und Detektion von Tumoren
<130> GLY12912PCTEPD1
<140> PCT/DE2004/000132
   <141> 2004-01-23
<150> DE 103 03 664.4
   <151> 2003-01-23
<160> 69
<170> PatentIn Ver. 2.1
<210> 1
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 1
   Asp Ala Trp Met Asp
    1               5
<210> 2
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 2
   Asn Tyr Trp Met Asn
    1               5
<210> 3
   <211> 19
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 3
   Glu Ile Arg Ser Lys Ala Asn Asn His Ala Thr Tyr Tyr Ala Glu Ser
    1               5                  10                  15
   Val Lys Gly
<210> 4
   <211> 19
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 4
   Glu Ile Arg Leu Lys Ser Asn Asn Tyr Thr Thr His Tyr Ala Glu Ser
   1               5                   10                  15
   Val Lys Gly
<210> 5
   <211> 7
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 5
   Gly Gly Tyr Gly Phe Asp Tyr
   1               5
<210> 6
   <211> 6
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 6
   His Tyr Tyr Phe Asp Tyr
   1               5
<210> 7
   <211> 16
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 7
   Arg Ser Ser Gln Ser Ile Val His Ser Asn Gly Asn Thr Tyr Leu Glu
   1 5   10 15
<210> 8
   <211> 16
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 8
   Arg Ser Ser Lys Ser Leu Leu His Ser Asn Gly Ile Thr Tyr Phe Phe
   1 5 10 15
<210> 9
   <211> 7
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 9
   Lys Val Ser Asn Arg Phe Ser
   1 5
<210> 10
   <211> 7
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 10
   Gln Met Ser Asn Leu Ala Ser
   1 5
<210> 11
   <211> 9
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 11
   Phe Gln Gly Ser His Val Pro Leu Thr
   1 5
<210> 12
   <211> 9
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 12
   Ala Gln Asn Leu Glu Leu Pro Pro Thr
   1 5
<210> 13
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 13
   Asn Tyr Trp Val Asn
   1 5
<210> 14
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 14
   Asn Tyr Trp Ile Asn
   1 5
<210> 15
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 15
   Asn Tyr Trp Tyr Asn
   1 5
<210> 16
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 16
   Asn Tyr Trp Trp Asn
   1 5
<210> 17
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 17
   Asp Ala Trp Ile Asp
   1 5
<210> 18
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 18
   Asp Ala Trp Val Asp
   1 5
<210> 19
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 19
   Asp Ala Trp Tyr Asp
   1 5
<210> 20
   <211> 5
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 20
   Asp Ala Trp Trp Asp
    1               5
<210> 21
   <211> 19
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 21
   Glu Ile Arg Ser Lys Ala Asn Asn Tyr Ala Thr Tyr Tyr Ala Glu Ser
    1               5                  10                  15
   Val Lys Gly
<210> 22
   <211> 19
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 22
   Glu Ile Arg Leu Lys Ser Asn Lys Tyr Thr Thr His Tyr Ala Glu Ser
    1               5                  10                  15
   Val Lys Gly
<210> 23
   <211> 19
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 23
   Glu Ile Arg Leu Lys Ser Asn Ser Tyr Thr Thr His Tyr Ala Glu Ser
   1 5 10 15
   Val Lys Gly
<210> 24
   <211> 16
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 24
   Arg Pro Ser Gln Ser Ile Val His Ser Asn Gly Asn Thr Tyr Leu Glu
   1 5 10 15
<210> 25
   <211> 16
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 25
   Arg Ser Ser Gln Ser Ile Val His Ser Asn Gly Asn Thr Tyr Phe Glu
   1 5 10 15
<210> 26
   <211> 16
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 26
   Arg Pro Ser Gln Ser Ile Val His Ser Asn Gly Asn Thr Tyr Phe Glu
   1 5 10 15
<210> 27
   <211> 16
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 27
   Arg Pro Ser Lys Ser Leu Leu His Ser Asn Gly Ile Thr Tyr Phe Phe
   1 5 10 15
<210> 28
   <211> 16
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 28
   Arg Ser Ser Lys Ser Leu Leu His Ser Asn Gly Ile Thr Tyr Leu Phe
   1 5 10 15
<210> 29
   <211> 16
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 29
   Arg Pro Ser Lys Ser Leu Leu His Ser Asn Gly Ile Thr Tyr Leu Phe
   1 5 10 15
<210> 30
   <211> 9
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 30
   Phe Gln Gly Ser His Pro Pro Leu Thr
   1 5
<210> 31
   <211> 9
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 31
   Ala Gln Asn Leu Glu Pro Pro Pro Thr
   1 5
<210> 32
   <211> 118
   <212> PRT
   <213> Künstliche Sequenz
<220>
   <223> Beschreibung der künstlichen Sequenz: Erkennungsmolekül, das das glykosylierten MUC1-Tumorepitop erkennt
<400> 32
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser
           115
<210> 33
   <211> 117
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 33 Leu Thr Val Ser Ser 115
<210> 34
   <211> 114
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 34
   Asp Ile Val Leu Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Leu Thr Phe Gly Asp Gly Thr Lys Leu Glu Leu Lys
   100 105 110
   Arg Ala
<210> 35
   <211> 114
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 35
   Asp Ile Val Met Thr Gln Ala Ala Phe Ser Asn Pro Val Thr Leu Gly
   1               5                   10                  15
   Thr Ser Ala Ser Ile Ser Cys Arg Ser Ser Lys Ser Leu Leu His Ser
               20                  25                  30
   Asn Gly Ile Thr Tyr Phe Phe Trp Tyr Leu Gln Lys Pro Gly Leu Ser
           35                  40                  45
   Pro Gln Leu Leu Ile Tyr Gln Met Ser Asn Leu Ala Ser Gly Val Pro
       50                  55                  60
   Asp Arg Phe Ser Ser Ser Gly Ser Gly Thr Asp Phe Thr Leu Arg Ile
   65                  70                  75                  80
   Ser Arg Val Glu Ala Glu Asp Val Gly Val Tyr Tyr Cys Ala Gln Asn
                   85                  90                  95
   Leu Glu Leu Pro Pro Thr Phe Gly Gly Gly Thr Lys Leu Glu Ile Lys
               100                 105                 110
   Arg Ala
<210> 36
   <211> 275
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 36
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Ser Ser Gly Gly Gly Gly Ser Gly Gly
           115                 120                 125
   Gly Gly Ser Gly Gly Ser Ala Arg Asp Ile Val Leu Thr Gln Thr Pro
       130                 135                 140
   Leu Ser Leu Pro Val Ser Leu Gly Asp Gln Ala Ser Ile Ser Cys Arg
   145                 150                 155                 160
   Ser Ser Gln Ser Ile Val His Ser Asn Gly Asn Thr Tyr Leu Glu Trp
                   165                 170                 175
   Tyr Leu Gln Lys Pro Gly Gln Ser Pro Lys Leu Leu Ile Tyr Lys Val
               180                 185                 190
   Ser Asn Arg Phe Ser Gly Val Pro Asp Arg Phe Ser Gly Ser Gly Ser
           195                 200                 205
   Gly Thr Asp Phe Thr Leu Lys Ile Ser Arg Val Glu Ala Glu Asp Leu
       210                 215                 220
   Gly Val Tyr Tyr Cys Phe Gln Gly Ser His Val Pro Leu Thr Phe Gly
   225                 230                 235                 240
   Asp Gly Thr Lys Leu Glu Leu Lys Arg Ala Ala Ala His His His His
                   245                 250                 255
   His His Gly Ala Ala Glu Gln Lys Leu Ile Ser Glu Glu Asp Leu Asn
               260                 265                 270
   Gly Ala Ala
           275
<210> 37
   <211> 266
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 37
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Ser Ser Gly Ser Gly Ser Ser Ala Asp
           115                 120                 125
   Ile Val Leu Thr Gln Thr Pro Leu Ser Leu Pro Val Ser Leu Gly Asp
       130                 135                 140
   Gln Ala Ser Ile Ser Cys Arg Ser Ser Gln Ser Ile Val His Ser Asn
   145                 150                 155                 160
   Gly Asn Thr Tyr Leu Glu Trp Tyr Leu Gln Lys Pro Gly Gln Ser Pro
                   165                 170                 175
   Lys Leu Leu Ile Tyr Lys Val Ser Asn Arg Phe Ser Gly Val Pro Asp
               180                 185                 190
   Arg Phe Ser Gly Ser Gly Ser Gly Thr Asp Phe Thr Leu Lys Ile Ser
           195                 200                 205
   Arg Val Glu Ala Glu Asp Leu Gly Val Tyr Tyr Cys Phe Gln Gly Ser
       210                 215                 220
   His Val Pro Leu Thr Phe Gly Asp Gly Thr Lys Leu Glu Leu Lys Arg
   225                 230                 235                 240
   Ala Ala Ala His His His His His His Gly Ala Ala Glu Gln Lys Leu
                   245                 250                 255
   Ile Ser Glu Glu Asp Leu Asn Gly Ala Ala
               260                 265
<210> 38
   <211> 265
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 38
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Ser Ser Gly Gly Ser Ser Ala Asp Ile
           115                 120                 125
   Val Leu Thr Gln Thr Pro Leu Ser Leu Pro Val Ser Leu Gly Asp Gln
       130                 135                 140
   Ala Ser Ile Ser Cys Arg Ser Ser Gln Ser Ile Val His Ser Asn Gly
   145                 150                 155                 160
   Asn Thr Tyr Leu Glu Trp Tyr Leu Gln Lys Pro Gly Gln Ser Pro Lys
                   165                 170                 175
   Leu Leu Ile Tyr Lys Val Ser Asn Arg Phe Ser Gly Val Pro Asp Arg
               180                 185                 190
   Phe Ser Gly Ser Gly Ser Gly Thr Asp Phe Thr Leu Lys Ile Ser Arg
           195                 200                 205
   Val Glu Ala Glu Asp Leu Gly Val Tyr Tyr Cys Phe Gln Gly Ser His
       210                 215                 220
   Val Pro Leu Thr Phe Gly Asp Gly Thr Lys Leu Glu Leu Lys Arg Ala
   225                 230                 235                 240
   Ala Ala His His His His His His Gly Ala Ala Glu Gln Lys Leu Ile
                   245                 250                 255
   Ser Glu Glu Asp Leu Asn Gly Ala Ala
               260                 265
<210> 39
   <211> 264
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 39
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Ser Ser Gly Ser Ser Ala Asp Ile Val
           115                 120                 125
   Leu Thr Gln Thr Pro Leu Ser Leu Pro Val Ser Leu Gly Asp Gln Ala
       130                 135                 140
   Ser Ile Ser Cys Arg Ser Ser Gln Ser Ile Val His Ser Asn Gly Asn
   145                 150                 155                 160
   Thr Tyr Leu Glu Trp Tyr Leu Gln Lys Pro Gly Gln Ser Pro Lys Leu
                   165                 170                 175
   Leu Ile Tyr Lys Val Ser Asn Arg Phe Ser Gly Val Pro Asp Arg Phe
               180                 185                 190
   Ser Gly Ser Gly Ser Gly Thr Asp Phe Thr Leu Lys Ile Ser Arg Val
           195                 200                 205
   Glu Ala Glu Asp Leu Gly Val Tyr Tyr Cys Phe Gln Gly Ser His Val
       210                 215                 220
   Pro Leu Thr Phe Gly Asp Gly Thr Lys Leu Glu Leu Lys Arg Ala Ala
   225                 230                 235                 240
   Ala His His His His His His Gly Ala Ala Glu Gln Lys Leu Ile Ser
                   245                 250                 255
   Glu Glu Asp Leu Asn Gly Ala Ala
               260
<210> 40
   <211> 263
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 40
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Ser Ser Ser Ser Ala Asp Ile Val Leu
           115                 120                 125
   Thr Gln Thr Pro Leu Ser Leu Pro Val Ser Leu Gly Asp Gln Ala Ser
       130                 135                 140
   Ile Ser Cys Arg Ser Ser Gln Ser Ile Val His Ser Asn Gly Asn Thr
   145                 150                 155                 160
   Tyr Leu Glu Trp Tyr Leu Gln Lys Pro Gly Gln Ser Pro Lys Leu Leu
                   165                 170                 175
   Ile Tyr Lys Val Ser Asn Arg Phe Ser Gly Val Pro Asp Arg Phe Ser
               180                 185                 190
   Gly Ser Gly Ser Gly Thr Asp Phe Thr Leu Lys Ile Ser Arg Val Glu
   195 200 205
   Ala Glu Asp Leu Gly Val Tyr Tyr Cys Phe Gln Gly Ser His Val Pro
   210 215 220
   Leu Thr Phe Gly Asp Gly Thr Lys Leu Glu Leu Lys Arg Ala Ala Ala
   225 230 235 240
   His His His His His His Gly Ala Ala Glu Gln Lys Leu Ile Ser Glu
   245 250 255
   Glu Asp Leu Asn Gly Ala Ala
   260
<210> 41
   <211> 262
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 41
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Ser Ser Ser Ala Asp Ile Val Leu Thr
           115                 120                 125
   Gln Thr Pro Leu Ser Leu Pro Val Ser Leu Gly Asp Gln Ala Ser Ile
       130                 135                 140
   Ser Cys Arg Ser Ser Gln Ser Ile Val His Ser Asn Gly Asn Thr Tyr
   145                 150                 155                 160
   Leu Glu Trp Tyr Leu Gln Lys Pro Gly Gln Ser Pro Lys Leu Leu Ile
                   165                 170                 175
   Tyr Lys Val Ser Asn Arg Phe Ser Gly Val Pro Asp Arg Phe Ser Gly
               180                 185                 190
   Ser Gly Ser Gly Thr Asp Phe Thr Leu Lys Ile Ser Arg Val Glu Ala
           195                 200                 205
   Glu Asp Leu Gly Val Tyr Tyr Cys Phe Gln Gly Ser His Val Pro Leu
       210                 215                 220
   Thr Phe Gly Asp Gly Thr Lys Leu Glu Leu Lys Arg Ala Ala Ala His
   225                 230                 235                 240
   His His His His His Gly Ala Ala Glu Gln Lys Leu Ile Ser Glu Glu
                   245                 250                 255
   Asp Leu Asn Gly Ala Ala
               260
<210> 42
   <211> 261
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 42
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Ser Ser Ala Asp Ile Val Leu Thr Gln
           115                 120                 125
   Thr Pro Leu Ser Leu Pro Val Ser Leu Gly Asp Gln Ala Ser Ile Ser
       130                 135                 140
   Cys Arg Ser Ser Gln Ser Ile Val His Ser Asn Gly Asn Thr Tyr Leu
   145                 150                 155                 160
   Glu Trp Tyr Leu Gln Lys Pro Gly Gln Ser Pro Lys Leu Leu Ile Tyr
                   165                 170                 175
   Lys Val Ser Asn Arg Phe Ser Gly Val Pro Asp Arg Phe Ser Gly Ser
               180                 185                 190
   Gly Ser Gly Thr Asp Phe Thr Leu Lys Ile Ser Arg Val Glu Ala Glu
           195                 200                 205
   Asp Leu Gly Val Tyr Tyr Cys Phe Gln Gly Ser His Val Pro Leu Thr
       210                 215                 220
   Phe Gly Asp Gly Thr Lys Leu Glu Leu Lys Arg Ala Ala Ala His His
   225                 230                 235                 240
   His His His His Gly Ala Ala Glu Gln Lys Leu Ile Ser Glu Glu Asp
                   245                 250                 255
   Leu Asn Gly Ala Ala
               260
<210> 43
   <211> 260
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 43
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Ser Ala Asp Ile Val Leu Thr Gln Thr
           115                 120                 125
   Pro Leu Ser Leu Pro Val Ser Leu Gly Asp Gln Ala Ser Ile Ser Cys
       130                 135                 140
   Arg Ser Ser Gln Ser Ile Val His Ser Asn Gly Asn Thr Tyr Leu Glu
   145                 150                 155                 160
   Trp Tyr Leu Gln Lys Pro Gly Gln Ser Pro Lys Leu Leu Ile Tyr Lys
                   165                 170                 175
   Val Ser Asn Arg Phe Ser Gly Val Pro Asp Arg Phe Ser Gly Ser Gly
               180                 185                 190
   Ser Gly Thr Asp Phe Thr Leu Lys Ile Ser Arg Val Glu Ala Glu Asp
           195                 200                 205
   Leu Gly Val Tyr Tyr Cys Phe Gln Gly Ser His Val Pro Leu Thr Phe
       210                 215                 220
   Gly Asp Gly Thr Lys Leu Glu Leu Lys Arg Ala Ala Ala His His His
   225                 230                 235                 240
   His His His Gly Ala Ala Glu Gln Lys Leu Ile Ser Glu Glu Asp Leu
                   245                 250                 255
   Asn Gly Ala Ala
               260
<210> 44
   <211> 259
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 44
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Ala Asp Ile Val Leu Thr Gln Thr Pro
           115                 120                 125
   Leu Ser Leu Pro Val Ser Leu Gly Asp Gln Ala Ser Ile Ser Cys Arg
       130                 135                 140
   Ser Ser Gln Ser Ile Val His Ser Asn Gly Asn Thr Tyr Leu Glu Trp
   145                 150                 155                 160
   Tyr Leu Gln Lys Pro Gly Gln Ser Pro Lys Leu Leu Ile Tyr Lys Val
                   165                 170                 175
   Ser Asn Arg Phe Ser Gly Val Pro Asp Arg Phe Ser Gly Ser Gly Ser
               180                 185                 190
   Gly Thr Asp Phe Thr Leu Lys Ile Ser Arg Val Glu Ala Glu Asp Leu
           195                 200                 205
   Gly Val Tyr Tyr Cys Phe Gln Gly Ser His Val Pro Leu Thr Phe Gly
       210                 215                 220
   Asp Gly Thr Lys Leu Glu Leu Lys Arg Ala Ala Ala His His His His
   225                 230                 235                 240
   His His Gly Ala Ala Glu Gln Lys Leu Ile Ser Glu Glu Asp Leu Asn
                   245                 250                 255
   Gly Ala Ala
<210> 45
   <211> 255
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 45
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Ala Asp Ile Val Leu Thr Gln Thr Pro Leu
           115                 120                 125
   Ser Leu Pro Val Ser Leu Gly Asp Gln Ala Ser Ile Ser Cys Arg Ser
       130                 135                 140
   Ser Gln Ser Ile Val His Ser Asn Gly Asn Thr Tyr Leu Glu Trp Tyr
   145                 150                 155                 160
   Leu Gln Lys Pro Gly Gln Ser Pro Lys Leu Leu Ile Tyr Lys Val Ser
                   165                 170                 175
   Asn Arg Phe Ser Gly Val Pro Asp Arg Phe Ser Gly Ser Gly Ser Gly
               180                 185                 190
   Thr Asp Phe Thr Leu Lys Ile Ser Arg Val Glu Ala Glu Asp Leu Gly
           195                 200                 205
   Val Tyr Tyr Cys Phe Gln Gly Ser His Val Pro Leu Thr Phe Gly Asp
       210                 215                 220
   Gly Thr Lys Leu Glu Leu Lys Arg Ala Ala Ala His His His His His
   225                 230                 235                 240
   His Gly Ala Ala Glu Gln Lys Leu Ile Ser Glu Glu Val His Gln
                   245                 250                 255
<210> 46
   <211> 257
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 46
   Glu Val Lys Leu Val Glu Ser Gly Gly Gly Leu Val Gln Pro Gly Gly
    1               5                  10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ser Asp Ile Val Leu Thr Gln Thr Pro Leu Ser
           115                 120                 125
   Leu Pro Val Ser Leu Gly Asp Gln Ala Ser Ile Ser Cys Arg Ser Ser
       130                 135                 140
   Gln Ser Ile Val His Ser Asn Gly Asn Thr Tyr Leu Glu Trp Tyr Leu
   145                 150                 155                 160
   Gln Lys Pro Gly Gln Ser Pro Lys Leu Leu Ile Tyr Lys Val Ser Asn
                   165                 170                 175
   Arg Phe Ser Gly Val Pro Asp Arg Phe Ser Gly Ser Gly Ser Gly Thr
               180                 185                 190
   Asp Phe Thr Leu Lys Ile Ser Arg Val Glu Ala Glu Asp Leu Gly Val
           195                 200                 205
   Tyr Tyr Cys Phe Gln Gly Ser His Val Pro Leu Thr Phe Gly Asp Gly
       210                 215                 220
   Thr Lys Leu Glu Leu Lys Arg Ala Ala Ala His His His His His His
   225                 230                 235                 240
   Gly Ala Ala Glu Gln Lys Leu Ile Ser Glu Glu Asp Leu Asn Gly Ala
                   245                 250                 255
   Ala
<210> 47
   <211> 256
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 47
   Glu Val Lys Leu Val Glu Ser Gly Gly Gly Leu Val Gln Pro Gly Gly
    1               5                  10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Asp Ile Val Leu Thr Gln Thr Pro Leu Ser Leu
           115                 120                 125
   Pro Val Ser Leu Gly Asp Gln Ala Ser Ile Ser Cys Arg Ser Ser Gln
       130                 135                 140
   Ser Ile Val His Ser Asn Gly Asn Thr Tyr Leu Glu Trp Tyr Leu Gln
   145                 150                 155                 160
   Lys Pro Gly Gln Ser Pro Lys Leu Leu Ile Tyr Lys Val Ser Asn Arg
                   165                 170                 175
   Phe Ser Gly Val Pro Asp Arg Phe Ser Gly Ser Gly Ser Gly Thr Asp
               180                 185                 190
   Phe Thr Leu Lys Ile Ser Arg Val Glu Ala Glu Asp Leu Gly Val Tyr
           195                 200                 205
   Tyr Cys Phe Gln Gly Ser His Val Pro Leu Thr Phe Gly Asp Gly Thr
       210                 215                 220
   Lys Leu Glu Leu Lys Arg Ala Ala Ala His His His His His His Gly
   225                 230                 235                 240
   Ala Ala Glu Gln Lys Leu Ile Ser Glu Glu Asp Leu Asn Gly Ala Ala
                   245                 250                 255
<210> 48
   <211> 274
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 48
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Ser Ser Gly Gly Gly Gly Ser Gly Gly Gly
           115                 120                 125
   Gly Ser Gly Gly Ser Ala Arg Asp Ile Val Met Thr Gln Ala Ala Phe
       130                 135                 140
   Ser Asn Pro Val Thr Leu Gly Thr Ser Ala Ser Ile Ser Cys Arg Ser
   145                 150                 155                 160
   Ser Lys Ser Leu Leu His Ser Asn Gly Ile Thr Tyr Phe Phe Trp Tyr
                   165                 170                 175
   Leu Gln Lys Pro Gly Leu Ser Pro Gln Leu Leu Ile Tyr Gln Met Ser
               180                 185                 190
   Asn Leu Ala Ser Gly Val Pro Asp Arg Phe Ser Ser Ser Gly Ser Gly
           195                 200                 205
   Thr Asp Phe Thr Leu Arg Ile Ser Arg Val Glu Ala Glu Asp Val Gly
       210                 215                 220
   Val Tyr Tyr Cys Ala Gln Asn Leu Glu Leu Pro Pro Thr Phe Gly Gly
   225                 230                 235                 240
   Gly Thr Lys Leu Glu Ile Lys Arg Ala Ala Ala His His His His His
                   245                 250                 255
   His Gly Ala Ala Glu Gln Lys Leu Ile Ser Glu Glu Asp Leu Asn Gly
               260                 265                 270
   Ala Ala
<210> 49
   <211> 265
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 49
   Glu Val Lys Leu Val Glu Ser Gly Gly Gly Leu Val Gln Pro Gly Gly
    1               5                  10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Ser Ser Gly Ser Gly Ser Ser Ala Asp Ile
           115                 120                 125
   Val Met Thr Gln Ala Ala Phe Ser Asn Pro Val Thr Leu Gly Thr Ser
       130                 135                 140
   Ala Ser Ile Ser Cys Arg Ser Ser Lys Ser Leu Leu His Ser Asn Gly
   145                 150                 155                 160
   Ile Thr Tyr Phe Phe Trp Tyr Leu Gln Lys Pro Gly Leu Ser Pro Gln
                   165                 170                 175
   Leu Leu Ile Tyr Gln Met Ser Asn Leu Ala Ser Gly Val Pro Asp Arg
               180                 185                 190
   Phe Ser Ser Ser Gly Ser Gly Thr Asp Phe Thr Leu Arg Ile Ser Arg
           195                 200                 205
   Val Glu Ala Glu Asp Val Gly Val Tyr Tyr Cys Ala Gln Asn Leu Glu
       210                 215                 220
   Leu Pro Pro Thr Phe Gly Gly Gly Thr Lys Leu Glu Ile Lys Arg Ala
   225                 230                 235                 240
   Ala Ala His His His His His His Gly Ala Ala Glu Gln Lys Leu Ile
                   245                 250                 255
   Ser Glu Glu Asp Leu Asn Gly Ala Ala
               260                 265
<210> 50
   <211> 264
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 50
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Ser Ser Gly Gly Ser Ser Ala Asp Ile Val
           115                 120                 125
   Met Thr Gln Ala Ala Phe Ser Asn Pro Val Thr Leu Gly Thr Ser Ala
       130                 135                 140
   Ser Ile Ser Cys Arg Ser Ser Lys Ser Leu Leu His Ser Asn Gly Ile
   145                 150                 155                 160
   Thr Tyr Phe Phe Trp Tyr Leu Gln Lys Pro Gly Leu Ser Pro Gln Leu
                   165                 170                 175
   Leu Ile Tyr Gln Met Ser Asn Leu Ala Ser Gly Val Pro Asp Arg Phe
               180                 185                 190
   Ser Ser Ser Gly Ser Gly Thr Asp Phe Thr Leu Arg Ile Ser Arg Val
   195 200 205
   Glu Ala Glu Asp Val Gly Val Tyr Tyr Cys Ala Gln Asn Leu Glu Leu
   210 215 220
   Pro Pro Thr Phe Gly Gly Gly Thr Lys Leu Glu Ile Lys Arg Ala Ala
   225 230 235 240
   Ala His His His His His His Gly Ala Ala Glu Gln Lys Leu Ile Ser
   245 250 255
   Glu Glu Asp Leu Asn Gly Ala Ala
   260
<210> 51
   <211> 263
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 51
   Glu Val Lys Leu Val Glu Ser Gly Gly Gly Leu Val Gln Pro Gly Gly
    1               5                  10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Ser Ser Gly Ser Ser Ala Asp Ile Val Met
           115                 120                 125
   Thr Gln Ala Ala Phe Ser Asn Pro Val Thr Leu Gly Thr Ser Ala Ser
       130                 135                 140
   Ile Ser Cys Arg Ser Ser Lys Ser Leu Leu His Ser Asn Gly Ile Thr
   145                 150                 155                 160
   Tyr Phe Phe Trp Tyr Leu Gln Lys Pro Gly Leu Ser Pro Gln Leu Leu
                   165                 170                 175
   Ile Tyr Gln Met Ser Asn Leu Ala Ser Gly Val Pro Asp Arg Phe Ser
               180                 185                 190
   Ser Ser Gly Ser Gly Thr Asp Phe Thr Leu Arg Ile Ser Arg Val Glu
           195                 200                 205
   Ala Glu Asp Val Gly Val Tyr Tyr Cys Ala Gln Asn Leu Glu Leu Pro
       210                 215                 220
   Pro Thr Phe Gly Gly Gly Thr Lys Leu Glu Ile Lys Arg Ala Ala Ala
   225                 230                 235                 240
   His His His His His His Gly Ala Ala Glu Gln Lys Leu Ile Ser Glu
                   245                 250                 255
   Glu Asp Leu Asn Gly Ala Ala
               260
<210> 52
   <211> 262
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 52
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Ser Ser Ser Ser Ala Asp Ile Val Met Thr
           115                 120                 125
   Gln Ala Ala Phe Ser Asn Pro Val Thr Leu Gly Thr Ser Ala Ser Ile
       130                 135                 140
   Ser Cys Arg Ser Ser Lys Ser Leu Leu His Ser Asn Gly Ile Thr Tyr
   145                 150                 155                 160
   Phe Phe Trp Tyr Leu Gln Lys Pro Gly Leu Ser Pro Gln Leu Leu Ile
                   165                 170                 175
   Tyr Gln Met Ser Asn Leu Ala Ser Gly Val Pro Asp Arg Phe Ser Ser
               180                 185                 190
   Ser Gly Ser Gly Thr Asp Phe Thr Leu Arg Ile Ser Arg Val Glu Ala
           195                 200                 205
   Glu Asp Val Gly Val Tyr Tyr Cys Ala Gln Asn Leu Glu Leu Pro Pro
       210                 215                 220
   Thr Phe Gly Gly Gly Thr Lys Leu Glu Ile Lys Arg Ala Ala Ala His
   225                 230                 235                 240
   His His His His His Gly Ala Ala Glu Gln Lys Leu Ile Ser Glu Glu
                   245                 250                 255
   Asp Leu Asn Gly Ala Ala
               260
<210> 53
   <211> 261
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 53
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Ser Ser Ser Ala Asp Ile Val Met Thr Gln
           115                 120                 125
   Ala Ala Phe Ser Asn Pro Val Thr Leu Gly Thr Ser Ala Ser Ile Ser
       130                 135                 140
   Cys Arg Ser Ser Lys Ser Leu Leu His Ser Asn Gly Ile Thr Tyr Phe
   145                 150                 155                 160
   Phe Trp Tyr Leu Gln Lys Pro Gly Leu Ser Pro Gln Leu Leu Ile Tyr
                   165                 170                 175
   Gln Met Ser Asn Leu Ala Ser Gly Val Pro Asp Arg Phe Ser Ser Ser
               180                 185                 190
   Gly Ser Gly Thr Asp Phe Thr Leu Arg Ile Ser Arg Val Glu Ala Glu
           195                 200                 205
   Asp Val Gly Val Tyr Tyr Cys Ala Gln Asn Leu Glu Leu Pro Pro Thr
       210                 215                 220
   Phe Gly Gly Gly Thr Lys Leu Glu Ile Lys Arg Ala Ala Ala His His
   225                 230                 235                 240
   His His His His Gly Ala Ala Glu Gln Lys Leu Ile Ser Glu Glu Asp
                   245                 250                 255
   Leu Asn Gly Ala Ala
               260
<210> 54
   <211> 260
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 54
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Ser Ser Ala Asp Ile Val Met Thr Gln Ala
           115                 120                 125
   Ala Phe Ser Asn Pro Val Thr Leu Gly Thr Ser Ala Ser Ile Ser Cys
       130                 135                 140
   Arg Ser Ser Lys Ser Leu Leu His Ser Asn Gly Ile Thr Tyr Phe Phe
   145                 150                 155                 160
   Trp Tyr Leu Gln Lys Pro Gly Leu Ser Pro Gln Leu Leu Ile Tyr Gln
                   165                 170                 175
   Met Ser Asn Leu Ala Ser Gly Val Pro Asp Arg Phe Ser Ser Ser Gly
               180                 185                 190
   Ser Gly Thr Asp Phe Thr Leu Arg Ile Ser Arg Val Glu Ala Glu Asp
           195                 200                 205
   Val Gly Val Tyr Tyr Cys Ala Gln Asn Leu Glu Leu Pro Pro Thr Phe
       210                 215                 220
   Gly Gly Gly Thr Lys Leu Glu Ile Lys Arg Ala Ala Ala His His His
   225                 230                 235                 240
   His His His Gly Ala Ala Glu Gln Lys Leu Ile Ser Glu Glu Asp Leu
                   245                 250                 255
   Asn Gly Ala Ala
               260
<210> 55
   <211> 259
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 55
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Ser Ala Asp Ile Val Met Thr Gln Ala Ala
           115                 120                 125
   Phe Ser Asn Pro Val Thr Leu Gly Thr Ser Ala Ser Ile Ser Cys Arg
       130                 135                 140
   Ser Ser Lys Ser Leu Leu His Ser Asn Gly Ile Thr Tyr Phe Phe Trp
   145                 150                 155                 160
   Tyr Leu Gln Lys Pro Gly Leu Ser Pro Gln Leu Leu Ile Tyr Gln Met
                   165                 170                 175
   Ser Asn Leu Ala Ser Gly Val Pro Asp Arg Phe Ser Ser Ser Gly Ser
               180                 185                 190
   Gly Thr Asp Phe Thr Leu Arg Ile Ser Arg Val Glu Ala Glu Asp Val
           195                 200                 205
   Gly Val Tyr Tyr Cys Ala Gln Asn Leu Glu Leu Pro Pro Thr Phe Gly
       210                 215                 220
   Gly Gly Thr Lys Leu Glu Ile Lys Arg Ala Ala Ala His His His His
   225                 230                 235                 240
   His His Gly Ala Ala Glu Gln Lys Leu Ile Ser Glu Glu Asp Leu Asn
                   245                 250                 255
   Gly Ala Ala
<210> 56
   <211> 258
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 56
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Ala Asp Ile Val Met Thr Gln Ala Ala Phe
           115                 120                 125
   Ser Asn Pro Val Thr Leu Gly Thr Ser Ala Ser Ile Ser Cys Arg Ser
       130                 135                 140
   Ser Lys Ser Leu Leu His Ser Asn Gly Ile Thr Tyr Phe Phe Trp Tyr
   145                 150                 155                 160
   Leu Gln Lys Pro Gly Leu Ser Pro Gln Leu Leu Ile Tyr Gln Met Ser
                   165                 170                 175
   Asn Leu Ala Ser Gly Val Pro Asp Arg Phe Ser Ser Ser Gly Ser Gly
               180                 185                 190
   Thr Asp Phe Thr Leu Arg Ile Ser Arg Val Glu Ala Glu Asp Val Gly
           195                 200                 205
   Val Tyr Tyr Cys Ala Gln Asn Leu Glu Leu Pro Pro Thr Phe Gly Gly
       210                 215                 220
   Gly Thr Lys Leu Glu Ile Lys Arg Ala Ala Ala His His His His His
   225                 230                 235                 240
   His Gly Ala Ala Glu Gln Lys Leu Ile Ser Glu Glu Asp Leu Asn Gly
                   245                 250                 255
   Ala Ala
<210> 57
   <211> 257
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 57
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Ala Asp Ile Val Met Thr Gln Ala Ala Phe Ser
           115                 120                 125
   Asn Pro Val Thr Leu Gly Thr Ser Ala Ser Ile Ser Cys Arg Ser Ser
       130                 135                 140
   Lys Ser Leu Leu His Ser Asn Gly Ile Thr Tyr Phe Phe Trp Tyr Leu
   145                 150                 155                 160
   Gln Lys Pro Gly Leu Ser Pro Gln Leu Leu Ile Tyr Gln Met Ser Asn
                   165                 170                 175
   Leu Ala Ser Gly Val Pro Asp Arg Phe Ser Ser Ser Gly Ser Gly Thr
               180                 185                 190
   Asp Phe Thr Leu Arg Ile Ser Arg Val Glu Ala Glu Asp Val Gly Val
           195                 200                 205
   Tyr Tyr Cys Ala Gln Asn Leu Glu Leu Pro Pro Thr Phe Gly Gly Gly
       210                 215                 220
   Thr Lys Leu Glu Ile Lys Arg Ala Ala Ala His His His His His His
   225                 230                 235                 240
   Gly Ala Ala Glu Gln Lys Leu Ile Ser Glu Glu Asp Leu Asn Gly Ala
                   245                 250                 255
   Ala
<210> 58
   <211> 256
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 58
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ser Asp Ile Val Met Thr Gln Ala Ala Phe Ser Asn
           115                 120                 125
   Pro Val Thr Leu Gly Thr Ser Ala Ser Ile Ser Cys Arg Ser Ser Lys
       130                 135                 140
   Ser Leu Leu His Ser Asn Gly Ile Thr Tyr Phe Phe Trp Tyr Leu Gln
   145                 150                 155                 160
   Lys Pro Gly Leu Ser Pro Gln Leu Leu Ile Tyr Gln Met Ser Asn Leu
                   165                 170                 175
   Ala Ser Gly Val Pro Asp Arg Phe Ser Ser Ser Gly Ser Gly Thr Asp
               180                 185                 190
   Phe Thr Leu Arg Ile Ser Arg Val Glu Ala Glu Asp Val Gly Val Tyr
           195                 200                 205
   Tyr Cys Ala Gln Asn Leu Glu Leu Pro Pro Thr Phe Gly Gly Gly Thr
       210                 215                 220
   Lys Leu Glu Ile Lys Arg Ala Ala Ala His His His His His His Gly
   225                 230                 235                 240
   Ala Ala Glu Gln Lys Leu Ile Ser Glu Glu Asp Leu Asn Gly Ala Ala
                   245                 250                 255
<210> 59
   <211> 255
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 59
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Asp Ile Val Met Thr Gln Ala Ala Phe Ser Asn Pro
           115                 120                 125
   Val Thr Leu Gly Thr Ser Ala Ser Ile Ser Cys Arg Ser Ser Lys Ser
       130                 135                 140
   Leu Leu His Ser Asn Gly Ile Thr Tyr Phe Phe Trp Tyr Leu Gln Lys
   145                 150                 155                 160
   Pro Gly Leu Ser Pro Gln Leu Leu Ile Tyr Gln Met Ser Asn Leu Ala
                   165                 170                 175
   Ser Gly Val Pro Asp Arg Phe Ser Ser Ser Gly Ser Gly Thr Asp Phe
               180                 185                 190
   Thr Leu Arg Ile Ser Arg Val Glu Ala Glu Asp Val Gly Val Tyr Tyr
           195                 200                 205
   Cys Ala Gln Asn Leu Glu Leu Pro Pro Thr Phe Gly Gly Gly Thr Lys
       210                 215                 220
   Leu Glu Ile Lys Arg Ala Ala Ala His His His His His His Gly Ala
   225                 230                 235                 240
   Ala Glu Gln Lys Leu Ile Ser Glu Glu Asp Leu Asn Gly Ala Ala
                   245                 250                 255
<210> 60
   <211> 219
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 60
   Asp Ile Val Leu Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Leu Thr Phe Gly Asp Gly Thr Lys Leu Glu Leu Lys
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Lys Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Asp Ser
                   165                 170                 175
   Thr Tyr Ser Met Ser Ser Thr Leu Thr Leu Thr Lys Asp Glu Tyr Glu
               180                 185                 190
   Arg His Asn Ser Tyr Thr Cys Glu Ala Thr His Lys Thr Ser Thr Ser
           195                 200                 205
   Pro Ile Val Lys Ser Phe Asn Arg Asn Glu Cys
       210                 215
<210> 61
   <211> 219
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 61
   Asp Ile Val Met Thr Gln Ala Ala Phe Ser Asn Pro Val Thr Leu Gly
   1               5                   10                  15
   Thr Ser Ala Ser Ile Ser Cys Arg Ser Ser Lys Ser Leu Leu His Ser
               20                  25                  30
   Asn Gly Ile Thr Tyr Phe Phe Trp Tyr Leu Gln Lys Pro Gly Leu Ser
           35                  40                  45
   Pro Gln Leu Leu Ile Tyr Gln Met Ser Asn Leu Ala Ser Gly Val Pro
       50                  55                  60
   Asp Arg Phe Ser Ser Ser Gly Ser Gly Thr Asp Phe Thr Leu Arg Ile
   65                  70                  75                  80
   Ser Arg Val Glu Ala Glu Asp Val Gly Val Tyr Tyr Cys Ala Gln Asn
                   85                  90                  95
   Leu Glu Leu Pro Pro Thr Phe Gly Gly Gly Thr Lys Leu Glu Ile Lys
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Lys Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Asp Ser
                   165                 170                 175
   Thr Tyr Ser Met Ser Ser Thr Leu Thr Leu Thr Lys Asp Glu Tyr Glu
               180                 185                 190
   Arg His Asn Ser Tyr Thr Cys Glu Ala Thr His Lys Thr Ser Thr Ser
           195                 200                 205
   Pro Ile Val Lys Ser Phe Asn Arg Asn Glu Cys
       210                 215
<210> 62
   <211> 441
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 62
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Ala Lys Thr Thr Pro Pro Ser Val Tyr Pro Leu
           115                 120                 125
   Ala Pro Gly Ser Ala Ala Gln Thr Asn Ser Met Val Thr Leu Gly Cys
       130                 135                 140
   Leu Val Lys Gly Tyr Phe Pro Glu Pro Val Thr Val Thr Trp Asn Ser
   145                 150                 155                 160
   Gly Ser Leu Ser Ser Gly Val His Thr Phe Pro Ala Val Leu Glu Ser
                   165                 170                 175
   Asp Leu Tyr Thr Leu Ser Ser Ser Val Thr Val Pro Ser Ser Pro Arg
               180                 185                 190
   Pro Ser Glu Thr Val Thr Cys Asn Val Ala His Pro Ala Ser Ser Thr
           195                 200                 205
   Lys Val Asp Lys Lys Ile Val Pro Arg Asp Cys Gly Cys Lys Pro Cys
       210                 215                 220
   Ile Cys Thr Val Pro Glu Val Ser Ser Val Phe Ile Phe Pro Pro Lys
   225                 230                 235                 240
   Pro Lys Asp Val Leu Thr Ile Thr Leu Thr Pro Lys Val Thr Cys Val
                   245                 250                 255
   Val Val Asp Ile Ser Lys Asp Asp Pro Glu Val Gln Phe Ser Trp Phe
               260                 265                 270
   Val Asp Asp Val Glu Val His Thr Ala Gln Thr Gln Pro Arg Glu Glu
           275                 280                 285
   Gln Phe Asn Ser Thr Phe Arg Ser Val Ser Glu Leu Pro Ile Met His
       290                 295                 300
   Gln Asp Trp Leu Asn Gly Lys Glu Phe Lys Cys Arg Val Asn Ser Ala
   305                 310                 315                 320
   Ala Phe Pro Ala Pro Ile Glu Lys Thr Ile Ser Lys Thr Lys Gly Arg
                   325                 330                 335
   Pro Lys Ala Pro Gln Val Tyr Thr Ile Pro Pro Pro Lys Glu Gln Met
               340                 345                 350
   Ala Lys Asp Lys Val Ser Leu Thr Cys Met Ile Thr Asp Phe Phe Pro
           355                 360                 365
   Glu Asp Ile Thr Val Glu Trp Gln Trp Asn Gly Gln Pro Ala Glu Asn
       370                 375                 380
   Tyr Lys Asn Thr Gln Pro Ile Met Asn Thr Asn Gly Ser Tyr Phe Val
   385                 390                 395                 400
   Tyr Ser Lys Leu Asn Val Gln Lys Ser Asn Trp Glu Ala Gly Asn Thr
                   405                 410                 415
   Phe Thr Cys Ser Val Leu His Glu Gly Leu His Asn His His Thr Glu
               420                 425                 430
   Lys Ser Leu Ser His Ser Pro Gly Lys
           435                 440
<210> 63
   <211> 440
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 63
   Glu Val Lys Leu Val Glu Ser Gly Gly Gly Leu Val Gln Pro Gly Gly
    1               5                  10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Ala Lys Thr Thr Pro Pro Ser Val Tyr Pro Leu Ala
           115                 120                 125
   Pro Gly Ser Ala Ala Gln Thr Asn Ser Met Val Thr Leu Gly Cys Leu
   130 135 140
   Val Lys Gly Tyr Phe Pro Glu Pro Val Thr Val Thr Trp Asn Ser Gly
   145 150 155 160
   Ser Leu Ser Ser Gly Val His Thr Phe Pro Ala Val Leu Glu Ser Asp
   165 170 175
   Leu Tyr Thr Leu Ser Ser Ser Val Thr Val Pro Ser Ser Pro Arg Pro
   180 185 190
   Ser Glu Thr Val Thr Cys Asn Val Ala His Pro Ala Ser Ser Thr Lys
   195 200 205
   Val Asp Lys Lys Ile Val Pro Arg Asp Cys Gly Cys Lys Pro Cys Ile
   210 215 220
   Cys Thr Val Pro Glu Val Ser Ser Val Phe Ile Phe Pro Pro Lys Pro
   225 230 235 240
   Lys Asp Val Leu Thr Ile Thr Leu Thr Pro Lys Val Thr Cys Val Val
   245 250 255
   Val Asp Ile Ser Lys Asp Asp Pro Glu Val Gln Phe Ser Trp Phe Val
   260 265 270
   Asp Asp Val Glu Val His Thr Ala Gln Thr Gln Pro Arg Glu Glu Gln
   275 280 285
   Phe Asn Ser Thr Phe Arg Ser Val Ser Glu Leu Pro Ile Met His Gln
   290 295 300
   Asp Trp Leu Asn Gly Lys Glu Phe Lys Cys Arg Val Asn Ser Ala Ala
   305 310 315 320
   Phe Pro Ala Pro Ile Glu Lys Thr Ile Ser Lys Thr Lys Gly Arg Pro
   325 330 335
   Lys Ala Pro Gln Val Tyr Thr Ile Pro Pro Pro Lys Glu Gln Met Ala
   340 345 350
   Lys Asp Lys Val Ser Leu Thr Cys Met Ile Thr Asp Phe Phe Pro Glu
   355 360 365
   Asp Ile Thr Val Glu Trp Gln Trp Asn Gly Gln Pro Ala Glu Asn Tyr
   370 375 380
   Lys Asn Thr Gln Pro Ile Met Asn Thr Asn Gly Ser Tyr Phe Val Tyr
   385 390 395 400
   Ser Lys Leu Asn Val Gln Lys Ser Asn Trp Glu Ala Gly Asn Thr Phe
   405 410 415
   Thr Cys Ser Val Leu His Glu Gly Leu His Asn His His Thr Glu Lys
   420 425 430
   Ser Leu Ser His Ser Pro Gly Lys
   435 440
<210> 64
   <211> 447
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 64
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Gly Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Asp Glu Leu Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 65
   <211> 446
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 65
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                      80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Gly Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Leu Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Pro Ala Pro Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Arg Asp Glu Leu Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Gln Gly Asn Val Phe Ser Cys Ser Val Met His Glu Ala Leu His
               420                 425                 430
   Asn His Tyr Thr Gln Lys Ser Leu Ser Leu Ser Pro Gly Lys
           435                 440                 445
<210> 66
   <211> 570
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 66
   Glu Val Lys Leu Val Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Val
           35                  40                  45
   Ala Glu Ile Arg Ser Lys Ala Asn Asn His Ala Thr Tyr Tyr Ala Glu
       50                  55                  60
   Ser Val Lys Gly Arg Phe Thr Ile Ser Arg Asp Val Ser Lys Ser Ser
   65                  70                  75                  80
   Val Tyr Leu Gln Met Asn Asn Leu Arg Ala Glu Asp Thr Gly Ile Tyr
                   85                  90                  95
   Tyr Cys Thr Arg Gly Gly Tyr Gly Phe Asp Tyr Trp Gly Gln Gly Thr
               100                 105                 110
   Thr Leu Thr Val Ser Gly Ser Ala Ser Ala Pro Thr Leu Phe Pro Leu
           115                 120                 125
   Val Ser Cys Glu Asn Ser Pro Ser Asp Thr Ser Ser Val Ala Val Gly
       130                 135                 140
   Cys Leu Ala Gln Asp Phe Leu Pro Asp Ser Ile Thr Leu Ser Trp Lys
   145                 150                 155                 160
   Tyr Lys Asn Asn Ser Asp Ile Ser Ser Thr Arg Gly Phe Pro Ser Val
                   165                 170                 175
   Leu Arg Gly Gly Lys Tyr Ala Ala Thr Ser Gln Val Leu Leu Pro Ser
               180                 185                 190
   Lys Asp Val Met Gln Gly Thr Asp Glu His Val Val Cys Lys Val Gln
           195                 200                 205
   His Pro Asn Gly Asn Lys Glu Lys Asn Val Pro Leu Pro Val Ile Ala
       210                 215                 220
   Glu Leu Pro Pro Lys Val Ser Val Phe Val Pro Pro Arg Asp Gly Phe
   225                 230                 235                 240
   Phe Gly Asn Pro Arg Lys Ser Lys Leu Ile Cys Gln Ala Thr Gly Phe
                   245                 250                 255
   Ser Pro Arg Gln Ile Gln Val Ser Trp Leu Arg Glu Gly Lys Gln Val
               260                 265                 270
   Gly Ser Gly Val Thr Thr Asp Gln Val Gln Ala Glu Ala Lys Glu Ser
           275                 280                 285
   Gly Pro Thr Thr Tyr Lys Val Thr Ser Thr Leu Thr Ile Lys Glu Ser
       290                 295                 300
   Asp Trp Leu Gly Gln Ser Met Phe Thr Cys Arg Val Asp His Arg Gly
   305                 310                 315                 320
   Leu Thr Phe Gln Gln Asn Ala Ser Ser Met Cys Val Pro Asp Gln Asp
                   325                 330                 335
   Thr Ala Ile Arg Val Phe Ala Ile Pro Pro Ser Phe Ala Ser Ile Phe
               340                 345                 350
   Leu Thr Lys Ser Thr Lys Leu Thr Cys Leu Val Thr Asp Leu Thr Thr
           355                 360                 365
   Tyr Asp Ser Val Thr Ile Ser Trp Thr Arg Gln Asn Gly Glu Ala Val
       370                 375                 380
   Lys Thr His Thr Asn Ile Ser Glu Ser His Pro Asn Ala Thr Phe Ser
   385                 390                 395                 400
   Ala Val Gly Glu Ala Ser Ile Cys Glu Asp Asp Trp Asn Ser Gly Glu
                   405                 410                 415
   Arg Phe Thr Cys Thr Val Thr His Thr Asp Leu Pro Ser Pro Leu Lys
               420                 425                 430
   Gln Thr Ile Ser Arg Pro Lys Gly Val Ala Leu His Arg Pro Asp Val
           435                 440                 445
   Tyr Leu Leu Pro Pro Ala Arg Glu Gln Leu Asn Leu Arg Glu Ser Ala
       450                 455                 460
   Thr Ile Thr Cys Leu Val Thr Gly Phe Ser Pro Ala Asp Val Phe Val
   465                 470                 475                 480
   Gln Trp Met Gln Arg Gly Gln Pro Leu Ser Pro Glu Lys Tyr Val Thr
                   485                 490                 495
   Ser Ala Pro Met Pro Glu Pro Gln Ala Pro Gly Arg Tyr Phe Ala His
               500                 505                 510
   Ser Ile Leu Thr Val Ser Glu Glu Glu Trp Asn Thr Gly Glu Thr Tyr
           515                 520                 525
   Thr Cys Val Val Ala His Glu Ala Leu Pro Asn Arg Val Thr Glu Arg
       530                 535                 540
   Thr Val Asp Lys Ser Thr Gly Lys Pro Thr Leu Tyr Asn Val Ser Leu
   545                 550                 555                 560
   Val Met Ser Asp Thr Ala Gly Thr Cys Tyr
                   565                 570
<210> 67
   <211> 569
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 67
   Glu Val Lys Leu Val Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asn Asn Tyr Thr Thr His Tyr Ala Glu
       50                  55                  60
   Ser Val Lys Gly Arg Phe Thr Ile Ser Arg Asp Asp Ser Lys Ser Ser
   65                  70                  75                  80
   Val Ser Leu Gln Met Asn Asn Leu Arg Val Glu Asp Thr Gly Ile Tyr
                   85                  90                  95
   Tyr Cys Thr Arg His Tyr Tyr Phe Asp Tyr Trp Gly Gln Gly Thr Thr
               100                 105                 110
   Leu Thr Val Ser Gly Ser Ala Ser Ala Pro Thr Leu Phe Pro Leu Val
           115                 120                 125
   Ser Cys Glu Asn Ser Pro Ser Asp Thr Ser Ser Val Ala Val Gly Cys
       130                 135                 140
   Leu Ala Gln Asp Phe Leu Pro Asp Ser Ile Thr Leu Ser Trp Lys Tyr
   145                 150                 155                 160
   Lys Asn Asn Ser Asp Ile Ser Ser Thr Arg Gly Phe Pro Ser Val Leu
                   165                 170                 175
   Arg Gly Gly Lys Tyr Ala Ala Thr Ser Gln Val Leu Leu Pro Ser Lys
               180                 185                 190
   Asp Val Met Gln Gly Thr Asp Glu His Val Val Cys Lys Val Gln His
           195                 200                 205
   Pro Asn Gly Asn Lys Glu Lys Asn Val Pro Leu Pro Val Ile Ala Glu
       210                 215                 220
   Leu Pro Pro Lys Val Ser Val Phe Val Pro Pro Arg Asp Gly Phe Phe
   225                 230                 235                 240
   Gly Asn Pro Arg Lys Ser Lys Leu Ile Cys Gln Ala Thr Gly Phe Ser
                   245                 250                 255
   Pro Arg Gln Ile Gln Val Ser Trp Leu Arg Glu Gly Lys Gln Val Gly
               260                 265                 270
   Ser Gly Val Thr Thr Asp Gln Val Gln Ala Glu Ala Lys Glu Ser Gly
           275                 280                 285
   Pro Thr Thr Tyr Lys Val Thr Ser Thr Leu Thr Ile Lys Glu Ser Asp
       290                 295                 300
   Trp Leu Gly Gln Ser Met Phe Thr Cys Arg Val Asp His Arg Gly Leu
   305                 310                 315                 320
   Thr Phe Gln Gln Asn Ala Ser Ser Met Cys Val Pro Asp Gln Asp Thr
                   325                 330                 335
   Ala Ile Arg Val Phe Ala Ile Pro Pro Ser Phe Ala Ser Ile Phe Leu
               340                 345                 350
   Thr Lys Ser Thr Lys Leu Thr Cys Leu Val Thr Asp Leu Thr Thr Tyr
           355                 360                 365
   Asp Ser Val Thr Ile Ser Trp Thr Arg Gln Asn Gly Glu Ala Val Lys
       370                 375                 380
   Thr His Thr Asn Ile Ser Glu Ser His Pro Asn Ala Thr Phe Ser Ala
   385                 390                 395                 400
   Val Gly Glu Ala Ser Ile Cys Glu Asp Asp Trp Asn Ser Gly Glu Arg
                   405                 410                 415
   Phe Thr Cys Thr Val Thr His Thr Asp Leu Pro Ser Pro Leu Lys Gln
               420                 425                 430
   Thr Ile Ser Arg Pro Lys Gly Val Ala Leu His Arg Pro Asp Val Tyr
           435                 440                 445
   Leu Leu Pro Pro Ala Arg Glu Gln Leu Asn Leu Arg Glu Ser Ala Thr
       450                 455                 460
   Ile Thr Cys Leu Val Thr Gly Phe Ser Pro Ala Asp Val Phe Val Gln
   465                 470                 475                 480
   Trp Met Gln Arg Gly Gln Pro Leu Ser Pro Glu Lys Tyr Val Thr Ser
                   485                 490                 495
   Ala Pro Met Pro Glu Pro Gln Ala Pro Gly Arg Tyr Phe Ala His Ser
               500                 505                 510
   Ile Leu Thr Val Ser Glu Glu Glu Trp Asn Thr Gly Glu Thr Tyr Thr
           515                 520                 525
   Cys Val Val Ala His Glu Ala Leu Pro Asn Arg Val Thr Glu Arg Thr
       530                 535                 540
   Val Asp Lys Ser Thr Gly Lys Pro Thr Leu Tyr Asn Val Ser Leu Val
   545                 550                 555                 560
   Met Ser Asp Thr Ala Gly Thr Cys Tyr
                   565
<210> 68
   <211> 219
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 68
   Asp Ile Val Leu Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Leu Thr Phe Gly Asp Gly Thr Lys Leu Glu Leu Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 69
   <211> 219
   <212> PRT
   <213> Künstliche Sequenz
<220>
   <223> Beschreibung der künstlichen Sequenz: Erkennungsmolekül, das das glykosylierte MUC1-Tumorepitop erkennt
<400> 69
   Asp Ile Val Met Thr Gln Ala Ala Phe Ser Asn Pro Val Thr Leu Gly
   1               5                   10                  15
   Thr Ser Ala Ser Ile Ser Cys Arg Ser Ser Lys Ser Leu Leu His Ser
               20                  25                  30
   Asn Gly Ile Thr Tyr Phe Phe Trp Tyr Leu Gln Lys Pro Gly Leu Ser
           35                  40                  45
   Pro Gln Leu Leu Ile Tyr Gln Met Ser Asn Leu Ala Ser Gly Val Pro
       50                  55                  60
   Asp Arg Phe Ser Ser Ser Gly Ser Gly Thr Asp Phe Thr Leu Arg Ile
   65                  70                  75                  80
   Ser Arg Val Glu Ala Glu Asp Val Gly Val Tyr Tyr Cys Ala Gln Asn
                   85                  90                  95
   Leu Glu Leu Pro Pro Thr Phe Gly Gly Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215

## Beschreibung

Die Erfindung betrifft Erkennungsmoleküle, die gegen Tumore gerichtet sind, pharmazeutische Zusammensetzungen, die die Erkennungsmoleküle umfassen, Verfahren zur Herstellung der Erkennungsmoleküle und die Verwendung der Erkennungsmoleküle zur Herstellung einer pharmazeutischen Zusammensetzung zur Diagnose und Therapie von Tumorerkrankungen.

Tumorerkrankungen gehören zu den häufigsten Erkrankungen von Organismen, insbesondere von Säugern wie dem Menschen. Die erfolgreiche Behandlung der Tumorerkrankung hängt insbesondere davon ab, in welchem Entwicklungsstadium des Tumors mit der Therapie begonnen wird. Vorteilhaft ist es, wenn mit der Therapie zu einem Zeitpunkt begonnen werden kann, wenn der Tumor eine minimale Ausweitung und Ausbreitung im Körper aufweist, wobei unter Ausweitung die Individualgröße des Tumorgewebes und unter Ausbreitung die mögliche Metastasierung oder Infiltration in umliegende Organe verstanden wird. Ein Organismus, der eine Tumorerkrankung aufweist, sezerniert bestimmte komplexere Substanzen oder einfachere Moleküle, die für die Diagnose von Tumoren in einem frühen Stadium der Erkrankung - d.h. bei kleineren Tumoren - eingesetzt werden können. Die bekanntesten von diesen Strukturen sind Tumormarker. Tumormarker sind in der Regel chemische Substanzen, die mehr oder weniger spezifisch für einen bestimmten Tumortyp sind bzw. in Assoziation mit einem Tumor vermehrt auftreten. Tumormarker können beispielsweise zellulärer Natur sein, wie zum Beispiel Onkogene, bestimmte Hormonrezeptoren oder Membranantigene, wie zum Beispiel CA 2, CA 3, CA 19-19 und andere. Tumormarker können jedoch auch humorale Marker sein, die entweder vom Tumor produziert oder vom Tumor induziert werden. Zu der Gruppe der von dem Tumor produzierten Tumormarker gehören insbesondere die Tumor-assoziierten Antigene, Hormone, Enzyme und sonstige Verbindungen. Von dem Tumor induzierte humorale Tumormarker sind beispielsweise Enzyme, wie die alkalische Phosphatase oder zum Beispiel das Akute-Phase-Protein (zum Beispiel Ferritin). Da Tumormarker im Stand der Technik vorzugsweise mit immunologischen Methoden nachgewiesen werden, wird für Tumormarker vielfach auch das Synonym Tumorantigen benutzt. Bekannte Tumorantigene sind onkofetale Antigene, Blutgruppen-assoziierte Antigene, organspezifische Antigene und die sonstigen Antigene, wie zum Beispiel CA 15-3.

Die Krebsdiagnostik mit Tumormarkern mit Erkennungsmolekülen weist mehrere Nachteile auf. So können bestimmte Tumormarker auch bei nichtkanzerogenen Krankheiten auftreten, wodurch die eingesetzten Erkennungsmoleküle eine positive Reaktion anzeigen; weiterhin bedeutet eine Nichtinteraktion der Erkennungsmoleküle nicht, dass keine Tumorkrankheit vorhanden ist. Ein weiterer Nachteil ist, dass die bekannten Erkennungsmoleküle in der Regel unspezifisch sind. Das bedeutet, dass ein positiver Nachweis nur in seltenen Fällen auf eine bestimmte Art der Tumorerkrankung weist. Ein weiterer, ganz entscheidender Nachteil der bekannten Erkennungsmoleküle ist außerdem, dass sie nur bedingt zur Verlaufskontrolle der Entwicklung von Tumoren, beispielsweise nach einer Operation, verwendet werden können. Das heißt, die bekannten Tumormarker können in der Regel nicht zur Früherkennung oder zur Nachbehandlung, insbesondere nicht zur Prophylaxe, eingesetzt werden.

Neben diesen allgemeinen Nachteilen treten bei Erkennungsmolekülen, die gegen Tumorantigene, die sich nur in ihrer Glykosylierung vom entsprechenden Antigen im Normalgewebe unterscheiden, spezielle Nachteile auf. Die Antikörper müssen glykosylierungsabhängig sein und die Veränderung des Glykosylierungsstatus des Antigens im Tumor widerspiegeln. So z.B. ist die Glykosylierung von MUC1 auf Brusttumorzellen verändert. Es zeigt sich eine starke Reduktion der Kettenlänge der O-Glykane und eine Reduktion der Sialinsäure O-Acetylierung.

Ein weiterer Nachteil der bekannten Erkennungsmoleküle gegen Tumormarker ist der, dass sie den Tumor erst erkennbar machen, wenn dieser eine kritische Größe bereits erreicht hat. Das heißt, frühe Stadien des Tumorwachstums können mit den bekannten Erkennungsmolekülen, die gegen Tumormarker gerichtet sind, nicht bestimmt werden.

Das polymorphe epitheliale Muzin MUC1 ist als Gesamtmolekül ein anerkannter Tumormarker. Von Price M. et al., Tumor Biology 1989, 19 (1): 1-20 wird die Analyse einer Vielzahl von monoklonalen Antikörpern gegen das MUC1 Muzin beschrieben. Aufgrund der Komplexität des Moleküls, das sehr groß ist, stark glykosyliert ist, extrazellulär im wesentlichen aus einer großen polymorphen Anzahl von Tandem Repeats aus 20 Aminosäureresten besteht und bezüglich der Tandem Repeats heterogen glykosyliert ist, besitzt MUC1 eine Vielzahl von Epitopen. Ein bestehender Nachteil ist, dass es nicht bekannt ist, welches Epitop auf MUC1 als Zielstruktur zur Tumortherapie und -diagnose optimal geeignet ist. Nachteilhaft ist weiterhin, dass herkömmliche MUC1-spezifische Antikörper, (die ein relativ gutes Erkennen von MUC1 auf bestimmten Tumoren leisten,) in hohem Maß auch solches MUC1 erkennen, das von Tumorzellen in das Serum abgegeben wird (Shedding). Diese hohe Bindung an im Serum vorliegendes MUC1 im Tumorpatienten ist offensichtlich ein Nachteil in der Therapie von Tumorerkrankungen mit solchen MUC1-spezifischen Antikörpern. Ein weiterer Nachteil ist, dass die meisten MUC1-spezifischen Antikörper auch Bindung gegen mehrere Normalgewebe zeigen.

Aufgabe der Erfindung ist es daher, Erkennungsmoleküle bereitzustellen, mit denen zum einen Tumore einfach, sicher und effizient detektiert werden können, und die weiterhin in der Prophylaxe, Therapie und/oder Nachbehandlung von Tumoren und/ Metastasen eingesetzt werden können, und die keine oder nur eine geringe Bindung an ins Serum abgegebenes MUC1 und keine oder eine geringe Bindung an Normalgewebe aufweisen.

Die Erfindung löst dieses technische Problem durch die Bereitstellung von Erkennungsmolekülen
dadurch gekennzeichnet, dass sie folgende Aminosäuresequenzen umfassen:
(i) eine Aminosäuresequenz, die eine Homologie von mindestens 60%, vorzugsweise 70%, bevorzugt 80%, ganz besonders bevorzugt 90% zu der Aminosäuresequenz SEQ ID NO:1 oder 2 hat; und
(ii) eine Aminosäuresequenz, die eine Homologie von mindestens 60%, vorzugsweise 70%, bevorzugt 80%, ganz besonders bevorzugt 90% zu der Aminosäuresequenz SEQ ID NO:3 oder 4 hat; und
(iii) eine Aminosäuresequenz, die eine Homologie von mindestens 60%, vorzugsweise 70%, bevorzugt 80%, ganz besonders bevorzugt 90% zu der Aminosäuresequenz SEQ ID NO:5 oder 6 hat"
und
das glykosylierte MUC1-Tumorepitop spezifisch in der Weise bindet, dass die Stärke der Bindung im Vergleich zur Bindung an das nicht-glykosylierte Peptid gleicher Länge und Peptidsequenz mindestens um den Faktor 20 erhöht ist,

Die Definitionen der Begriffe, die im Folgenden gemacht werden, treffen mutatis mutandis auf zuvor gemachte, diese und die nachfolgenden Ausführungen zu.

Unter dem Begriff Erkennungsmolekül versteht man erfindungsgemäß ein Molekül, das, insbesondere unter stringenten Bedingungen, das glykosylierte MUC1-Tumorepitop spezifisch bindet. Stringente Bedingungen sind beispielsweise Hochsalzkonzentrationen und exzessives Waschen mit milden Detergenzien wie NP-40 oder Tween.

Unter dem "glykosylierten MUCl-Tumorepitop" versteht man erfindungsgemäß ein Epitop, das mindestens eine PDTRP Sequenz des MUCl Tandem Repeats umfasst und am Threonin des PDTRP mit GalNAc oder Gal-GalNAc glykosyliert ist.

Unter einer spezifischen Bindung des glykosylierten MUC1-Tumorepitops versteht man erfindungsgemäß eine Bindung der erfindungsgemäßen Erkennungsmoleküle umfassend eine Kombination von folgenden Bindungseigenschaften :
a) Bindung in Testmethoden wie unter Beispiel 5 beschrieben an die glykosylierte PDTRP Region innerhalb einer MUC1 Tandem Repeat Sequenz, die aus 1 bis 1,5 Tandem Repeats besteht (Molekül aus 30 Aminosäuren siehe Beispiel 5) und am Threonin mit GalNAcalphal-O-Thr (weiter als GalNAc bezeichnet) oder Galbetal-3GalNAcalfal-O-Thr (weiter als Gal-GalNAc bezeichnet) glykosyliert ist, wobei die Stärke der Bindung im Vergleich zu dem nicht-glykosylierten Peptid gleicher Länge und Peptidsequenz um ein Vielfaches erhöht wird. Definiert wird dabei eine Erhöhung um das Vielfache indem das Bindungsverhältnis des am PDTRP glykosylierten MUC1 Glykopeptids zu dem nicht-glykosylierten Peptid in einem Test wie unter Beispiel 5.1 beschrieben (mit dem dort beschriebenen MUC1 Peptid bzw. Glykopeptid mit einer Länge von 30 Aminosäuren, die 1,5 Tandem Repeats entspricht), einen Faktor von >4,5 erreicht.
b) Bindung in Testmethoden wie unter Beispiel 5.2 beschrieben an multiple nicht-glykosylierte MUC1 Tandem Repeats, die aus mindestens 3 Tandem Repeats bevorzugt 5 Tandem Repeats bestehen.
c) Eine statistisch deutlich verminderte Bindung an im Serum von Colonkarzinompatienten vorkommendes MUC1, das von den Tumorzellen abgegeben wurde, im Vergleich zu Antikörpern des CA15-3 Tests (Beispiel 11) und des HMFG-1 (siehe auch Beispiel 11). Das Testverfahren hierzu ist im Beispiel 11 näher ausgeführt.
d) Die Interaktion zwischen Antigen und Erkennungsmolekül wird wie in Beispiel 6 beschrieben durch Neuraminidase-Behandlung erhöht oder nicht beeinflußt.
e) Es erfolgt keine oder eine nahezu nicht detektierbare Bindung an Colon-Normalgewebe und eine spezifische starke Bindung an Colontumorgewebe (siehe Beispiel 6).

Die erfindungsgemäßen Erkennungsmoleküle besitzten aufgrund der umfassten erfindungsgemäß definierten Aminosäuresequenzen, die oben und im Folgenden aufgeführt sind, eine Struktur, die die spezifische Interaktion der Erkennungsmoleküle mit dem MUC1 in Form einer spezifischen Bindung des glykosylierten MUC1 Tumorepitops mit den beschriebenen Bindungseigenschaften bedingt.

In einer bevorzugten Ausführungsform der Erfindung umfasst das Erkennungsmolekül eine Aminosäuresequenz, die die Aminosäuresequenz SEQ ID NO. 1 und die Aminosäuresequenz SEQ ID NO. 3 und die Aminosäuresequenz SEQ ID NO. 5 enthält, wobei das Erkennungsmolekül das glykosylierte MUC1-Tumorepitop spezifisch bindet.

In einer weiter bevorzugten Ausführungsform der Erfindung umfasst das Erkennungsmolekül eine Aminosäuresequenz, die die Aminosäuresequenz SEQ ID NO. 2 und die Aminosäuresequenz SEQ ID NO. 4 und die Aminosäuresequenz SEQ ID NO. 6 enthält, wobei das Erkennungsmolekül das glykosylierte MUC1-Tumorepitop spezifisch bindet. Diese Erkennungsmoleküle weisen eine Bindungserhöhung eines Faktors von >20 nach a) auf. Vorteilhafterweise weisen diese Erkennungsmoleküle weiterhin mindestens eine der folgenden Eigenschaften auf:
f) eine Bindung an nichtglykosylierte multiple Tandem Repeats wie unter b) mit einem Faktor von > 8 des Verhältnisses der Bindung an ein nichtglykosyliertes MUC1 Tandem Repeat mit 5 Tandem Repeats zu einem nichtglykosylierten MUC1 Peptid mit einem Tandem Repeat (Sequenz der Peptide und Testverfahren siehe Beispiel 5). Das Testverfahren zur Bestimmung des Faktors ist im Beispiel 5.2 näher ausgeführt.
g) eine Erhöhung der Bindungsstärke durch eine Erhöhung der Anzahl der glykosylierten Tandem Repeats (multiple glykosylierte PDTR-Region) (siehe Beispiel 5.3).

In vorteilhafterweise besitzen die weiter bevorzugten Erkennungsmoleküle alle Bindungseigenschaften a bis g.

Die erfindungsgemäßen Erkennungsmoleküle vereinen die beschriebenen Eigenschaften und sind damit vorteilhaft für die Tumordiagnose und -therapie. Sie unterscheiden sich nicht nur aufgrund ihrer neuartigen sequenzen, sondern ebenfalls aufgrund ihrer Feinspezifitäten gegen MUC1 von bekannten Antikörpern, indem sie spezifisch das glykosylierte MUC1-Tumorepitop binden, dabei nur geringe Bindung gegen MUC1 im Serum von Colonkarzinompatienten aufweisen und normales Colongewebe praktisch nicht und Colontumorgewebe stark binden. Darüber hinaus erkennen die erfindungsgemäßen Erkennungsmoleküle Colontumore bereits als Carzinom in situ, nicht jedoch milde Displasien und unterscheiden somit zwischen den gefährlichen Tumoren und den benignen Erkrankungen. Mit diesen Eigenschaften und ihrer hohen Affinität sind sie besonders für die therapeutische aber auch die diagnostische Anwendung geeignet und somit vorteilhaft gegenüber bekannten MUC1-Antikörper.
Die Kombination der Eigenschaften der erfindungsgemäßen Erkennungsmoleküle ist überraschend, da es bisher aus der Vielzahl der bekannten MUC1 Antikörper keine Antikörper mit entsprechenden Eigenschaften gab.
Dies zeigt die Überlegenheit der erfindungsgemäßen Erkennungsmoleküle gegenüber herkömmlichen MUC1-Antikörpern.
Herkömmliche Antikörper haben damit deutliche Nachteile gegenüber den erfindungsgemäßen Erkennungsmolekülen, wie im Folgenden kurz beispielhaft beschrieben ist.
Beispiele für Unterschiede in der Feinspezifität: HMFG-1 (US5804187, US6315997) und C595 (WO0244217) binden in den unter dem Bespiel 5.1 gezeigten Untersuchungen an MUC1-Peptide unabhängig von der Glykosylierung und daher nicht an das glykosylierte MUC1-Tumorepitop. HMFG-1 beispielsweise bindet darüber hinaus auch an normales Colongewebe. Sie zeigen damit nicht die vorteilhafte Bindung der erfindungsgemäßen Erkennungsmoleküle. SM3 (US5683674), der durch Immunisierung mit abgeleitetem MUC1 aus humanen Nichttumormaterial (Milchfetttröpfchen) stammt, bindet glykosylierte und nicht-glykosylierte MUC1-abgeleitete Peptide gemäß des Tests aus dem Beispiel 5.1 nahezu gleichermaßen mit einer leichten Erhöhung um einem Faktor von ca. 1,5 und zeigt damit nicht die vorteilhafte Bindung an das Tumorepitop. Außerdem zeigt der SM3 weder eine ausgeprägte Abhängigkeit von der Anzahl der nicht-glykosylierten noch der glykosylierten MUC1 Tandem Repeats (Beispiel 5.2 und 5.3). Der DF3 ist als MUC1 spezifischer Antikörper, der bevorzugt glykosylierungsunabhängig an MUC1-abgeleitete Peptide bindet, beschrieben (WO9320841, US5506343). Außerdem wird die Bindung des DF3 an MUC1 oder MUC1-tragende Tumorzellen durch Neuraminidase-Behandlung drastisch reduziert oder vollständig inhibiert (Dai J et al., 1998; Hinoda et al., 1992). HMFG-1 und DF-3 binden außerdem stark an Tumor-MUC1 im Serum, vor allem bei Mammakarzinompatienten, aber auch bei Colonkarzinompatienten (siehe Beispiel 11). Sie zeigen damit nicht die vorteilhaften Bindungseigenschaften der erfindungsgemäßen Erkennungsmoleküle.

Die Charakterisierung der Erkennungsmolküle erfolgt nach obiger Definition im Wesentlichen über Bindungseigenschaften gegenüber Colonnormal- und tumorgewebe. Dies zeigt, dass die Erkennungsmoleküle für die Therapie und Diagnostik von Colontumoren und deren Metastasen gegenüber herkömmlichen anti-MUC1 Antikörpern vorteilhaft sind. Allerdings sind die Erkennungsmoleküle nicht nur vorteilhaft für die Behandlung und Diagnose vor Colonkarzinomen und anderen Gastrointestinalen Tumoren, sondern auch für andere Tumorerkrankungen und Metastasen, bevorzugt MUC1-positiven Tumorerkrankungen und Metastasen, beispielsweise Mammakarzinomen, Gastrointestinaltumoren, einschließlich Kolonkarzinomen, Magenkarzinomen, Dickdarmkrebs und Dünndarmkrebs, Pankreaskarzinomen, Ovarialkarzinomen, Lungenkrebs, Nierenzellkarzinomen, Multiples Myelom und/oder deren Metastasen. Dies wird durch die Daten des Beispiels 6 untermauert, in dem die spezifische Bindung der Erkennungsmoleküle an Tumorzellen unterschiedlicher Tumorerkrankungen gezeigt wird. Die detaillierte Beschreibung über die Bindungseigenschaften gegenüber Colonkarzinomen dient zur Verdeutlichung der Vorteile und zur Festlegung geeigneter Parameter für die Bindungscharakterisierung von erfindungsgemäßen Erkennungsmolekülen.

In einer bevorzugten Ausführungsform umfasst ein erfindungsgemäßes Erkennungsmolekül, das das glykosylierte MUC1-Tumorepitop spezifisch bindet:
a) eine erste Aminosäuresequenz, die die Aminosäuresequenz SEQ ID NO. 1 oder 2 und die Aminosäuresequenz SEQ ID NO. 3 oder 4 und die Aminosäuresequenz SEQ ID NO. 5 oder 6 enthält; und
b) eine zweite Aminosäuresequenz, die die Aminosäuresequenz SEQ ID NO. 7 oder 8 und die Aminosäuresequenz SEQ ID NO. 9 oder 10 und die Aminosäuresequenz SEQ ID NO. 11 oder 12 enthält.

Die erste und zweite Aminosäuresequenz können dabei auf einem oder mehreren, dort bevorzugt zwei, Polypeptiden vorkommen.

Im Folgenden werden die erfindungsgemäßen Erkennungsmoleküle, die das glykosylierte MUC1-Tumorepitop im Sinne der Erfindung spezifisch binden, der Einfachheit halber auch MUC1 bindende oder spezifische Erkennungsmoleküle genannt.

Die bevorzugten MUC1 bindenden Erkennungsmoleküle sind dadurch charakterisiert, dass sie ein definiertes Set von einzelnen Aminosäuresequenzen umfassen. Die Kombination der Aminosäuresequenzen bedingt eine Struktur der Erkennungsmoleküle, die als Eigenschaft die oben beschriebene Kombination von Bindungseigenschaften gegenüber dem glykosylierten MUC1 Tumorepitop aufweist. Die Aminosäuresequenz dieser Erkennungsmoleküle umfasst ein oder zwei Tripletts definierter Sequenzen. Diese Sequenzen stellen die Bindungsdomänen dar und definieren die Spezifität des Erkennungsmoleküls. Das 1-Triplett-Erkennungsmolekül umfasst die Aminosäuresequenz SEQ ID NO. 1 oder 2, die Aminosäuresequenz SEQ ID NO. 3 oder 4 und die Aminosäuresequenz SEQ ID NO. 5 oder 6. MUC1-spezifische Erkennungsmoleküle, die durch zwei Tripletts definiert sind, umfassen für das erste Triplett die Aminosäuresequenz SEQ ID NO. 1 oder 2, die Aminosäuresequenz SEQ ID NO. 3 oder 4 und die Aminosäuresequenz SEQ ID NO. 5 oder 6 und für das zweite Triplett die Aminosäuresequenz SEQ ID NO. 7 oder 8, die Aminosäuresequenz SEQ ID NO. 9 oder 10 und die Aminosäuresequenz SEQ ID NO. 11 oder 12. Dabei können das erste und zweite Triplett entweder auf einer oder mehreren Polypeptidketten vorkommen, die im letzteren Fall gemeinsam das bindende Erkennungsmolekül bilden. Im Weiteren werden diese Tripletts im Sinne der Erfindung als Triplettsequenz 1 für die erste umfasste Aminosäuresequenz und als Triplettsequenz 2 für die zweite umfasste Aminosäuresequenz, siehe Definition a) und b) der obigen Beschreibung, bezeichnet. Das Erkerinungsmolekül kann erfindungsgemäß ein Antikörper sein, insbesondere ein muriner, chimärer oder humaner IgG oder IgM, eine scFv-Struktur oder ein anderes aus Antikörpern abgeleitetes Fragment wie beispielsweise Fab, F(ab)₂, Fv oder F(v)₂ Fragmente.

In einer bevorzugten Ausführungsform umfassen die erfindungsgemäßen MUC1 bindenden Erkennungsmoleküle als Triplettsequenz 1 die Aminosäuresequenz SEQ ID NO. 1, die Aminosäuresequenz SEQ ID NO. 3, und die Aminosäuresequenz SEQ ID NO. 5.

In einer weiter bevorzugten Ausführungsform umfassen die erfindungsgemäßen MUC1 bindenden Erkennungsmoleküle als Triplettsequenz 1 die Aminosäuresequenz SEQ ID NO. 2, die Aminosäuresequenz SEQ ID NO. 4 und die Aminosäuresequenz SEQ ID NO. 6.

In einer bevorzugten Ausführungsform umfassen die erfindungsgemäßen MUC1 bindenden Erkennungsmoleküle als Triplettsequenz 1 die Aminosäuresequenz SEQ ID NO. 1, die Aminosäuresequenz SEQ ID NO. 3 und die Aminosäuresequenz SEQ ID NO. 5 und als Triplettsequenz 2 die Aminosäuresequenz SEQ ID NO. 7, die Aminosäuresequenz SEQ ID NO. 9 und die Aminosäuresequenz SEQ ID NO. 11.

In einer weiter bevorzugten Ausführungsform umfassen die erfindungsgemäßen MUC1 bindenden Erkennungsmoleküle als Triplettsequenz 1 die Aminosäuresequenz SEQ ID NO. 2, die Aminosäuresequenz SEQ ID NO. 4 und die Aminosäuresequenz SEQ ID NO. 6 und als Triplettsequenz 2 die Aminosäuresequenz SEQ ID NO. 8, die Aminosäuresequenz SEQ ID NO. 10 und die Aminosäuresequenz SEQ ID NO. 12.

In einer weiteren Ausführungsform kann ein Erkennungsmolekül durch eine oder mehrere Mutationen in einer oder mehreren Aminosäuresequenzen ausgewählt aus SEQ ID NO. 1 bis 12 modifiziert sein, wobei einzelne Aminosäuren durch Aminosäuren mit analogen physikochemischen Eigenschaften ersetzt werden, die die 3-dimensionale Struktur der Bindungsdomäne des Erkennungsmoleküls mit Vorteil nicht grundlegend verändern, so dass die MUC1 Spezifität des Erkennungsmoleküls erhalten bleibt. Aminosäuren mit analogen physikochemischen Eigenschaften im Sinne der Erfindung können in 6 verschiedene Gruppen zusammengefaßt werden und sind in Tabelle 1 dargestellt.

**Tabelle 1: Aminosäuren mit analogen physikochemischen Eigenschaften unberücksichtigt der molekularen Größe.**

| Eigenschaft oder funktionelle Gruppe | Aminosäure |
|---|---|
| aliphatisch | Glycin |
| | Alanin |
| | Valin |
| | Leucin |
| | Isoleucin |
| Hydroxy-Gruppe | Serin |
| | Threonin |
| Carboxy-Gruppe | Asparaginsäure |
| | Glutaminsäure |
| Amid-Gruppe | Asparagin |
| | Glutamin |
| Amino-Gruppe | Lysin |
| | Arginin |
| aromatisch | Phenylalanin |
| | Tyrosin |
| | Tryptophan |

In einer weiter bevorzugten Ausführungsform der erfindungsgemäßen Erkennungsmoleküle, die MUC1 spezifisch binden, ist mindestens eine Aminosäuresequenz der Aminosäuresequenzen SEQ ID NO. 1, 2, 3, 4, 7, 8, 11 und/oder 12 durch kanonische Strukturvarianten bzw. äquivalente Strukturen mit den Aminosäuresequenzen SEQ ID NO. 13 bis 31 ersetzt, wobei die SEQ ID NO. 1 oder 2 durch eine Sequenz der Sequenzen SEQ ID NO. 13 bis 20 (CDRH1), die SEQ ID NO. 3 oder 4 durch eine Sequenz der Sequenzen SEQ ID NO. 21 bis 23 (CDRH2), die SEQ ID NO. 7 oder 8 durch eine Sequenz der Sequenzen SEQ ID NO. 24 bis 29 (CDRL1) und die SEQ ID NO. 11 oder 12 durch eine Sequenz der Sequenzen SEQ ID NO. 30 bis 31 (CDRL3) ersetzt ist.

Der generelle Zusammenhang zwischen einer Aminosäuresequenz und der Tertiärstruktur der von diesen Sequenzen gebildeten Loops ist dem Fachmann bekannt und wurde ausführlich untersucht [Rooman et al., 1989; Martin, Thornton, 1996]. Ein spezielles Beispiel stellen die Immunglobuline dar. Durch Analyse der Loop-Konformationen der hypervariablen Regionen (complementarity determining regions, CDRs) in der leichten und der schweren Kette von Antikörpermolekülen wurden sogenannte kanonische Klassen definiert [Chothia, Lesk, 1987; Chothia et al., 1986, 1989, 1992; Wu, Cygler, 1993]. Auf dieser Grundlage wurden die kanonischen Strukturvarianten SEQ ID NO. 13 bis 31 der SEQ ID NO 1, 2, 3, 4, 7, 8, 11 und 12 abgeleitet. Unter einer äquivalenten kanonischen Strukturvariante versteht man erfindungsgemäß Aminosäuresequenzen, die sich von den Ausgangssequenzen insoweit unterscheiden, daß an definierten Positionen mindestens eine Aminosäure ausgetauscht ist, ohne daß es zu einer Änderung der kanonischen Klasse kommt.

Die Aminosäuresequenzen SEQ ID NO. 1 bis 12 oder deren Modifikationen in einem MUC1 spezifischen Erkennungsmolekül im Sinne der Erfindung bilden räumliche Strukturen aus, beispielsweise so genannte Loops, die dadurch gekennzeichnet sind, dass sie eine definierbare Tertiärstruktur und/oder Quartärstruktur besitzen. Die Bindungsregion eines Erkennungsmoleküls mit dem MUC1 Antigen wird von Aminosäureresten gebildet, die von bis zu sechs variablen Loops an der Oberfläche des Moleküls bereitgestellt werden, und die spezifisch mit MUC1 interagieren.

In einer weiteren Ausführungsform der Erfindung werden Erkennungsmoleküle, die MUC1 spezifisch binden, bereitgestellt, bei denen mindestens eine Sequenz der Sequenzen des Tripletts weggelassen wird, die nicht unmittelbar an der Interaktion mit dem MUC1 Antigen beteiligt ist.

In einer weiteren Ausführungsform umfassen die Erkennungsmoleküle mindestens eine der Aminosäuresequenzen der SEQ ID NO. 1 bis 12 oder deren oben beschriebene Varianten doppelt oder mehrfach, wobei diese Dopplungen auch als Varianten der gleichen Aminosäuresequenz vorkommen können. Alle die in diesem Abschnitt beschriebenen Erkennungsmoleküle erkennen vorteilhafterweise das Antigen MUC1 spezifisch. Im Folgenden werden auch diese Erkennungsmoleküle, die streng genommen aufgrund des Weglassens oder Vervielfältigen von Sequenzen keine Triplettsequenzen tragen, trotzdem als Triplettsequenz 1 oder Triplettsequenz 2 bezeichnet, um die Anschaulichkeit zu vereinfachen.

In einer weiteren Ausführungsform, umfassen die erfindungsgemäßen Erkennungsmoleküle, die das glykosylierte MUC1-Tumorepitop spezifisch binden, Aminosäuresequenzen, die eine Homologie von mindestens 60%, vorzugsweise 70%, bevorzugt 80%, ganz besonders bevorzugt 90% gegenüber den Sequenzen SEQ ID NO. 1 bis 12 aufweisen.

Die Erkennungsmoleküle im Sinne der Erfindung können weiterhin Gerüstsequenzen umfassen, die die umfassenden Aminosäuresequenzen Aminosäuresequenz SEQ ID NO. 1 oder 2 und die Aminosäuresequenz SEQ ID NO. 3 oder 4 und die Aminosäuresequenz SEQ ID NO. 5 oder 6, oder deren oben beschriebene Varianten, voneinander trennen, und Gerüstsequenzen, die die Aminosäuresequenzen SEQ ID NO. 7 oder 8 und die Aminosäuresequenz SEQ ID NO. 9 oder 10 und die Aminosäuresequenz SEQ ID NO. 11 oder 12, oder deren oben beschriebene Varianten, voneinander trennen. Die erste und zweite Aminosäuresequenz können dabei auf einem oder mehreren, bevorzugt zwei, Polypeptidketten vorkommen. Diese Gerüstsequenzen werden im Sinne der Erfindung als Spacer oder Frameworksequenzen bezeichnet und können unterschiedliche Längen und Sequenzen haben. Dabei sind ebenfalls solche Erkennungsmoleküle ausdrücklich mit eingeschlossen, bei denen nicht alle Aminosäuresequenzen der SEQ ID NO. 1 bis 12 oder deren oben beschriebenen Varianten durch Spacer getrennt werden. Darüber hinaus haben die Erkennungsmoleküle vorzugsweise weitere flankierende Aminosäuresequenzen, die im Sinne der Erfindung auch als Gerüstsequenzen bezeichnet werden.

Die Gerüstsequenzen haben insbesondere die Aufgabe, die beschriebenen Aminosäuresequenzen, die für die MUC1 spezifische Bindung der Erkennungsmoleküle verantwortlich beziehungsweise beteiligt sind, in eine geeignete Anordnung und räumliche Struktur zu bringen, damit die Bindung an das MUC1 erfolgen kann. Es kann vorgesehen sein, dass die Aminosäuresequenzen SEQ ID NO. 1 bis NO. 12 ohne mindestens eine zusätzliche Aminosäuresequenz als Gerüstsequenz das MUC1 Antigen im Sinne der Erfindung nicht spezifisch binden können. Darüber hinaus können die Gerüstsequenzen den Erkennungsmolekülen z.B. die notwendige biologische und chemische Stabilität geben, damit die räumliche Struktur effektiv aufgebaut und für die Funktion und Anwendung in einer geeigneten funktionellen Form, die die MUC1 Bindung beinhaltet, erhalten werden kann.

In einer bevorzugten Ausführungsform werden die Triplettsequenzen in bestehende Proteine durch Austausch von Aminosäuresequenzen und/oder durch Hinzufügung eingefügt, wobei die bestehenden Proteinsequenzen als Gerüstsequenzen im Sinne der Erfindung dienen, beziehungsweise Gerüstsequenzen aus geeigneten Proteinen entnommen sind. Dabei können diese Gerüstsequenzen beispielsweise durch Mutationen, Deletionen oder Insertionen verändert werden. Hierbei bedient man sich dem Fachmann an sich bekannter Methoden der Molekularbiologie, Biochemie und Protein-Engineering. Bevorzugte Proteine hierfür sind Proteine der Immunglobulin-Superfamilie, Protease-Inhibitoren, Lektine, Helix-Bündel-Proteine und Lipocaline, wie sie z.B. offenbart sind in: Nygren und Uhlen, 1997; Nuttall SD et al., 1999 und Skerra, 2000.

In einer weiter bevorzugten Ausführungsform sind die Gerüstsequenzen Antikörpergerüstsequenzen aus einer oder verschiedenen Spezies oder Aminosäuresequenzen, die die Consensussequenz der Gerüstsequenzen muriner, humaner und/oder Antikörper anderer Säuger nachahmen. Eine Consensussequenz ist eine idealisierte Sequenz, in der repräsentativ an jeder Position die am meisten vorkommende Aminosäure steht, wenn viele existierende Sequenzen, beispielsweise aus Antikörper-Datenbanken, miteinander verglichen werden. Dabei sind die hier bevorzugten Erkennungsmoleküle dadurch gekennzeichnet, dass die Gerüstsequenzen für die erste Triplettsequenz 1 umfassend die Aminosäuresequenz SEQ ID NO. 1 oder 2, die Aminosäuresequenz SEQ ID NO. 3 oder 4 und die Aminosäuresequenz SEQ ID NO. 5 oder 6, oder deren oben beschriebene Varianten, Antikörpergerüstsequenzen der variablen schweren Kette VH, die in der Literatur auch als Framework-Sequenzen bezeichnet werden, sind und die Gerüstsequenzen für die Triplettsequenz 2 umfassend die Aminosäuresequenz SEQ ID NO. 7 oder 8, die Aminosäuresequenz SEQ ID NO. 9 oder 10 und die Aminosäuresequenz SEQ ID NO. 11 oder 12, oder deren oben beschrieben Varianten, Antikörpergerüstsequenzen der variablen leichten Kette VL sind.

Bevorzugt sind weiterhin Antikörpergerüstsequenzen von Antikörpern aus Säugetieren, besonders bevorzugt sind Antikörpergerüstsequenzen humanen und/oder murinen Ursprungs. Die Gerüstsequenzen können dabei aus Antikörpergerüstsequenzen verschiedener Spezies kombiniert werden. Diese Antikörpergerüstsequenzen sind dem Fachmann bekannt und in verschiedenen Datenbanken zugänglich wie der Kabat-Datenbank (immuno.bme.nwu.edu) oder der Datenbank des National Center for Biotechnology Information (www.ncbi.nlm.nih.gov). Ebenfalls können diese Antikörpergerüststrukturen durch weitere Aminosäuren verlängert, und/oder durch eine oder mehrere Mutationen, z.B. Deletionen und/oder Insertionen, verändert werden, wobei die spezifische Bindung an das glykosylierte MUC1-Tumorepitop erhalten bleibt.

Werden in einer bevorzugten Variante der Erfindung die Triplettsequenzen mit Antikörpergerüstsequenzen kombiniert, stellt das Erkennungsmolekül eine variable Kette eines Antikörpers oder eine davon abgeleitete Struktur dar.

Besonders bevorzugte Antikörpergerüstsequenzen als Gerüstsequenzen im Sinne der Erfindung sind für die variable schwere Kette die Aminosäuresequenzen entsprechend FRH1, FRH2, FRH3 und FHR4 in Tabelle 2 und für die variable leichte Kette die Aminosäuresequenzen entsprechend FRL1, FRL2, FRL3 und FRL4 in Tabelle 2, wobei die Aminosäuresequenzen der Triplettsequenzen 1 und 2 mit den SEQ ID NO. 1 bis 12 den entsprechenden CDR-Regionen der Antikörper entsprechen. Dabei setzen sich die variable schwere (VH) bzw. leichte (VL) Antikörperkette wie folgt zusammen: die VH: FRH1-CDRH1-FRH2-CDRH2-FRH3-CDRH3-FRH4 und die VL: FRL1-CDRL1-FRL2-CDRL2-FRL3-CDRL3-FRL4. Tabelle 2 erläutert die Positionen im Detail. Die Positionen der einzelnen Aminosäuren bzw. Aminosäuresequenzen entsprechen der Nummerierung von Aminosäuren in Antikörpermolekülen nach Kabat.

**Tabelle 2:**

| **Name** | **Positionsbereich** | **Pos.** | **Aminosäure bzw. Aminosäuresequenz** |
|---|---|---|---|
| FRH1 | 1 bis 30 | 1 | E |
| | | 2 | V |
| | | 3 | K |
| | | 4 | L |
| | | 5 | V |
| | | 6 | E |
| | | 7 | S |
| | | 8 | G |
| | | 9 | G |
| | | 10 | G |
| | | 11 | L |
| | | 12 | V |
| | | 13 | Q |
| | | 14 | P |
| | | 15 | G |
| | | 16 | G |
| | | 17 | S |
| | | 18 | M |
| | | 19 | K |
| | | 20 | L |
| | | 21 | S |
| | | 22 | c |
| | | 23 | A oder V |
| | | 24 | A, V, S oder T |
| | | 25 | S |
| | | 6 | G |
| | | 27 | Y, F, S oder D |
| | | 28 | T |
| | | 29 | F, L oder I |
| | | 30 | S |
| CDRH1 | 31 bis 35 | | SEQ ID NO. 1 oder 2 und Varianten |
| FRH2 | 36 bis 49 | 36 | W |
| | | 37 | V |
| | | 38 | R |
| | | 39 | Q |
| | | 40 | S |
| | | 41 | P |
| | | 42 | E |
| | | 43 | K |
| | | 44 | G |
| | | 45 | L |
| | | 46 | E |
| | | 47 | W |
| | | 48 | V |
| | | 49 | A |
| CDRH2 | 50 bis 65, wobei zusätzlich die Pos. 52a, 52b und 52c eingeführt sind | | SEQ ID NO. 3 oder 4 und Varianten |
| FRH3 | 66 bis 94 | 66 | R |
| | | 67 | F |
| | | 68 | T |
| | | 69 | I |
| | | 70 | S |
| | | 71 | R |
| | | 72 | D |
| | | 73 | D oder V |
| | | 74 | S |
| | | 75 | K |
| | | 76 | S |
| | | 77 | S |
| | | 78 | V |
| | | 79 | Y oder S |
| | | 80 | L |
| | | 81 | Q |
| | | 82 | M |
| | | 82a | N |
| | | 82b | N |
| | | 82c | L |
| | | 83 | R |
| | | 84 | A oder V |
| | | 85 | E |
| | | 86 | D |
| | | 87 | T |
| | | 88 | G |
| | | 89 | I |
| | | 90 | Y |
| | | 91 | Y |
| | | 92 | C |
| | | 93 | T |
| | | 94 | R, G, N, K oder S |
| CDRH3 | 95 bis 102, wobei die Pos. 100 nicht und die Pos. 99 teilweise nicht existiert | | SEQ ID NO. 5 oder 6 und Varianten |
| FRH4 | 103 bis 113 | 103 | W |
| | | 104 | G |
| | | 105 | Q |
| | | 106 | G |
| | | 107 | T |
| | | 108 | T |
| | | 109 | L |
| | | 110 | T |
| | | 111 | V |
| | | 112 | S |
| | | 113 | S oder A |
| FRL1 | 1 bis 23 | 1 | D |
| | | 2 | I, V oder L |
| | | 3 | V |
| | | 4 | M oder L |
| | | 5 | T |
| | | 6 | Q |
| | | 7 | T oder A |
| | | 8 | P oder A |
| | | 9 | L oder F |
| | | 10 | S |
| | | 11 | L oder N |
| | | 12 | P |
| | | 13 | V |
| | | 14 | S oder T |
| | | 15 | L |
| | | 16 | G |
| | | 17 | D oder T |
| | | 18 | Q oder S |
| | | 19 | A |
| | | 20 | S |
| | | 21 | I |
| | | 22 | S |
| | | 23 | C |
| CDRL1 | 24 bis 34, wobei zusätzlich die Pos. 27a, 27b, 27c, 27d und 27e eingeführt sind | | SEQ ID NO. 7 oder 8 und Varianten |
| FRL2 | 35 bis 49 | 35 | W |
| | | 36 | Y |
| | | 37 | L |
| | | 38 | Q |
| | | 39 | K |
| | | 40 | P |
| | | 41 | G |
| | | 42 | Q oder L |
| | | 43 | S |
| | | 44 | P |
| | | 45 | K oder Q |
| | | 46 | L |
| | | 47 | L |
| | | 48 | I oder V |
| | | 49 | Y |
| CDRL2 | 50 bis 56 | | SEQ ID NO. 9 oder 10 und Varianten |
| FRL3 | 57 bis 88 | 57 | G |
| | | 58 | V |
| | | 59 | P |
| | | 60 | D |
| | | 61 | R |
| | | 62 | F |
| | | 63 | S |
| | | 64 | G oder S |
| | | 65 | S |
| | | 66 | G |
| | | 67 | S |
| | | 68 | G |
| | | 69 | T |
| | | 70 | D |
| | | 71 | F |
| | | 72 | T |
| | | 73 | L |
| | | 74 | K oder R |
| | | 75 | I |
| | | 76 | S |
| | | 77 | R |
| | | 78 | V |
| | | 79 | E |
| | | 80 | A |
| | | 81 | E |
| | | 82 | D |
| | | 83 | L oder V |
| | | 84 | G |
| | | 85 | V |
| | | 86 | Y |
| | | 87 | Y |
| | | 88 | C |
| CDRL3 | 89 bis 97 | | SEQ ID NO. 11 oder 12 und Varianten |
| FRL4 | 98 bis 108 | 98 | F |
| | | 99 | G |
| | | 100 | G oder D |
| | | 101 | G |
| | | 102 | T |
| | | 103 | K |
| | | 104 | L |
| | | 105 | E |
| | | 106 | I oder L |
| | | 106a | K |
| | | 107 | R |
| | | 108 | A |

Die Aminosäuresequenzen SEQ ID NO. 32 und 33 entsprechen Aminosäuresequenzen mit bevorzugten Gerüstsequenzen für die variable schwere Kette. Die Aminosäuresequenzen SEQ ID NO. 34 und 35 entsprechen Aminosäuresequenzen mit bevorzugten Gerüstsequenzen für die variable leichte Kette. Bevorzugt ist dabei die Kombination SEQ ID NO. 32 und 34. Weiter bevorzugt ist die Kombination SEQ ID NO. 33 und 35.

Die zu verwendenden Techniken und Methoden zur Herstellung dieser Sequenzen sind dem Fachmann bekannt, ebenso ist der Fachmann in der Lage, geeignete Gerüstsequenzen und/oder Mutationen auszuwählen.

Im Sinne der Erfindung können die MUC1 spezifischen Erkennungsmoleküle in verschiedenen Formaten vorliegen. Die grundlegende Struktur des Erkennungsmoleküls sind eine oder mehrere Polypeptidketten, die oben beschriebenen erfindungsgemäßen Triplettsequenz 1 oder Triplettsequenzen 1 und 2 und Gerüstsequenzen umfassen. Beispielsweise sind die Aminosäuresequenz der variablen schweren Kette mit den Gerüstsequenzen und den Triplettsequenzen 1 und die Aminosäuresequenz der variablen leichten Kette mit den Gerüstsequenzen und den Triplettsequenzen 2 nicht-kovalent oder kovalent miteinander verknüpft, und können auf einer oder mehreren Polypeptidketten liegen. Mehrere Polypeptidketten können kovalent, beispielsweise durch Disulfidbrücken, oder nicht-kovalent verbunden als Erkennungsmolekül vorliegen.

Zu den unterschiedlichen erfindungsgemäßen Formaten der Erkennungsmoleküle gehören insbesondere die Verknüpfungen der Triplettsequenzen mit Aminosäuresequenzen, die über die oben beschriebenen Gerüstsequenzen hinausgehen. In einer bevorzugten Variante umfassen daher erfindungsgemäße Erkennungsmoleküle neben den Triplettsequenzen und den Gerüstsequenzen weitere Zusatzsequenzen. Zusatzsequenzen sind insbesondere Aminosäuresequenzen, die primär nicht der räumliche Anordnung der Triplettsequenzen wie in Form der Gerüstsequenzen dienen, diese jedoch vorteilhaft durch sekundäre oder tertiäre Wechselwirkungen beeinflussen können. Beispielsweise stabilisieren Zusatzsequenzen in Form von konstanten Domänen eines Antikörpers den Antikörper und bewirken eine Dimerisierung, wodurch es zu einer verbesserten Bindung des Antikörpers kommt, oder beispielsweise bewirkt eine Fusion eines scFv mit einer Domäne eines Bakteriophagenhüllproteins eine Aktivitätssteigerung der scFv-Bindung, wie sie z.B. in Jensen KB et al., 2002 offenbart ist.

In einer Ausführungsform umfassen die Erkennungsmoleküle Aminosäuresequenzen mit Gerüstsequenzen auf Antikörperbasis und neben den Triplettsequenzen weitere Zusatzsequenzen. Die Zusatzsequenzen haben insbesondere mindestens eine der folgenden Aufgaben:
a) Verknüpfung einer Triplettsequenz mit ihren entsprechend geeigneten Gerüstsequenzen mit mindestens einer weiteren Triplettsequenz mit ihren entsprechend geeigneten Gerüstsequenzen, um beispielsweise eine Bindungsfähigkeit zu erzeugen oder zu verbessern;
b) der Stabilisierung der Domänen, beispielsweise durch einen Linker zwischen zwei Proteindomänen oder Aminosäuresequenzen, die mit anderen der gleichen oder einer zweiten Kette in Wechselwirkung treten;
c) Effektorfunktionen für immunologische Aufgaben, beispielsweise durch Fusion mit Fc-Teil von Antikörpern, Chemokinen, Cytokinen, Wachstumsfaktoren oder Teilen davon, oder Antikörpern mit einer anderen Spezifität oder Fragmenten davon, zur Rekrutierung von Zellen des Immunsystems, beispielsweise Makrophagen, oder Teilen des Komplementsystems;
d) Fusion mit Tags, beispielsweise Multimerisierungssequenzen - zum Beispiel µ-tail-Sequenz aus IgM oder Assoziationsdomäne aus p53 oder MBL - zur Multimerisierung der Core-1 bindenden Anteile für eine multivalente Bindung oder zur Aufreinigung der Erkennungsmoleküle, beispielsweise His-Tag oder zum Nachweis, beispielsweise myc-Tag oder zur Markierung oder Chelatisierung von Erkennungsmolekülen, beispielsweise durch Lysin-reiche Sequenzen.

Geeignete Strukturen sind dem Fachmann bekannt oder durch logische Schlußfolgerung bzw. Routineversuche aus dem Stand der Technik abzuleiten.

Dabei weiter bevorzugte Ausführungsformen sind erfindungsgemäße Erkennungsmoleküle, die folgende Formate umfassen: single chain Antikörperfragment (scFv), Fv-Fragment, (Fv)₂-Fragment, Fab-Fragment, F(ab)₂-Fragment, Multibody (Dia-, Tria-, Tetrabody), Immunglobulin der Isotypen IgG, IgM, IgA, IgE, IgD oder deren Subklassen, beispielsweise IgG1, oder von Immunglobulinen abgeleitete Erkennungsmoleküle, die mindestens eine konstante Domäne umfassen.

Unter Multibodyl" versteht man erfindungsgemäß ein single chain Antikörperfragment, wobei die variable schwere Kette und die variable leichte Kette direkt oder durch einen Linker so miteinander verbunden sind, daß eine Zusammenlagerung der VH und der VL nicht intramolekular sondern nur intermolekular stattfinden kann und so eine Ausbildung von Dia-, Tria- und/oder Tetrabodies erfolgt.

Unter "Linker" versteht man erfindungsgemäß eine Aminosäure oder eine Aminosäuresequenz von bis zu 20 Aminosäuren, die die variable schwere Kette und die variable leichte Kette in einem single chain Antikörperfragment verbindet.

In einer bevorzugten Ausführungsform sind die erfindungsgemäßen Erkennungsmoleküle aus einer schweren und einer leichten Polypeptidkette zusammengesetzt, wobei die Aminosäuresequenzen der schweren und leichten Kette jeweils eine der oben beschriebenen Triplettstrukturen umfassen, die die CDR Regionen des Antikörpers darstellen, die entsprechenden Antikörpergerüstsequenzen, die die Frameworksequenzen der Antikörper darstellen, und Zusatzsequenzen, die mindestens eine der konstanten Domänen des Antikörperisotyps umfassen. Die beiden Ketten können miteinander kovalente Bindungen eingehen. Die konstanten Regionen und variablen Regionen können dabei Sequenzen von Antikörpern aus einer oder verschiedenen Spezies enthalten. Es können Teile von konstanten Domänen oder ganze konstante Domänen deletiert oder mutiert sein, beispielsweise um die Effektorfunktion der Zusatzsequenzen zu verändern, beispielsweise die Bindung an Fc-Rezeptoren zu verhindern oder zu verbessern. In einer bevorzugten Ausführungsform ist das Erkennungsmolekül ein muriner, chimärisierter, humanisierter, partiell humaner oder humaner Antikörper oder Antikörperfragment. Die Chimärisierung erfolgt beispielsweise durch Verknüpfung der variablen Antikörperdomänen mit konstanten Antikörperdomänen oder Fragmenten der konstanten Domäne von Antikörpern verschiedener Spezies. Bevorzugt sind Sequenzen der konstanten Domänen humaner Antikörper. Beispiele für murine Antikörper sind mIgG-Pankol bestehend aus den Sequenzen SEQ ID NO. 60 und 62 und mIgG-Panko2 bestehend aus den Sequenzen SEQ CD NO. 61 und 63. Beispiele für chimäre Antikörper sind die Erkennungsmoleküle cIgG-Pankol bestehend aus den Sequenzen SEQ ID NO. 64 und 68 und cIgG-Panko2 bestehend aus den Sequenzen SEQ ID NO. 65 und 69.

Die Antikörpergerüstsequenzen können so gewählt werden, dass die Sequenzen weitestgehend homolog zu humanen Antikörpersequenzen sind. Die Wahl für den Speziesursprung der Gerüstsequenzen hängt auch von der Anwendung ab. So werden für eine therapeutische Anwendung in bestimmten Bereichen möglichst große Anteile an humanen Gerüstsequenzen bevorzugt, vor allem dann, wenn eine human anti-Maus Antikörperantwort (HAMA) vermieden werden soll. In anderen therapeutischen Bereichen ist ein Xenoanteil vorteilhaft, da er das Immunsystem in einer zusätzlichen Weise stimuliert. Eine Kombination beider ist in einigen Fällen besonders geeignet, vor allem dann, wenn in einer Erstimmunisierung ein Xenoanteil vorteilhaft und bei späteren Anwendungen ein spezieskonformer und damit humaner Anteil vorteilhaft ist.

Bevorzugt ist eine Homologie zu humanen Consensussequenzen, wobei für die variable schwere Kette die HuHIII und für die variable leichte Kette die HuKII bevorzugt wird. Besonders bevorzugt ist eine Homologie zu humanen Keimbahnsequenzen, die dem Fachmann bekannt sind und zum Beispiel über die V BASE Datenbank (www.mrc-cpe.cam.ac.uk) oder die Datenbank des National Center for Biotechnology Information (www.ncbi.nlm.nih.gov) zugänglich sind.

Die zu verwendenden Techniken und Methoden zur Herstellung dieser Sequenzen sind dem Fachmann bekannt, ebenso ist der Fachmann in der Lage, - geeignete humane sequenzen auszuwählen und/oder möglicherweise notwendige Mutationen der Sequenzen durchzuführen.

In einer weiteren Ausführungsform sind zusätzlich die Triplettsequenzen, die im allgemeinen den Bindungs-Loops (CDR-Regionen) entsprechen und die bevorzugt starke Homologien zu den entsprechenden Sequenzbereichen in der humanen Keimbahnsequenz haben, diesen schrittweise durch einfache Mutationen angeglichen, ohne die spezifische Bindung an das glykosylierte MUCI-Tumorepitop zu beeinträchtigen. Erkennungsmoleküle mit diesen Sequenzen werden hier als partiell humane Antikörper oder Antikörperfragmente bezeichnet.

In einer weiteren bevorzugten Ausführungsform werden bestimmte Aminosäuren der Antikörpergerüstsequenzen einer Spezies durch andere ausgetauscht, um normalerweise weniger immunogene Regionen zu generieren. Dies beinhaltet dem Fachmann an sich bekannte Technologien, beispielsweise Technologien der Humanisierung, beispielsweise CDR-Grafting, Resurfacing, Chain-Shuffling mit Mutationen und Deimmunisierung durch Mutation oder Deletion von humanen MHC Epitopen.

In einer bevorzugten Ausführungsform handelt es sich um ein vom IgM abgeleitetes Erkennungsmolekül mit den entsprechenden konstanten Domänen eines IgM, bevorzugt humanen Sequenzen. Im Sinne der Erfindung setzten sich Immunglobuline aus der schweren Kette und der leichten Kette eines Antikörpers zusammen, wobei bevorzugt 2 leichte und 2 schwere Ketten eine Einheit darstellen. Immunglobuline des IgM Typs bestehen meist aus 5 solchen Einheiten, die zusätzlich zu Disulfidbrücken durch die J-Kette miteinander verknüpft sind.

In einer besonders bevorzugten Ausführungsform ist die J-Kette nicht vorhanden, wobei es ebenfalls zur Multimerisierung der Untereinheiten kommt, wobei hier hexa- und pentamere Strukturen vorliegen können. Beispiele für chimäre IgM Antikörper sind die Erkennungsmoleküle cIgM-Pankol bestehend aus den Sequenzen SEQ ID NO. 66 und 68 und cIgM-Panko2 bestehend aus den Sequenzen SEQ ID NO. 67 und 69.

In einer bevorzugten Ausführungsform der Erkennungsmoleküle handelt es sich um Single chain Antikörperfragmente umfassend eine Triplettstruktur 1 mit entsprechenden oben beschriebenen Antikörpergerüstsequenzen, die die CDR Regionen des Antikörpers und Frameworksequenzen der variablen Domäne der schweren Kette von Antikörpern darstellen, und eine Triplettstruktur 2 mit den entsprechenden oben beschriebenen Antikörpergerüstsequenzen, die die CDR Regionen des Antikörpers und Frameworksequenzen der variablen Domäne der leichten Kette von Antikörpern darstellen, die kovalent miteinander in Form eines Fusionsproteins verknüpft sind. Hierbei sind die Sequenzen direkt oder durch einen Linker miteinander verknüpft.
Bevorzugt sind hier scFv Formate ohne Linker oder mit einem Linker von 1 bis 9 Aminosäuren Länge. Diese scFv Antikörper bilden multimere Strukturen (beispielsweise Dia-, Tria-, Tetrabodies), die im Sinne der Erfindung auch als Multibodies bezeichnet werden und zeigen aufgrund der Multivalenz eine höhere Avidität zum glykosylierten MUC1-Tumorepitop. Wie in Beispiel 8 ausgeführt bindet im radioaktiven Zellbindungstest der Multibody mit der Sequenz SEQ ID NO. 45 um ein Vielfaches besser an die Brusttumorzelllinie T47D als der scFv Antikörper mit der Sequenz SEQ ID NO. 36. Diese multivalenten Fragmente im Dia/Triabody Format sind besonders bevorzugte Ausführungsformen der Erfindung und sind aufgrund verbesserter pharmakokinetischer Eigenschaften für die Tumortherapie von Vorteil. Bevorzugte Sequenzen sind die Sequenzen SEQ ID NO. 36 bis 47. Besonders bevorzugt sind die Sequenzen SEQ ID NO. 48 bis 59.

Besonders bevorzugte Ausführungsformen der erfindungsgemäßen Erkennungsmoleküle sind Erkennungsmoleküle, die die Sequenzen SEQ ID NO. 48 bis 59, SEQ ID NO. 61, 63, 65 oder 69 umfassen, da diese wie in den Beispielen näher beschrieben eine Kombination aller Bindungseigenschaften a) bis h) aufweisen.

In einer weiter bevorzugten Ausführungsform sind die Erkennungsmoleküle fusioniert, chemisch gekoppelt, kovalent oder nicht-kovalent assoziiert mit (i) Immunglobulindomänen verschiedener Spezies, (ii) Enzymmolekülen, (iii) Interaktionsdomänen, (iv) Signalsequenzen, (v) Fluoreszenzfarbstoffen, (vi) Toxinen, (vii) katalytischen Antikörpern, (viii) einem oder mehreren Antikörpern oder Antikörperfragmenten mit anderer Spezifität, (ix) zytolytischen Komponenten, (x) Immunmodulatoren, (xi) Immuneffektoren, (xii) MHC-Klasse I oder Klasse II Antigenen, (xiii) Chelatoren zur radioaktiven Markierung, (xiv) Radioisotopen, (xv) Liposomen, (xvi) Transmembrandomänen, (xvii) Viren und/oder Zellen. Zellen können Bakterien-, Hefe-, Pflanzen-, Insekten- und/oder Säugerzellen sein. Bevorzugt sind Säugerzellen wie beispielsweise murine, humane oder Hamsterzellen. Besonders bevorzugt sind Effektorzellen. "Effektorzellen" im Sinne der Erfindung sind Zellen, bevorzugt humane Zellen, die Immunreaktionen vermitteln wie beispielsweise Makrophagen, Dentritische Zellen, Lymphozyten und NK-Zellen. Außerdem können die Erkennungsmoleküle insbesondere mit einem Tag fusioniert sein, die die Detektion des Erkennungsmoleküls und deren Aufreinigung ermöglichen, wie zum Beispiel ein Myc-Tag oder ein His-Tag. Diese zusammengesetzten Moleküle werden im Sinne der Erfindung als "Konstrukte" bezeichnet. Technologien zur Herstellung dieser Konstrukte sind dem Fachmann bekannt, ebenso ist der Fachmann in der Lage, geeignete Sequenzen und Komponenten auszuwählen und mit den erfindungsgemäßen Erkennungsmolekülen in geeigneter Weise zu verbinden.

In einer weiteren bevorzugten Ausführungsform sind die beschriebenen Erkennungsmoleküle auf Antikörper- oder Antikörperfragment-Basis mit Peptiden oder Proteinen, die nicht von Immunglobulinen abgeleitet sind, fusioniert. Beispielsweise wird die Multimerisierungsdomäne eines Nicht-Immunglobulinmoleküls mit einem scFv fusioniert, insbesondere das C-terminale Ende der alpha-Kette des C4 Bindungsproteins, wie es bei Tonye Libyh M. et al., 1997 beschrieben ist, und somit ein multivalentes Erkennungsmolekül konstruiert.

In einer weiteren Ausführungsform wird ein scFv mit einer Transmembrandomäne eines Nicht-Immunglobulinmoleküls fusioniert, beispielsweise mit der Transmembrandomäne des c-erb B2, des h-PDGFR, des humanen Transferrinrezeptors oder des humanen Asialoglykoprotein-Rezeptors (Liao et al., 2000), und somit die Expression von Bindungsmolekülen auf der Oberfläche von Zellen ermöglicht.

Eine weitere bevorzugte Ausführungsform der Erfindung umfasst erfindungsgemäße Erkennungsmoleküle, die weiterhin Aminosäuresequenzen umfassen, die spezifisch an Makrophagen oder andere Immuneffektorzellen binden. Beispielsweise umfassen die erfindungsgemäßen Erkennungsmoleküle weiterhin eine Antikörperbindungsstelle gegen CD64, wodurch es in Form eines bispezifischen Antikörpers beziehungsweise Antikörperfragments (Diabodies) zur Bindung von Makrophagen an MUC1 positive Tumorzellen kommt, was zu deren Bekämpfung und/oder Zerstörung führt.

Eine bevorzugte Ausführungsform der Erfindung betrifft radioaktiv markierte MUC1 spezifische Erkennungsmoleküle. Eine bevorzugte Form sind Erkennungsmoleküle auf der Basis von Antikörpern oder Antikörperfragmenten. Eine weitere bevorzugte Ausführungsform sind radioaktiv markierte erfindungsgemäße Erkennungsmoleküle im single chain Format (einschließlich als Dia-, Tria-, Tetrabodies). Weitere bevorzugte Formen sind radioaktiv markierte single chain Antikörperfragmente und ganze Immunglobuline, beispielsweise erfindungsgemäße murine, chimäre oder humanisierte IgG oder IgM Antikörper oder murine oder humanisierte Antikörperfragmente. Die Erfindung ist selbstverständlich nicht auf diese Antikörper, die radioaktive Markierung und diese Formate der Antikörper beschränkt.

In einer bevorzugten Ausführungsform handelt es sich um radioaktiv-markierte oder toxin-markierte Erkennungsmoleküle, die xenogene Anteile, beispielsweise murine Anteile, im Fc Teil des Antikörpers besitzen, damit die radioaktiven Antikörper im Menschen im Blut eine kürzere Verweildauer haben und schneller ausgeschieden werden. Beispiele hierfür sind die Erkennungsmoleküle mIgG-Pankol bestehend aus den Sequenzen SEQ ID NO. 60 und 62 und mIgG-Panko2 bestehend aus den Sequenzen SEQ ID NO. 61 und 63. Eine weiter bevorzugte Variante sind solche Erkennungsmoleküle auf Antikörperbasis, bei denen die murinen Anteile minimiert sind, jedoch die murinen Anteile enthalten, die für die Entfernung aus dem Blut (clearance) verantwortlich sind. Dem Fachmann ist bekannt wie er die entsprechenden Anteile ermittelt.

Antikörperfragmente wie die bevorzugten multivalenten scFv Fragmente insbesondere ohne oder mit sehr kurzem Linker bieten gegenüber intakten monoklonalen Antikörpern einen Vorteil für das Targeting von soliden Tumoren. Bei intakten Antikörpern, die in Biodistributionsstudien eine spezifische Anreicherung im Tumorareal zeigen, fällt bei genauer Untersuchung des Tumors eine inhomogene Antikörperverteilung mit vornehmlicher Anreicherung im Randbereich auf. Zentral gelegene Tumoranteile werden aufgrund von Tumornekrosen, inhomogener Antigenverteilung sowie einem erhöhten interstitiellen Gewebedruck mit diesen Antikörperkonstrukten nicht erreicht. Kleinere Antikörperfragmente zeigen dagegen eine schnelle Tumormarkierung, dringen tiefer in den Tumor ein und werden gleichzeitig relativ schnell aus der Blutbahn entfernt. Die Dissoziationskonstante von monovalenten Antikörperfragmenten wie Fabs oder scFv ist allerdings oftmals zu niedrig, was in einer kurzen Verweildauer an den Tumorzellen resultiert. Deshalb bieten multivalente Antikörperfragmente und -konstrukte wie beispielsweise Multibodies (Diabodies, Tria/Tetrabodies), F(ab')₂ und andere Minibodies (multivalente Antikörperkonstrukte bestehend aus der Bindungsdomäne und einer Multimerisierungssequenz, beispielsweise scFv und CH3 Domäne eines IgG) in der Tumortherapie viele Vorteile. Multivalenten Antikörperfragmente im Dia/Triabody Format (Multibodies) sind bevorzugte Ausführungsformen der Erfindung und sind aufgrund verbesserter pharmakokinetischer Eigenschaften für die Tumortherapie von Vorteil und wurden zur Verwendung in der Tumortherapie weiter entwickelt. Sie können als Vehikel für die spezifische Anreicherung von zum Beispiel zytotoxischen Substanzen wie Chemotherapeutika oder Radionuklide im Tumor verwendet werden. Durch geeignete Radionuklidwahl können Tumorzellen über eine Distanz von mehreren Zelldurchmessern abgetötet werden, wodurch auch Antigen-negative Tumorzellen in einem Tumorareal erfasst und die schlechte Penetration der Antikörper in solide Tumoren zumindest teilweise ausgeglichen werden können.

Eine besonders bevorzugte Ausführungsform der Erfindung sind radioaktiv markierte Multibodies, die besonders vorteilhafte pharmakokinetischen Eigenschaften vereinen mit einer in der Kombination gegenüber ganzen Immunglobulinen und scFv verbesserten Tumorretention, Tumorpenetration, Serumhalbwertszeit und Serum zu Tumor-Verteilungsverhältnis. Weitere Vorteile sind die hohe Avidität und die bakterielle Expression, die es erlaubt, kostengünstig diese Erkennungsmoleküle herzustellen. Damit eignet sich dieses besondere Format der erfindungsgemäßen Erkennungsmoleküle vorteilhafterweise bevorzugt für die Behandlung von kleinen Primärtumoren, Metastasen und minimal residual Erkrankungen.

Eine bevorzugte Ausführungsform der Erfindung sind nicht radioaktiv markierte Erkennungsmoleküle. Eine bevorzugte Form hierbei sind Erkennungsmoleküle auf der Basis von Antikörpern oder Antikörperfragmenten.

Weitere bevorzugte Ausführungsformen sind toxin- oder Zytostatika-gekoppelte erfindungsgemäße chimärisierte oder humanisierte IgG und IgM basierte Erkennungsmoleküle und im Besonderen Multibodies (Dia- Tria-, Tetrabodies) mit besonders vorteilhaften pharmakokinetischen Eigenschaften wie oben ausgeführt.

Eine weitere bevorzugte Ausführungsform sind Liposomen, die beispielsweise mit Toxinen oder Zytostatika beladen sind, und die auf ihrer Oberfläche erfindungsgemäße Erkennungsmoleküle tragen.

Der Fachmann ist in der Lage, geeignete Radioisotope, Toxine und Zytostatika auszuwählen. Geeignete Techniken, Verfahren, Dosierungen und Formulierungen sind dem Fachmann bekannt.

Eine bevorzugte Ausführungsform der Erfindung sind Effektorzellen des Immunsystems, auf deren Oberfläche erfindungsgemäße Erkennungsmoleküle gebunden sind, die die Effektorzellen zu MUC1 tragenden Tumorzellen dirigieren/adressieren und dadurch deren Bekämpfung und/oder Zerstören vermitteln. Bevorzugte Effektorzellen sind Makrophagen, dendritische Zellen und NK-Zellen, die aus dem Patienten gewonnen werden und ex vivo mit den Erkennungsmolekülen gekoppelt werden. Weiter bevorzugt sind Zelllinien dieser Zelltypen. Die Kopplung erfolgt beispielsweise durch bispezifische Erkennungsmoleküle, die neben den MUC1 spezifischen Anteilen weiterhin Aminosäuren umfassen, die eine Bindung an die Effektorzellen vermitteln. Beispielsweise sind dies bispezifische Antikörper, Komplementanteile oder konstante Domänen von Antikörpern.

Eine weiter bevorzugte Ausführungsform sind hierbei Makrophagen aus dem Patienten, die nach Gewinnung mit einem bispezifischen Antikörper beispielsweise in Form ganzer Antikörper, bevorzugt chemisch gekoppelter Fab-Fragmente oder weiter bevorzugt Diabodies, die zum einen CD64 erkennen und zum anderen erfindungsgemäß MUC1 spezifisch sind. Diese Makrophagen, die über die CD64 Spezifität die bispezifischen Erkennungsmoleküle tragen, werden dem Patienten in einer geeigneten Formulierung wieder zugeführt, um den MUC1 positiven Tumor zu bekämpfen. Die hierzu verwendeten Techniken und die geeigneten Verfahren, Dosierungen und Formulierungen sind dem Fachmann bekannt. Eine weiter bevorzugte Ausführungsform sind Makrophagen aus dem Patienten, die nach Gewinnung mit einem erfindungsgemäßen MUC1 spezifischen Antikörper oder Antiköperfragment, die den konstanten Teil eines Antikörpers umfassen, der an Makrophagen über die an sich bekannten Fc-Rezeptoren bindet. Dabei können die Erkennungsmoleküle entweder als ganze Antikörper, bevorzugt chimäre oder humanisierte IgG oder IgM, oder als Antikörperfragment, beispielsweise scFv, Fab oder Multibodies in Form eines Fusionsproteins oder chemisch gekoppelt mit dem dem Fachmann bekannten Teil der konstanten Domäne von Antikörpern, an die Makrophagen binden. Diese die Erkennungsmoleküle tragenden Makrophagen werden dem Patienten in einer geeigneten Formulierung wieder zugeführt, um den MUC1 positiven Tumor zu bekämpfen. Die hierzu verwendeten Techniken und die geeigneten Verfahren, Dosierungen und Formulierungen sind dem Fachmann bekannt.

Eine weiter bevorzugte Ausführungsform der Konstrukte der Erfindung sind Zelllinien oder Zellen aus dem Körper wie die oben beschriebenen Effektorzellen, die mit Molekülen transfiziert werden, die erfindungsgemäße MUC1 spezifische Erkennungsmoleküle und weiterhin Elemente umfassen, die eine Expression und eine Verankerung in der Membran bewirken, beispielsweise transmembrane Domäne, und die Aktivierung der Effektorzellen bei Kontakt mit einer MUC1 tragenden Tumorzelle vermitteln. Die entsprechenden Elemente sind dem Fachmann bekannt. Beispielsweise wird eine dendritische Zelllinie mit einem Vektor transfiziert, der ein Erkennungsmolekül umfasst, das ein erfindungsgemäßes scFv oder Multibody und eine Transmembrandomäne und eine aktivierende Domäne umfasst. In einem anderen Beispiel werden dazu Makrophagen viral transfiziert. Diese die Erkennungsmoleküle tragenden Effektorzellen werden einem Patienten in einer geeigneten Formulierung zugeführt um den MUC1 positiven Tumor zu bekämpfen. Die hierzu verwendeten Techniken und die geeigneten Verfahren, Dosierungen und Formulierungen sind dem Fachmann bekannt.

Die Erfindung betrifft auch ein Verfahren zur Herstellung der erfindungsgemässen Erkennungsmoleküle oder Konstrukte, bei dem Nukleinsäuremoleküle, die ein oder mehrere genetische Sequenzen umfassen, die mindestens eines der oben beschriebenen erfindungsgemäßen Erkennungsmoleküle und/oder Konstrukte kodieren. Aufgrund des degenerierten genetischen Codes können diese Nukleinsäuremoleküle sehr unterschiedliche Sequenzen haben. Die Wahl der Codons ist ebenfalls von der Zelle abhängig, die für die Herstellung des Erkennungsmoleküls verwendet wird, da in unterschiedlichen Zellen aus unterschiedlichen Organismen häufig unterschiedliche Codons bevorzugt werden und die Expressionsrate stark beeinflusst werden kann, beispielsweise sind die in eukaryontischen Genen bevorzugt verwendeten Codons AGA und AGG für Arginin in Bakterien nur selten vertreten. Hier treten die Codons CGC und CGU deutlich häufiger auf. Das erfindungsgemäße Nukleinsäuremolekül ist in bevorzugten Ausführungsformen eine genomische DNA, eine cDNA und/oder eine RNA. Die Kriterien zur Wahl geeigneter Codons und die Herstellung eines geeigneten Nukleinsäuremoleküls sind dem Fachmann bekannt.

Erfindungsgemäβ werden in einem Verfahren Vektoren zur Expression der Erkennungsmoleküle insbesondere in Zellen verwendet. Unter einem Vektor versteht man im Sinne der Erfindung ein erfindungsgemäßes Nukleinsäuremolekül, das zur Expression des Erkennungsmoleküls dient und das eine Nukleinsäuresequenz, die ein oder mehrere genetische Sequenzen umfasst, die mindestens eines der oben beschriebenen Erkennungsmoleküle kodieren, und insbesondere mindestens einen Promotor umfasst, der die Expression des Erkennungsmoleküls bewirkt. Vektoren können dabei selbstverständlich weitere Elemente umfassen, die dem Fachmann bekannt sind und die beispielsweise der Vermehrung von Vektoren zur Herstellung in geeigneten Zellen und zur Klonierung dienen. Die Nukleinsäuresequenzen können auf einem oder mehreren Vektoren vorliegen, beispielsweise wird in einer bevorzugten Ausführungsform die schwere Kette eines erfindungsgemäßen Immunglobulins durch einen und die leichte Kette durch einen anderen Vektor kodiert. In einer weiteren bevorzugten Ausführungsform der Erfindung sind die variable Domäne der leichten Kette und die variable Domäne der schweren Kette auf dem gleichen Vektor unter einem Promotor als Fusionsprotein kodiert. Außerdem können im Sinne der Erfindung Nukleinsäuresequenzen, die Teile eines Erkennungsmoleküls kodieren, durch unterschiedliche dem Fachmann bekannte Promotoren exprimiert werden. In einer
Die unterschiedlichen Nukleinsäuresequenzen können auf einem gemeinsamen Vektor liegen. Dabei kann jede Sequenz durch einen eigenen, gleichen oder unterschiedlichen, Promotor exprimiert werden oder die Sequenzen können in einem bicistronischen Vektor unter einem Promotor vorliegen. Bevorzugt werden durch die unterschiedlichen Promotoren unterschiedliche Expressionsraten der Teile der Erkennungsmoleküle erreicht, die eine Bildung des gesamten Erkennungsmoleküls gegenüber einer gleichen Expressionsrate der verschiedenen Teile verbessert. Weiterhin bevorzugt werden Promotoren verwendet, die induzierbar sind, um eine Expression des Erkennungsmoleküls zu verbessern. Besonders bevorzugt umfassen die Vektoren weiterhin andere dem Fachmann bekannte regulatorische Elemente, beispielsweise Enhancer, die die Expression des Erkennungsmoleküls oder Teile davon verstärken, beispielsweise der CMV Enhancer oder Immunglobulin-Enhancer-Sequenzen. Bevorzugt umfassen die Nukleinsäuremoleküle und Vektoren zusätzlich Nukleinsäuresequenzen, die als Signalsequenzen zur Sekretion des Erkennungsmoleküls oder Teilen davon dienen, die dem Fachmann an sich bekannt sind, beispielsweise PelB, OmpA oder MalE für prokaryontische Zellsysteme bzw. das Signalpeptid des T-Zellrezeptors, der Immunglobulinketten, des t-PA oder EPO für eukaryontische Zellsysteme [Boel et al., 2000; Herrera et al., 2000]. Dies erleichtert vorteilhafterweise die Reinigung und/oder verbessert die Ausbeute der Erkennungsmoleküle. Die Verfahren zur Herstellung der oben beschriebenen Nukleinsäuren und Vektoren, geeigneter Promotoren, Enhancer und Vektorkonstrukte sowie die Kriterien zu deren Wahl sind dem Fachmann bekannt und werden in den Beispielen näher erläutert.

Der Vektor kann weiterhin Nukleinsäuresequenzen umfassen, die für virale Proteine kodieren. Als eine besondere Form eines Vektors wird dabei der virus selbst bezeichnet, dessen genetisches Material eine Nukleinsäuresequenz umfasst, die für ein erfindungsgemäßes Erkennungsmolekül kodiert. In einer bevorzugen Form ist das Erkennungsmolekül ein Fusionsprotein mit einem Virushüllprotein oder Teilen davon, das es ermöglicht, dass nicht nur das genetische Material die Nukleinsäuresequenz des Erkennungsmoleküls umfasst, sondern auch das Erkennungsmolekül selbst auf der Oberfläche des Virus bindungsaktiv vorliegt, beispielsweise ein erfindungsgemäßes scFv Erkennungsmolekül als Fusionsprotein mit einem Hüllprotein von für gentherapeutische Anwendungen geeigneten Adenoviren, Poxviren oder Vacciniaviren. Dies vermittelt die Adressierung des Virus zu einer MUC1 exprimierenden Tumorzelle, wodurch es zur Expression des Erkennungsmoleküls in der Tumorzelle kommt. Dies kann zur Expression des Erkennungsmoleküls in vivo im Organismus oder in vitro in der Zellkultur verwendet werden. Bevorzugt werden dabei bekannte Systeme verwendet, die einen Helfervirus zur Replikation verwenden, um beispielsweise die Sicherheit eines diesen Vektor umfassenden gentherapeutischen Verfahrens zu gewährleisten. Die Verfahren zur Herstellung der beschriebenen viralen Vektoren, zur Infektion und Expression der Erkennungsmoleküle sind dem Fachmann bekannt.

Der Vektorkann ein Fusionsprotein aus einem erfindungsgemäßen Erkennungsmolekül und einem Protein oder Peptid, das spezifisch an ein Virus bindet, umfassen. Die gewonnenen Erkennungsmoleküle können so mit Vorteil zur Adressierung des Virus an eine MUC1 exprimierende Zelle verwendet werden. So kann z.B. der Transfer des genetischen Materials über Infektionen vermittelt werden, wodurch es ermöglicht wird, spezifische Moleküle, die durch das genetische Material des Virus kodiert werden, in den Zellen in vivo im Organismus in Form einer Gentherapie oder in vitro in der Zellkultur zu exprimieren.

Das Verfahren zur Gewinnung der Erkennungsmoleküle umfasst das Einbringen von ein oder mehreren erfindungsgemäßen Vektoren, die ein oder mehrere erfindungsgemäße Nukleinsäuremoleküle enthalten, in eine geeignete Wirtszelle, die Kultivierung dieser Wirtszelle unter geeigneten Bedingungen und die Bereitstellung von ein oder mehreren Erkennungsmolekülen aus den Zellen oder dem Kulturmedium. Unter dem Begriff "Einbringen von Vektoren" versteht man im Sinne der Erfindung dem Fachmann an sich bekannte Technologien mit denen der Vektor in eine Wirtszelle gebracht wird, beispielsweise Elektroporation, Transfektion unter Verwendung kationischer Lipide oder Infektion und dort transient oder stabil verbleibt. Unter dem Begriff "Bereitstellung von einem oder mehreren Erkennungsmolekülen" versteht man im Sinne der Erfindung dem Fachmann an sich bekannte Technologien mit denen die während des Kultivierungsprozesses exprimierten Erkennungsmoleküle aus dem Kulturüberstand und/oder Zellen gewonnen werden, beispielsweise unterschiedliche proteinchemische Reinigungsschritte beispielsweise Fraktionierung, Konzentrierung, Fällungen, und/oder Chromatographie. Die in dem Verfahren zu verwendenden Techniken und Methoden sind dem Fachmann bekannt, ebenso ist der Fachmann in der Lage, geeignete Wirtszellen und Kultivierungsbedingungen sowie Methoden zur Bereitstellung aus den Zellen und/oder dem Kulturüberstand auszuwählen. Hierbei wählt der Fachmann beispielsweise, wie bereits oben ausgeführt, Nukleinsäuresequenzen mit geeigneten Codons und Promotorsequenzen abgestimmt auf die Wirtszelle, um eine möglichst starke Expression von aktiven Erkennungsmolekülen zu gewinnen. In einer bevorzugten Ausführungsform verwendet der Fachmann bespielsweise affinitätschromatographische Schritte, beispielsweise Chromatographie an Protein A oder Protein G oder Protein L oder beipielsweise Metallionen-Affinitätschromatographie über einen zusätzlich eingefügten His-Tag. In den Beispielen ist dies beispielhaft näher ausgeführt.

Der Begriff "Gewinnung" umfasst neben den zuvor explizit genannten Schritten auch zusätzliche Schritte, wie etwa Vorbehandlungen des Ausgangsstoffes oder Weiterbehandlungen des Endproduktes. Vorbehandlungsverfähren sind an sich dem Fachmann bekannt. Weiterbehandlungsverfahren umfassen neben den oben beschrieben Bereitstellungsverfahren beispielsweise auch die endgültige Zusammensetzungen und/oder Formulierung des mit dem Herstellungsverfahren gewonnenen Erkennungsmoleküls in geeigneten Verwendungs- und/oder Darreichungsformen. Die Art der Verwendungs- oder Darreichungsform, z.B. Lösung, Lyophilisat oder Tablette, hängt hierbei von der beabsichtigten Verwendung ab. Dem Fachmann ist hierbei bekannt, welche Darreichungsform sich für welchen Verwendungszweck eignet. Je nach Darreichungsform kann das durch das erfindungsgemäße Verfahren hergestellte Erkennungsmolekül zusammen mit Hilfs-, Träger- oder weiteren Wirkstoffen vorliegen. Hilfsstoffe sind hierbei vorzugsweise Adjuvantien, weitere Wirkstoffe, vorzugsweise immunstimulatorische Moleküle, wie Interleukine. Das mit dem erfindungsgemäßen Verfahren hergestellte Erkennungsmolekül kann auch in Weiterbehandlungsschritten chemisch modifiziert werden. Vorzugsweise wird das Erkennungsmolekül hierbei mit einem oder mehreren weiteren Molekülen in geeigneter Weise, d.h. durch chemische oder physikalische Interaktion, verbunden. Als weitere Moleküle im Sinne der Erfindung dienen bevorzugt anderen Proteine oder Peptide, die mit dem durch das erfindungsgemäße Verfahren hergestellten Erkennungsmolekül kovalent oder nicht-kovalent verknüpft werden, beispielsweise um bispezifische Erkennungsmoleküle herzustellen, indem ein erfindungsgemäßes Erkennungsmolekül, das spezifisch das MUC1 Antigen erkennt, mit einem zweiten Molekül verknüpft wird, das beispielsweise eine Immuneffektorzelle (beispielsweise Makrophage, NK-Zellen, Dendritische Zellen) spezifisch bindet oder beispielsweise eine Verknüpfung mit Interleukinen (beispielsweise IL-2, IL-7, IL-12, IL-15), Chemokinen oder Wachstumsfaktoren, wodurch über die Wirkung dieser Moleküle über die Bindung des erfindungsgemäßen Erkennungsmoleküls Immuneffektoren an die Core-1 positiven Tumorzellen dirigiert werden und diese beispielsweise bekämpfen und/oder zerstören. Diese weiteren Moleküle oder Teile davon können wie bereits weiter oben beschrieben auch Teil des Erkennungsmoleküls selbst sein und werden in diesem Fall nicht über die hier beschriebenen chemischen oder physikalischen' Methoden nach der Expression des Erkennungsmoleküls verknüpft. Unter "Immuneffektoren" versteht man im Sinne der Erfindung solche Komponenten der Erfindung die direkt oder indirekt eine Bekämpfung und/oder Zerstörung von MUC1 positiven Tumorzellen bewirken können, beispielsweise Immuneffektorzellen, wie beispielsweise Makrophagen, NK-Zellen, Dendritische Zellen, oder Effektormoleküle, wie beispielsweise Proteine oder Peptide des Komplementsystems. Als weitere Moleküle im Rahmen des erfindungsgemäßen Verfahrens sind besonders Substanzen geeignet, die eine therapeutische oder diagnostische Wirkung entfalten, beispielsweise Radioisotope oder Toxine. Diese Substanzen werden über an sich bekannte Verfahren mit den Erkennungsmolekülen verknüpft, beispielsweise werden Radioisotope entweder direkt eingelagert (beispielsweise Iod) oder über einen kovalent gekoppelten Chelator (beispielsweise Yttrium, Indium, Bismut) gebunden. Die Schritte des Weiterbehandlungsverfahrens sind dem Fachmann bekannt.

Die zur Expression der Erkennungsmoleküle erfindungsgemäß verwendeten Zellen können prokaryontische oder eukaryontische Zellen sein, beispielsweise Bakterien-, Hefe- (bevorzugt S.cerevisiae oder P.pastoris), Insekten-(D.melanogaster), Pflanzen-, Säugerzellen (bevorzugt Hamster-, Maus- oder humane Zelllinien) oder Organismen wie transgene Tiere und Pflanzen. Vorzugsweise werden zur Expression der erfindungsgemäßen Erkennungsmoleküle in einem prokaryontischen System E.coli und zur Expression in einem eukaryontischen System die Säugerzelllinien NS0, SP2/0, CHO-K1, CHOdhfr-, COS-1, COS-7, HEK293, K562, Namalwa oder Percy 6 verwendet.

Die Erfindung betrifft weiterhin Zusammensetzungen für therapeutische, prophylaktische oder diagnostische Zwecke umfassend mindestens ein erfindungsgemäßes Erkennungsmolekül in einer geeigneten, insbesondere einer pharmazeutisch geeigneten Form oder Zusammensetzung. Die pharmazeutische Zusammensetzung umfaßt insbesondere zusätzliche Stoffe und Substanzen, beispielsweise medizinische und/oder pharmazeutisch-technische Hilfsstoffe. Im Sinne der Erfindung gelten als Arzneimittel sowohl solche pharmazeutischen Zusammensetzungen, die für therapeutische und prophylaktische Zwecke verwendet werden, als auch solche pharmazeutischen Zusammensetzungen, die in vivo als Diagnostikum eingesetzt werden. In einer weiteren bevorzugten Ausführungsform handelt es sich um Zusammensetzungen für die ex vivo Diagnostik die zusätzliche Stoffe und Substanzen enthalten können. Diese Ausführungsform ist unter der Beschreibung für die Diagnostika näher ausgeführt.

"Arzneimittel oder pharmazeutische Zusammensetzungen", die vorliegend synonym verwendet werden, sind erfindungsgemäß Stoffe und Zubereitungen aus Stoffen, die dazu bestimmt sind, durch Anwendung am oder im menschlichen Körper Krankheiten, Leiden, Körperschäden oder krankhafte Beschwerden zu heilen, zu lindern oder zu verhüten. Medizinische Hilfsstoffe sind erfindungsgemäß solche Stoffe, die zur Produktion als aktive Ingredienzien von Arzneimitteln eingesetzt werden. Pharmazeutisch-technische Hilfsstoffe dienen der geeigneten Formulierung des Arzneimittels oder der pharmazeutischen Zusammensetzung und können sogar, sofern sie nur während des Herstellungsverfahrens benötigt werden, anschließend entfernt werden oder können als pharmazeutisch verträgliche Träger Teil der pharmazeutischen Zusammensetzung sein. Beispiele für pharmazeutisch verträgliche Träger sind nachstehend aufgeführt. Die Arzneimittelformulierung oder Formulierung der pharmazeutischen Zusammensetzung erfolgt gegebenenfalls in Kombination mit einem pharmazeutisch verträglichen Träger und/oder Verdünnungsmittel. Beispiele für geeignete pharmazeutisch verträgliche Träger sind dem Fachmann bekannt und umfassen z.B. Phosphat-gepufferte Kochsalzlösungen, Wasser, Emulsionen wie z.B. Öl/Wasser-Emulsionen, verschiedene Arten von Detergenzien, sterile Lösungen, etc. Arzneimittel oder pharmazeutische Zusammensetzungen, die solche Träger umfassen, können mittels bekannter konventioneller Methoden formuliert werden. Diese Arzneimittel oder pharmazeutischen Zusammensetzungen können einem Individuum in einer geeigneten Dosis verabreicht werden, beispielsweise in einem Bereich von 1µg bis 10 g an Erkennungsmolekülen pro Tag und Patient. Bevorzugt werden dabei Dosen von 1 mg bis 1 g. Bevorzugt wird eine Verabreichung von möglichst wenigen und niedrigen Dosen und weiter bevorzugt eine einmalige Dosis, beispielsweise eines radioaktiv-markierten Erkennungsmoleküls. Die Verabreichung kann auf verschiedenen Wegen erfolgen, beispielsweise intravenös, intraperitoneal, intrarektal, intragastrointestinal, intranodal, intramuskulär, lokal, beispielsweise in den Tumor, aber auch subkutan, intradermal oder auf der Haut oder über die Schleimhäute. Die Verabreichung von Nukleinsäuren kann auch in Form von Gen-Therapie geschehen, beispielsweise über weiter oben beschriebene virale Vektoren. Die Art der Dosierung und des verabreichungsweges kann vom behandelnden Arzt entsprechend den klinischen Faktoren bestimmt werden. Es ist dem Fachmann bekannt, dass die Art der Dosierung von verschiedenen Faktoren abhängig ist, wie z.B. der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden.

Eine "Vakzinen-Zusammensetzung" ist eine pharmazeutische Zusammensetzung zur prophylaktischen oder therapeutischen aktiven Immunisierung von Patienten, um über das immunologische Netzwerk eine spezifische Immunantwort gegen das glykosylierte MUC1-Tumorepitop im Patienten hervorzurufen.

Die pharmazeutischen Zusammensetzungen oder das Arzneimittel umfasst insbesondere eine pharmakologische Substanz, die ein oder mehrere erfindungsgemäße Erkennungsmoleküle oder/und diese kodierende Nukleinsäuremoleküle in einer geeigneten Lösung oder Verabreichungsform enthält. Diese können entweder alleine mit den entsprechenden unter Arzneimitteln oder pharmazeutischen Zusammensetzungen beschriebenen Hilfsstoffen oder in Kombination mit einem oder mehreren Adjuvantien, beispielsweise QS-21, GPI-0100 oder andere Saponine, Wasser-Öl emulsionen wie beispielsweise Montanide Adjuvantien, Polylysin, Polyargininverbindungen, DNA-Verbindungen wie beispielsweise CpG, Detox, bakterielle Vakzine wie beispielsweise Thyphusvakzine oder BCG-Vakzine, Salze wie beispielsweise Kalziumphosphate und/oder einem anderen geeigneten Stoff zur Wirkungsverstärkung verabreicht werden; vorzugsweise immunstimulatorische Moleküle, wie Interleukine, beispielsweise IL-2, IL-12, IL-4 und/oder Wachstumsfaktoren, beispielsweise GM-CSF. Diese werden in bekannten Methoden mit den erfindungsgemäßen Erkennungsmolekülen gemischt und in einer geeigneten Formulierung und Dosierung verabreicht. Formulierungen, Dosierungen und geeignete Komponenten sind dem Fachmann bekannt.

Die Herstellung der Arzneimittel oder pharmazeutischen Zusammensetzungen erfolgt nach an sich bekannten Methoden.

Die Arzneimittel oder pharmazeutischen Zusammensetzungen werden zur Prophylaxe oder Behandlung von Tumorerkarnkungen und/oder Metastasen eingesetzt, insbesondere zur Behandlung von MUC1, positiven Tumorerkrankungen und Metastasen, wie beispielsweise Mammakarzinome, Gastrointestinaltumore, einschließlich Kolonkarzinome, Magenkarzinome, Dickdarmkrebs und Dünndarmkrebs, Pankreaskarzinome, Ovarialkarzinome, Leberkarzinome, Lungenkrebs, Nierenzellkarzinome, Multiples Myelom. Die Behandlung geht beispielsweise gegen Primärtumoren, minimal residuale Tumorerkrankungen, Relapses und/oder Metastasen. Die Behandlung der Tumoren kann auch als adjuvante Behandlung erfolgen. Die Verwendung der Arzneimittel kann auch zur Prophylaxe von MUC1 positiven Tumorerkrankungen erfolgen. Die prophylaktische Anwendung zielt beispielsweise auf eine Prophylaxe des Tumors sowie von Metastasen. Die Tumormittel werden in einer geeigneten Form nach bekannten Methoden verabreicht. Eine bevorzugte Variante ist die Injektion beziehungsweise Verabreichung der Arzneimittel intravenös, lokal in Körperkavitäten, beispielsweise intraperitoneal, intrarektal, intragastrointestinal, lokal beispielsweise direkt in den Tumor, Organe oder Lymphgefäße (intranodal), aber auch subkutan, intradermal oder auf der Haut, intramuskulär. Veralbreichungsarten können bevorzugterweise auch kombiniert werden, wobei sie an verschiedenen Behandlungstagen oder an einem Behandlungstag verabreicht werden können. Dabei können auch 2 oder mehrere der erfindungsgemäßen Arzneimittel oder pharmazeutischen Zusammensetzungen kombiniert werden oder eine oder mehrere erfindungsgemäße Arzneimittel mit ein oder mehreren Arzneimitteln oder Tumorbehandlungen, wie beispielsweise Antikörpertherapien, Chemotherapien oder Radiotherapien, die zeitlich gemeinsam oder getrennt verabreicht beziehungsweise angewandt werden. Geeignete Formulierungen, Dosierungen und Kombinationen von Komponenten sind dem Fachmann bekannt oder können durch diesen nach bekannten Methoden bestimmt werden.

Das Verfahren zur Herstellung des erfindungsgemässen Arzneimittels oder der pharmazeutischen Zusammensetzung umfassend die Schritte der Herstellung von Erkennungsmolekülen und weiterhin umfassend den Schritt der Formulierung der erfindungsgemäßen Erkennungsmoleküle in pharmazeutisch verträglicher Form. Die hierfür bevorzugten erfindungsgemäßen Erkennungsmoleküle sind weiter oben als Ausführungsformen zur Behandlung von Tumorerkrankungen und Prophylaxe, sowie weiter unten unter in vivo Diagnostika näher beschrieben.

Die erfindungsgemäßen Erkennungsmoleküle oder Konstrukte können demgemäß bevorzugt zur Prophylaxe, Diagnose, Verlaufskontrolle und/oder Behandlung von Tumorerkrankungen verwendet werden. Die Verwendung der Erkennungsmoleküle, der Vektoren und/oder des Arzneimittels oder der pharmazeutischen Zusammensetzung zur Prophylaxe und/oder Behandlung von Krebserkrankungen, einschließlich Tumoren und Metastasen ist weiterhin bevorzugt.

In einer bevorzugten Ausführungsform ist die Krebserkrankung oder der Tumor, die/der behandelt, prophylaktisch verhindert oder dessen Wiederauftreten verhindert wird, ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenitalsystems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppression-bezogenen Malignitäten und/oder Tumor-Metastasen.

Insbesondere kann es sich bei den Tumoren um folgende Krebsarten handeln: Adenokarzinom der Brust, der Prostata und.des Dickdarms; alle Formen von Lungenkrebs, der von den Bronchien ausgeht; Knochenmarkkrebs, das Melanom, das Hepatom, das Neuroblästom; das Papillom; das Apudom, das Choristom, das Branchiom; das maligne Karzinoid-Syndrom; die Karzinoid-Herzerkrankung; das Karzinom (z.B. Walker-Karzinom, Basalzellen-Karzinom, basosquamöses Karzinom, Brown-Pearce-Karzinom, duktales Karzinom, Ehrlich-Tumor, in situ-Karzinom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchiolo-alveoläres Karzinom, Bronchiai-Karzinom, Plattenepithelkarzinom und Transitionalzell-Karzinom); histiocytische Funktionsstörung; Leukämie (z.B. in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom, Chordom, Craniopharyngiom, Dysgerminom, Hamartom; Mesenchymom; Mesonephrom, Myosarkom, Ameloblastom, Cementom; Odontom; Teratom; Thymom, Chorioblastom; Adenokarzinom, Adenom; Cholangiom; Cholesteatom; Cylindrom; Cystadenocarcinom, Cystadenom; Granulosazelltumor; Gynadroblastom; Hidradenom; Inselzelltumor; Leydig-Zelltumor; Papillom; Sertoli-ZellTumor, Thekazelltumor, Leiomyom; Leiomyosarkom; Myoblastom; Myom; Myosarkom; Rhabdomyom; Rhabdomyosarkom; Ependynom; Ganglioneurom, Gliom; Medulloblastom, Meningiom; Neurilemmom; Neuroblastom; Neuroepitheliom, Neurofibrom, Neurom, Paragangliom, nicht-chromaffines Paragangliom, Angiokeratom, angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangiomyom, Lymphangiosarkom; Pinealom; Cystosarkom phyllodes; Hemangiosarkom; Lymphangiosarkom; Myxosarkom, Ovarialkarzinom; Sarkom (z.B. Ewing-Sarkom, experimentell, Kaposi-Sarkom und Mastzell-Sarkom); Neoplasmen (z.B. Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des Urogenitaltrakts); Neurofibromatose und zervikale Plattenepitheldysplasie.

In einer weiter bevorzugten Ausführungsform ist die Krebserkrankung oder der Tumor, die/der behandelt, prophylaktisch verhindert oder dessen Wiederauftreten verhindert wird, ausgewählt aus der Gruppe von Krebserkrankungen oder Tumorerkrankungen, die Zellen umfassen, die das MUC1 in der erfindungsgemäßen Definition umfassen, ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehirnmetastasen, Lungenmetastasen, Lebernetastasen, Knochenmetastasen, pleurale und perikardiale Metastasen und maligne Aszites.

In einer weiter bevorzugten Ausführungsform ist die Krebserkrankung oder der Tumor, die/der behandelt, prophylaktisch verhindert oder dessen Wiederauftreten verhindert wird, ausgewählt aus der Gruppe umfassend Krebserkrankungen oder Tumorerkrankungen der Mammakarzinome, der Gastrointestinaltumore, einschließlich Kolonkarzinome, Magenkarzinome, Dickdarmkrebs und Dünndarmkrebs, der Pankreaskarzinome, der Ovarialkarzinome, Leberkarzinome, Lungenkrebs, Nierenzellkarzinome, Multiple Myelome.

In einer weiter bevorzugten Ausführungsform werden die erfindungsgemäßen Erkennungsmoleküle zur prophylaxe von Mamma- oder Ovarialkarzinomen bei Frauen mit erhöhtem Risiko für Brustkrebs eingesetzt.

Insbesondere werden in einer weiter bevorzugten Ausführungsform die Krebserkrankung, der Tumor oder Metastasierung behandelt, prophylaktisch verhindert oder dessen Wiederauftreten verhindert ausgewählt aus der Gruppe der gastrointestinalen Tumoren und dabei bevorzugt, Colonkarzinome, Magenkarzinome und rektale Karzinome.

Die erfindungsgemäßen Erkennungsmoleküle können zur Herstellung einer pharmezentischen Zusahnenschzung zur Behandlung oder Prophylaxe von Tumorerkrankungen direkt eingesetzt werden oder mit zusätzlichen Effektorstrukturen gekoppelt werden. Unter "Effektorstrukturen" versteht man erfindungsgemäß solche chemischen oder biochemischen Verbindnungen, Moleküle oder Atome, die direkt oder indirekt eine Abtötung oder Schädigung, einschließlich beispielsweise Wachstumsverlangsamung oder Wachstumsinhibition von Tumorzellen bewirken. Hierzu gehören beispielsweise Radioisotope, Toxine, Cytostatika und andere Effektormoleküle wie beispielsweise Cytokine und Chemokine oder andere Strukturen, die selbst Effektoren darstellen oder an die Effektormoleküle gekoppelt werden, beispielsweise mit Toxinen oder Cytostatika beladene Liposomen, die erfindungsgemäße Erkennungsmoleküle tragen. Beim letzteren Beispiel der Liposomen sind insbesondere auch solche Effektorstrukturen gemeint, die neben dem Erkennungsmolekül für die Tumorspezifität auch solche Moleküle tragen, die für eine Aufnahme der Effektorstrukturen oder Teile davon in die Zellen verantwortlich sind, wie beispielsweise Antikörper gegen Rezeptoren, die eine Rezeptor-vermittelte Endozytose bewirken. Vorzugsweise umfassen die Erkennungsmoleküle in diesen Fällen eine Transmembrandomäne, die ihnen eine Insertion in die Liposomenmembran erlaubt oder in einer anderen bevorzugten Ausführungsform werden die Erkennungsmoleküle chemisch auf die Liposomenoberfläche gekoppelt. Die hierfür verwendeten Techniken sind dem Fachmann bekannt, einschließlich der Herstellung der Liposomen. Auch die Verbindung der Erkennungsmoleküle mit den anderen Effektorstrukturen erfolgt nach an sich bekannten Methoden. Die Kopplungen können dabei wie bereits oben ausgeführt beispielsweise direkt durch kovalente oder nicht-kovalente Beladung erfolgen, durch chemische Kopplung, wobei ein zusätzliches chemisches oder biologisches Molekül notwendig sein kann, beispielsweise ein Chelator oder ein Linker, oder in Form von Fusionsproteinen oder -peptiden durch Fusion. Eingesetzt werden die Erkennungsmoleküle bei der Behandlung von Tumorerkrankungen mit MUC1 tragenden Tumoren oder zur Prophylaxe, die beispielsweise die Ausbildung von primären Tumoren oder Metastasen verhindert. Bevorzugtes Ziel ist dabei die Behandlung der minimalen residualen Erkrankung und von Metastasen. Die erfindungsgemäßen Erkennungsmoleküle werden dabei in einer geeigneten Formulierung einmalig oder wiederholt in geeigneten zeitlichen Abständen und Dosen verabreicht.

In einer bevorzugten Ausführungsform werden die erfindungsgemäßen oben beschriebenen radioaktiven Erkennungsmoleküle mit einer Applikation von nicht markierten erfindungsgemäßen MUC1 spezifischen Erkennungsmolekülen kombiniert. Dies dient der Verbesserung des Hintergrundes und damit einer spezifischeren Bindung an den Tumor, indem die geringe Menge an MUC1 tragenden Molekülen im Blut abgesättigt werden. Bevorzugt werden dabei IgG Moleküle und weiter bevorzugt werden IgM abgleitete Erkennungsmoleküle verwendet, beispielsweise ein cIgMG oder die humanisierte Form davon. da diese vor allem an MUC1 Antigen im Blut binden und damit den Hintergrund und die Serumbelastung mit Radioaktivität erniedrigen und das relative Tumortargeting erhöhen, während aufgrund der Größe der Moleküle ein Eindringen in Gewebe und Tumoren limitiert ist. Die hierfür verwendeten Verfahren und Technologien sind dem Fachmann bekannt, ebenfalls kann der Fachmann eine geeignete Dosis, Formulierungen, Applikationsroute und Zeitpunkt der Gabe der nicht-markierten Erkennungsmoleküle erstellen.

Das Verfahren zur Herstellung des Diagnostikums umfassend die Schritte des erfindungsgemäßen Verfahrens zur Herstellung der erfindungsgemäßen MUC11 spezifischen Erkennungsmoleküle und weiterhin umfassend den Schritt der Formulierung der Erkennungsmoleküle in einer diagnostisch verwendbaren Form.

Unter dem Begriff "Diagnostikum" sind erfindungsgemäß Stoffe und Zubereitungen aus Stoffen definiert, die dazu bestimmt sind, durch Anwendung am oder im menschlichen Körper oder Teilen davon Krankheiten, Leiden, Körperschäden oder krankhafte Beschwerden zu erkennen. Als Teile des menschlichen Körpers sind vorzugsweise Körperflüssigkeiten, wie Blut, Blutserum, Lymphe, Urin, Spinalflüssigkeit oder Sperma, oder Gewebebiopsien oder -proben zu verstehen.

Die Formulierung des Diagnostikums umfasst vorzugsweise die Modifikation der hergestellten Erkennungsmoleküle mit Substanzen, die einen Nachweis des Antigens MUC1 erlauben. Geeignete Substanzen sind im Stand der Technik bekannt. Ausgehend von der Wahl der Substanz ist der Fachmann in der Lage, geeignete Maßnahmen zur Formulierung des Diagnostikums einzusetzten.

Für die Diagnostik können erfindungsgemäß auch Substanzen nach an sich bekannten Methoden an die Erkennungsmoleküle gekoppelt werden, die einen Nachweis des MUC1 Antigens und/oder deren Trägerzellen erleichtern, beispielsweise durch Biotinylierung, Fluoreszenzmarkierung, radioaktive Markierung oder Enzymkopplung der Erkennungsmoleküle.

In einem weiteren Verfahren zur Tumordiagnostik und Prognose werden erfindungsgemäße Erkennungsmoleküle verwendet, die MUC1 Antigene im Serum von Menschen erkennen. Dies ist auch vor dem Hintergrund der Eigenschaft der Erkennungsmoleküle, dass sie im Vergleich zu den bekannten Antikörpern HMFG-1 und DF-3 (CA15-3 Test) viel weniger MUC1 im Serum binden möglich und vorteilhaft, da die erfindungsgemäßen Erkennungsmoleküle deutlich zwischen Normalseren und Tumorseren unterscheiden können, auch dann wenn die Bindung in Colontumorpatientenseren gering ist.

Die Bestimmung erfolgt bevorzugt qualitativ, quantitativ und/oder in zeitlich relativen Quantitäten nach an sich bekannten Methoden. Die gleichen Verfahren werden erfindungsgemäß auch zur Verlaufskontrolle von Tumorerkrankungen und zur Kontrolle von Behandlungsverläufen einschließlich des Monitorings von Immunantworten und zur Kontrolle und Dosierung von Tumorbehandlungen eingesetzt. Die in den Verfahren verwendeten Methoden sind an sich bekannt, beispielsweise ELISA, Westerblot, FACS (Fluoreszenzaktivierte Zellsortierung), MACS (Magnetvermittelte Zellsortierung), ADCC (Antikörpervermittelte Zellzytotoxizität), CDC (Komplement vermittelte Zytotoxizität), Immuncytochemie und Immunhistochemie.

In den bevorzugten erfindungsgemäßen Verfahren zur in vitro Tumordiagnostik und Prognose werden erfindungsgemäße MUC1 spezifische Erkennungsmoleküle in an sich bekannten Verfahren eingesetzt, um das Antigen glykosylierte MUC1-Tumorepitop im Serum oder in Gewebspräparaten nachzuweisen. Dabei wird das Antigen MUC1, in Immunkomplexen vorliegendes MUC1 und/oder auf Zellen gebundenes MUC1 nachgewiesen und das Vorhandensein des MUC1 Antigens qualitativ, quantitativ und/oder in relativen Quantitäten nach an sich bekannten Methoden bestimmt. Die gleichen Verfahren werden erfindungsgemäß auch zur Verlaufskontrolle von Tumorerkrankungen und zur Kontrolle von Behandlungsverläufen eingesetzt. Die in den Verfahren verwendeten Methoden sind an sich bekannt, beispielsweise ELISA, Western-Blot, FACS (Fluoreszenzaktivierte Zellsortierung), MACS (Magnetvermittelte Zellsortierung), ADCC (Antikörpervermittelte Zellzytotoxizität), CDC (Komplement vermittelte Zytotoxizität), Immuncytochemie und Immunhistochemie.

Eine bevorzugte Ausführungsform ist ein Gewebsschnelltest, bei dem in einem immunhistologischen Verfahren die Gewebsproben mit fluoreszenzmarkierten erfindungsgemäßen Erkennungsmolekülen gefärbt werden. In einem weiter bevorzugten Verfahren wird das erfindungsgemäße Erkennungsmolekül, bevorzugt ein Antikörper des Isotyps IgG, mit einem weiteren Antikörper, der spezifisch das Antigen Core-1 erkennt, bevorzugt Isotyp IgM, kombiniert. Der Vorteil dabei ist, dass beispielsweise für die Diagnostik von gastrointestinalen Karzinomen (z.B. Colorektale Karzinome und Magenkarzinome) diese in einem frühen Stadium erkannt und gleichzeitig eine Prognose bezüglich des Krankheitsverlaufes und/oder der Lebermetastasierungsgefahr gegeben werden kann, wobei ein höheres Niveau an Core-1 und MUC1 Antigen eine schlechtere Verlaufsprognose und ein höheres Niveau an Core-1 eine um das mehrfach höhere Wahrscheinlichkeit der Lebermetastasierung bedeutet. In einer weiter bevorzugten Ausführungsform sind die Antikörper und Erkennungsmoleküle direkt mit unterschiedlichen Fluoreszenzfarbstoffen, beispielsweise Cy3 und Cy5 oder Cy3 und FITC, markiert. In einer Ausführungsform, in der eine Signalverstärkung vorteilhaft ist, werden die Antikörper und/oder Erkennungsmoleküle durch markierte sekundäre Antikörper oder das Biotin-Streptavidin verstärkt. Dabei ist es vorteilhaft, unterschiedliche Isotypen und/oder Speziessequenzen im konstanten Teil der Antikörper zu verwenden. Die hierbei verwendeten Technologien und Methoden, beispielsweise der Markierung und der Immunhistologie, sowohl die Wahl der geeigneten Formate der Erkennungsmoleküle sind dem Fachmann bekannt. Das beschriebene diagnostische Verfahren ist nicht auf gastrointestinale Tumoren beschränkt, sondern anwendbar für alle das Antigen MUC1 tragende Tumorerkrankungen.

In einer weiter bevorzugten Ausführungsform der Erfindung werden für einen serologischen Tumortest die Bestimmung des MUC1 Antigens, wie zuvor beschrieben, mit der Bestimmung anderer serologischer Tumormarker kombiniert, beispielsweise PSA, CEA oder AFP. Eine hierbei bevorzugte Ausführungsform ist die Bestimmung des MUC1 und des Core-1 Antigens. In einer bevorzugten Ausführungsform wird hierbei das MUC1 mit Hilfe eines MUC1 spezifischen Antikörpers aus dem Serum an eine feste Phase immobilisiert und mit einem erfindungsgemäßen Erkennungsmoleküls, das das glykosylierte MUC1-Tumorepitop spezifisch bindet, als Nachweisantikörper nachgewiesen und das Core-1 Antigen auf dem mit Hilfe eines anti-MUC1 Fängerantikörpers wie die erfindungsgemäßen MUC1 spezifischen Erkennungsmoleküle immobilisierten MUC1 mit einem spezifischen anti-Core-1 Antikörper nachgewiesen. Dieser diagnostische Test verbindet eine Früherkennung mit einer prognostischen Aussage über den Krankheitsverlauf und/oder der Lebermetastasierungswahrscheinlichkeit. Die hierbei verwendeten Technologien, beispielsweise der Markierung und der Serologie, einschließlich der Nachweismethoden, sind dem Fachmann bekannt. Die beschriebenen diagnostischen Verfahren sind nicht auf gastrointestinale Tumoren beschränkt, sondern anwendbar für alle das Antigen MUC1 tragende Tumoren. Die beschriebenen serologischen Tests dienen der Diagnose, des Monitorings des Verlaufs der Tumorerkrankung und der Prognose von MUC1-positiven Tumoren.

In einem weiteren offenbarten Verfahren werden die erfindungsgemäßen MUC1-spezifischen Erkennungsmoleküle zu einer in vivo Diagnostik verwendet. Hierfür werden die Erkennungsmoleküle mit geeigneten an sich bekannten Verfahren markiert und somit für an sich bekannte bildgebende Verfahren am Menschen zugänglich gemacht, beispielsweise Radioimmundiagnostik, PET-Scan Verfahren oder Immunofluoreszenzendoskopie, beispielweise durch Kopplung und/oder Beladung mit entsprechenden Molekülen, beispielsweise radioaktiven Isotope, beispielsweise das Indium, oder Fluoreszenzfarbstoffe, beispielsweise dem Cy3, Cy2, Cy5 oder FITC. In einer bevorzugten Ausführungsform werden erfindungsgemäße Multibodies mit einem geeigneten Chelator (beispielsweise DOTA oder DTPA) kovalent gekoppelt und mit Indium-111 beladen und zur in vivo Diagnostik eingesetzt. Diese werden in einer bevorzugten ausführungsform intravenös in einer für das Individuum geeigneten Dosis verabreicht und die Lokalisation des MUC1 Antigens und eines potentiellen Tumors nach an sich bekannten Verfahren gemessen. Die hierfür verwendeten Verfahren und Technologien, einschließlich der bildgebenden Verfahren, sind dem Fachmann bekannt, ebenfalls kann der Fachmann eine geeignete Dosis und Formulierungen erstellen.

In einer weiteren bevorzugten Ausführungsform werden Immunglobuline, bevorzugt IgG, wie oben beschrieben und in den Beispielen näher ausgeführt, radioaktiv-markiert, beispielsweise mit Indium-111, und lokal in den Tumor oder in den Tumor versorgende oder entsorgende Blutgefäß gegeben. Dies dient in einer Ausführungsform zur Bestimmung der Größe des Tumors und in einer weiteren Ausführungsform der Bestimmung befallener Lymphknoten. Die hierfür verwendeten Verfahren und Technologien sind dem Fachmann bekannt, ebenfalls kann der Fachmann eine geeignete Dosis und Formulierungen erstellen.

Die als Diagnostikum bereitgestellten erfindungsgemäßen radioaktiv-markierten Erkennungsmoleküle können auch über andere Applikationsrouten verabreicht. Dabei bevorzugte Routen sind intraperitoneal, intranodal oder intrarectal bzw. intragastrointestinal. Intraperitoneal ist dabei besonders vorteilhaft zur Bestimmung von Tumoren, die über das Peritoneum zugänglich sind und/oder in dieses metastasieren, beispielsweise Ovarialkarzimome und bestimmte gastrointestinale Karzinome. Intrarectale bzw. intragastrointestinale Verabreichung ist vorteilhaft für bestimmte gastrointestinale Tumoren und deren Lokalisation und Größenbestimmung. Intranodal kann in bestimmten Fällen dafür verwendet werden einzelne Lymphknoten direkt zu infiltrieren.

Die erfindungsgemäßen oben beschriebenen radioaktiven Erkennungsmoleküle für in vivo Diagnostika können mit einer Applikation von nicht markierten erfindungsgemäßen MUC1 spezifischen Erkennungsmolekülen kombiniert werden. Dies dient der Verbesserung des Hintergrundes. Die hierfür verwendeten Verfahren und Technologien sind dem Fachmann bekannt, ebenfalls kann der Fachmann eine geeignete Dosis, Formulierungen, Applikationsroute und Zeitpunkt der Gabe der nicht-markierten Erkennungsmoleküle erstellen.

In einer weiteren bevorzugten Ausführungsform werden erfindungsgemäße Erkennungsmoleküle, bevorzugt Immunglobuline, Multibodies oder Antikörperfragmente, weiter bevorzugt IgG, Fab und Multibodies, mit einem Fluoreszenzfarbstoff markiert und in vivo verabreicht. Bevorzugte Applikationsrouten sind hierbei intrarectal, intragastrointestinal, intraperitoneal, intravenös und in zuführende oder abführende Blutgefäße. Eine besonders bevorzugte Ausführungsform dient der Lokalisation gastrointestinaler Karzinome, die durch eine Fluoreszenz-endoskopie nach Applikation der fluoreszenzmarkierten Erkennungsmoleküle durchgeführt wird. In einer weiter bevorzugten Ausführungsform wird ein erfindungsgemäßes Erkennungsmolekül mit mindestens einem Antikörper gegen ein weiteres Tumorantigen kombiniert, bevorzugt anti-Core-1 Antikörper. Bevorzugt werden dabei unterschiedliche Fluoreszenzfarbstoffe verwendet, die eine Unterscheidung der Erkennungsmoleküle und Antikörper erlauben, womit eine prognostische Aussage mit einer Früherkennung und einer größeren Anzahl an Fällen kombiniert wird. Bevorzugte Fluoreszenzfarbstoffe sind solche mit geringer Hintergrundfluoreszenz, die dem Fachmann bekannt sind. Die hierfür verwendeten Verfahren und. Technologien, einschließlich der bildgebenden Verfahren, beispielsweise der Fluoreszenz-Endoskopie, sind dem Fachmann bekannt, ebenfalls kann der Fachmann eine geeignete Dosis, Formulierungen, Applikationsroute und Zeitpunkt der Gabe der nicht-markierten Erkennungsmoleküle erstellen.

Die Erfindung weist mehrere Vorteile auf: Die erfindungsgemäßen MUC1 spezifischen Erkennungsmoleküle erkennen Tumorarten spezifisch, wodurch pharmazeutische Zusammensetzungen mit Erkennungs- molekülen mit Vorteil bei vielen Tumorpatienten verschiedener Indikation zu einer Diagnose und/oder Therapie verwendet werden können. Darüber hinaus binden die Erkennungsmoleküle vorteilhafterweise sehr gering auf oder an in vivo nicht zugänglichen Bereichen im normalen Geweben. Dies ist gegenüber den bekannten Tumormarkern Antikörpern ein besonderer Vorteil und eine herausragende Eigenschaft der erfindungsgemäßen Erkennungsmoleküle. Ein besonderer Vorteil der erfindungsgemäßen Erkennungsmoleküle ist die hohe Spezifität für das Tumorgewebe. Dies ist insbesondere in der hohen Spezifität für ein definiertes glykosyliertes MUC1-Tumorepitop begründet. Das polymorphe epitheliale Muzin MUC1 ist als Gesamtmolekül ein anerkannter Tumormarker. Aufgrund der Komplexität des Moleküls, das sehr groß ist, stark glykosyliert ist, extrazellulär im wesentlichen aus einer großen polymorphen Anzahl von Tandem Repeats aus 20 Aminosäureresten besteht und bezüglich der Tandem Repeats heterogen glykosyliert ist, besitzt MUC1 eine Vielzahl von Epitopen. Die erfindungsgemäßen Erkennungsmoleküle, die spezifisch das glykosylierte MUC1-Tumorepitop im Sinne der Erfindung spezifisch binden, detektieren ein definiertes Epitop im MUC1, was eine hohe Spezifität der Erkennungsmoleküle für Tumorgewebe zur Folge hat. Besonders vorteilhaft ist weiterhin, dass die erfindungsgemäßen Erkennungsmoleküle nur im geringen Maß solches MUC1 erkennen, das von Tumorzellen in das Serum abgegeben wird (Shedding). Diese reduzierte Bindung an im Serum vorliegendes MUC1 im Tumorpatienten ist ein großer Vorteil in der Therapie von Tumorerkrankungen mit den erfindungsgemäßen Erkennungsmolekülen. Weiterhin weisen die erfindungsgemäßen Erkennungsmoleküle eine hohe Affinität auf. Hierdurch ist insbesondere die Möglichkeit gegeben, geringervalente Fragmente zu konstruierten, wie scFv und Multibodies. Die Möglichkeit dieser verschiedenen Formate ist vorteilhaft für die Entwicklung von Therapeutika.

Im Folgenden soll die Erfindung anhand von Beispielen näher erläutert werden, ohne auf diese Beispiele beschränkt zu sein.

### Beispiele

### 1. Herstellung von scFv mit verschiedenen Linkerlängen, die das glykosylierte MUC1 Tumorepitop spezifisch erkennen

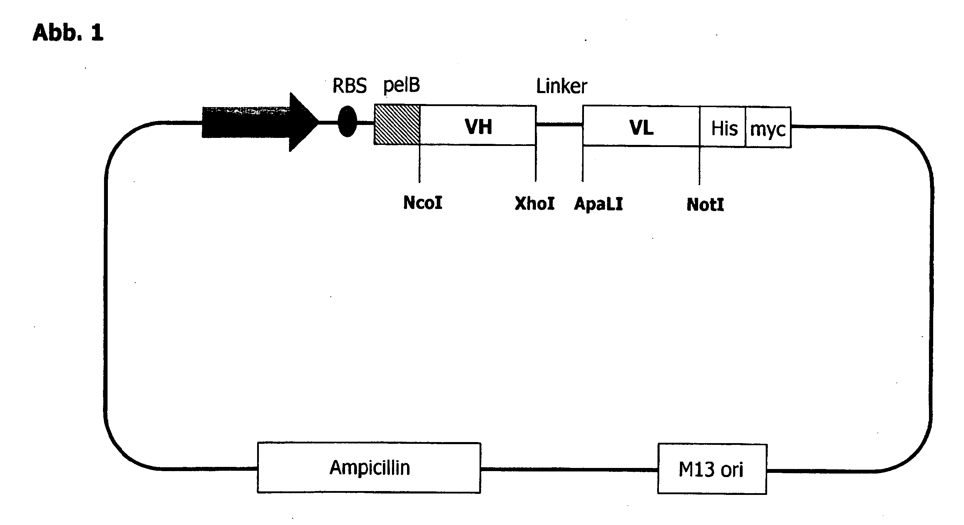

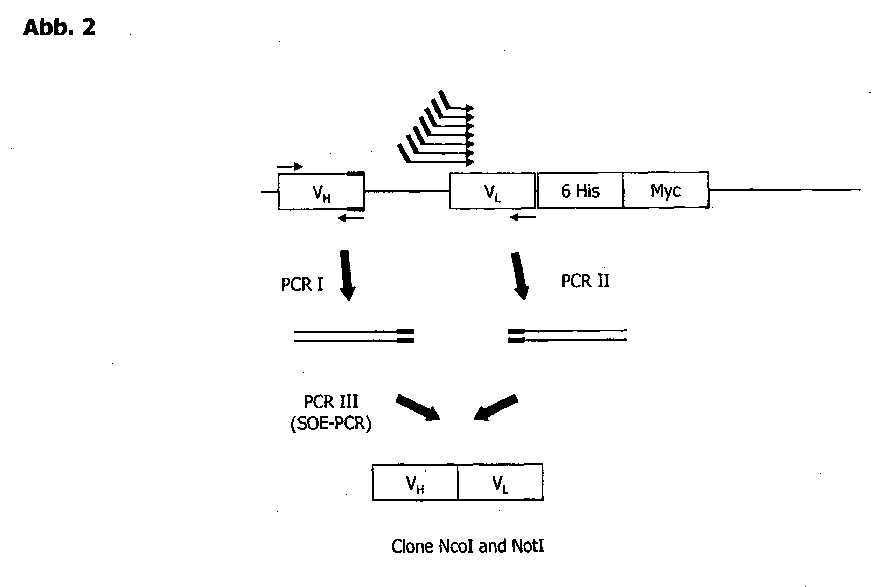

MUC1 spezifische scFv mit den Sequenzen SEQ ID NO. 36 bis 59 wurden durch PCR Amplifizierung und anschließender Klonierung der variablen Ketten in einen bakteriellen Expressionsvektor hergestellt. Dieser Vektor enthält den lacZ Promotor, eine Ribosomenbindungsstelle (RBS), das M13 origin, die pelB Signalsequenz zur Sekretion ins Periplasma, ein Ampicillin-Resistenzgen und eine Klonierungskassette, um an das C-terminale Ende des scFv ein Hexa-Histidin-Tag zur effizienten Aufreinigung und ein c-myc-Tag zu koppeln (Abb. 1). Für die verschiedenen Linkerlängen wurden die V_{H} und die V_{L} mit spezifischen Primern so amplifiziert, daß 22 Nukleotide am 3'-Ende der V_{H} und am 5'-Ende der V_{L} einen komplementären Bereich ausbilden (Abb. 2, PCR I und PCR II). Anschließend wurden die beiden PCR-Fragmente nach Aufreinigung in einer SOE-PCR miteinander verknüpft (Abb. 2, PCR III) und das PCR-Fragment über NcoI/NotI in den oben beschriebenen Vektor kloniert.

### 2. Bakterielle Expression und Aufreinigung der scFv, die das glykosylierte MUC1 Tumorepitop spezifisch erkennen

Die Antikörperfragmente aus Beispiel 1 wurden in *Escherichia coli* exprimiert und aufgereinigt. Hierfür wurde das entsprechende Plasmid durch Elektroporation in elektrokompetente *E. coli* transformiert und über Nacht in 2xTY Medium (10 g Hefeextrakt, 16 g Trypton, 5 g NaC1 per L) mit 100 *µ*g/mL Ampicillin kultiviert. Diese Kultur wurde 1:100 mit 2xTY Medium, dem 100 *µ*g/ml Ampicillin und 0,5% Glukose zugesetzt wurde, verdünnt und bei 37°C inkubiert, bis eine OD_{600 nm} von ca. 0, 6 erreicht war. Dann wurde der Kultur zur Induktion 1 mM IPTG zugesetzt und diese bei 25°C weitere 5 h inkubiert. Die Bakterien wurden durch Zentrifugation bei 4000xg für 20 min geerntet, das Zellpellet in TES Puffer (30 mM Tris-HCl, pH 8.0, 20% Saccharose, 1 mM EDTA) resuspendiert und 20 min auf Eis inkubiert. Anschließend wurden 5 mM MgSO₄ zugefügt und die Suspension für weitere 20 min auf Eis inkubiert. Durch Zentrifugation bei 4000 x g für 60 min wurde dann die Periplasmafraktion gewonnen und über Nacht bei 4°C gegen Bindungspuffer (50 mM Phosphatpuffer, pH 8.0, 300 mM NaCl, 10 mM Imidazol) dialysiert. Die in der Periplasmafraktion enthaltenen Antikörperfragmente wurden unter Verwendung des C-terminalen His-Tags durch Metallionen-Affinitätschromatographie (HiTrap Chelating HP, Amersham Pharmacia Biotech) aufgereinigt. Dafür wurde die dialysierte Fraktion auf die vorher mit Bindungspuffer äquilibrierte Säule gegeben und mit Waschpuffer (50 mM Phosphatpuffer, pH 8.0, 300 mM NaCl, 30 mM Imidazol) die nicht bindenden Proteine von der Säule gewaschen. Anschließend wurden die Antikörperfragmente mit Elutionspuffer (50 mM Phosphatpuffer, pH 8.0, 300 mM NaCl, 300 mM Imidazol) eluiert.

### 3. Klonierung der Vektoren zur Expression chimärer IgG und IgM Antikörper, die das glykosylierte MUC1 Tumorepitop spezifisch erkennen

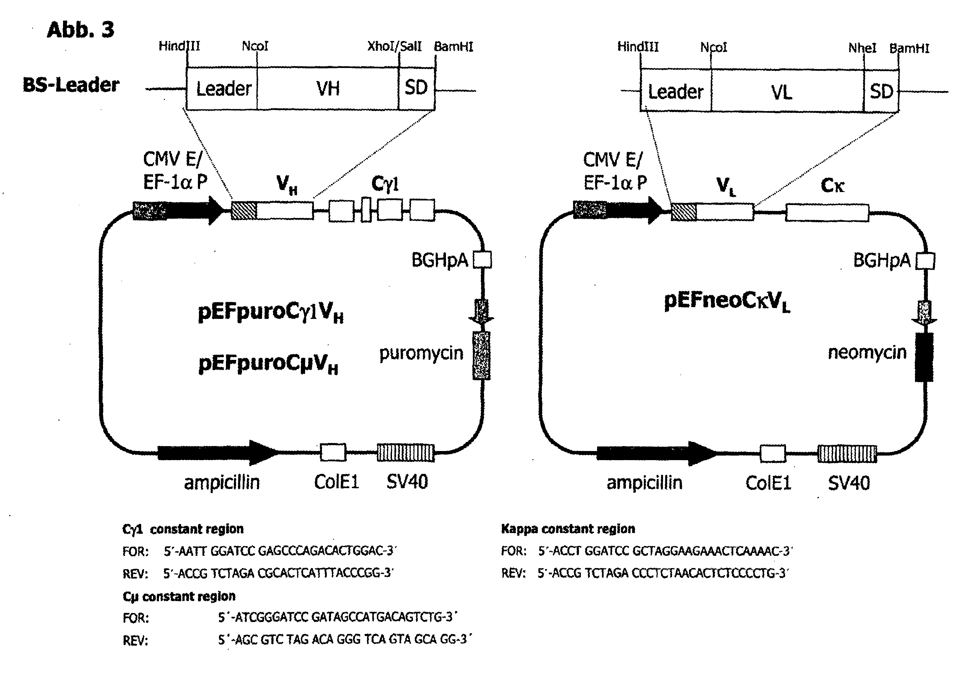

Das NcoI/XhoI DNA Fragment aus dem scFv Vektor, das für die V_{H} kodiert (Abb. 1), wurde in den NcoI/SalI geschnittenen. BS-Leader Vektor kloniert (Abb. 3). Der BS-Leader Vektor enthält eine Klonierungskassette zur Einführung der T-Zellrezeptor Signalpeptidsequenz an das 5'-Ende sowie einer Splice-Donor-Sequenz an das 3'-Ende der Sequenzen der variablen Domänen (Abb. 3). Die V_{L}-Sequenz des entsprechenden Antikörpers wurde mit spezifischen Primern zur Einführung der NcoI-Schnittstelle am 5'-Ende und der NheI-Schnittstelle am 3'-Ende in der PCR unter Verwendung der scFv Sequenz als Templat amplifiziert und nach NcoI/NheI Verdau in den gleich verdauten BS-Leader Vektor kloniert. Danach wurde jeweils das HindIII/BamHI Fragment aus dem BS-Leader Vektor in den entsprechenden eukaryontischen Expressionsvektor kloniert. Diese Vektoren (pEFpuroCγ1V_{H}, pEFpuroC*µ*V_{H} und pEFneoCκV_{L}) enthalten den EF-1α-Promotor und den HCMV-Enhancer, das SV40-origin, das BGH-Polyadenylierungssignal, das Puromycin-Resistenzgen im Vektor für die schwere Kette und das Neomycin-Resistenzgen im Vektor für die leichte Kette sowie die genomischen Sequenzen der humanen konstanten γ1 Region oder *µ* Region für die schwere Kette bzw. der humanen konstanten κ Region für die leichte Kette (Primer zur Amplifizierung aus genomischer humaner DNA und Vektorkarte siehe Abb. 3).

### 4. Eukaryontische Expression chimärer IgG und IgM Antikörper, die das glykosylierte MUC1 Tumorepitop spezifisch erkennen, in CHO Zellen und deren Aufreinigung

Zur Expression der chimären Antikörper cIgG-Pankol bestehend aus den Sequenzen SEQ ID NO. 64 und 68, cIgG-Panko2 bestehend aus den Sequenzen SEQ ID NO. 65 und 69, cIgM-Pankol bestehend aus den Sequenzen SEQ ID NO. 66 und 68 und cIgM-Panko2 bestehend aus den Sequenzen SEQ ID NO. 67 und 69 wurden CHOdhfr- Zellen (ATCC-NO. CRL-9096) mit einem Gemisch der Vektoren für die schwere und die leichte Kette (1:3) durch Elektroporation (10⁶ Zellen/ml, 500 V, 50 µs) cotransfiziert und in Selektionsmedium (CHO-S-SFM II Medium (Life Technologies), HT Supplement (Biochrom), 400 µg/ml G418, 5 µg/ml Puromycin) 2 Wochen kultiviert. Nach Einzelzellklonierung in einer 96-Loch-Platte wurden die Überstände im ELISA (glykosyliertes MUC1 Peptid (30-mer, siehe unten) als Antigen, anti human Fcγ1- POD gekoppelt bzw. anti human Fc5µ- POD gekoppelt (Dianova) als Sekundärantikörper) getestet und der Klon mit der höchsten Antikörperproduktionsrate selektioniert (ca. 0,5 µg/10⁶ Zellen/24 h).

Zur Antikörperproduktion wurden die stabil transfizierten, die chimären IgG bzw. IgM sekretierenden CHO Zellen in Spinnerflaschen oder Flaschenkultur in CHO-S-SFM II Medium, ergänzt durch HT Supplement, kultiviert, bis eine Zelldichte von ca. 1 x 10⁶ Zellen/ml erreicht war. Nach Abtrennung der Zellen vom Zellkulturüberstand durch Zentrifugation (400xg, 15 min) wurde der chimäre Antikörper unter Verwendung einer Protein A - Säule. (HiTrap rProtein A FF, Amersham Pharmacia Biotech) für die chimären IgG bzw. einer anti human Fc5µ- Antikörper-Affinitätssäule für den chimären IgM aufgereinigt. Die durch pH-Sprung eluierte gereinigte Antikörperfraktion wurde unter Verwendung von Centriprep-Zentrifugenröhrchen (cut off 50 kDa, Millipore) in PBS umgepuffert und aufkonzentriert.

### 5. Analyse der scFv und Antikörper, die das glykosylierte MUC1 Tumorepitop spezifisch erkennen, im ELISA

Als Antigene wurden verschiedene synthetische Peptide und Glykopeptide verwendet: ein nicht-glykosyliertes 30-mer mit der Sequenz APPAHGVTSAPDTRPAPGSTAPPAHGVTSA; ein glykosyliertes 30-mer mit der Sequenz APPAHGVTSAPDT[GalNAcα]RPAPGSTAPPAHGVTSA, eine Serie von nicht-glykosylierten MUC1-Peptiden unterschiedlicher Länge der Sequenz [VTSAPDTRPAPGSTAPPAHG]ₙ, wobei n = 1, 3 und 5 (TR1, TR3 und TR5) ist, und eine Serie von glykosylierten MUC1-Peptiden unterschiedlicher Länge der Sequenz A [HGVTSAPDT(GalNAcα)RPAPGSTAPPA]ₙ, wobei n = 1, 3 und 5 (TR1g, TR3g und TR5g) ist.

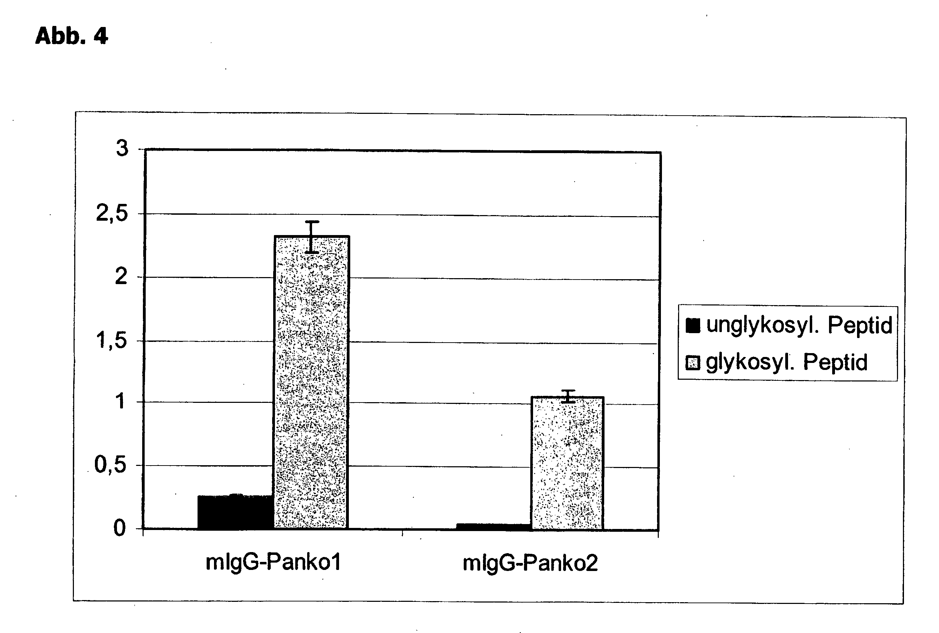

Aus den jeweiligen Stammlösungen (1 mg in 1ml Bi-dest. H₂O), die portioniert bei -20°C aufbewahrt werden, wurde eine Verdünnung von 1 µg/ml in PBS oder 10 µg/ml in Bi-dest. hergestellt. Davon wurden 50 µl/well in eine Mikrotiterplatte pipettiert und die Testplatte über Nacht inkubiert (für Peptide in PBS Verwendung von NUNC - Immuno plate F96 MAXISORP und Inkubation bei 4°C; für Peptide in Bi-dest Verwendung von Nunclon - TC plates und Antrocknung der Peptide bei 37°C). Am nächsten Tag wurde die Testplatte mit PBS/0, 2% Tween20 3x gewaschen. Anschließend wurden mit 2% BSA in PBS unspezifische Bindungsstellen blockiert und 50 µl des ersten Antikörpers aufgetragen (murine Antikörper: 10 - 10000 ng/ml PBS/1% BSA; chimärer IgG: 1 - 100 ng/ml in PBS/1% BSA; scFv: 50 - 500 ng/ml in PBS/1% BSA). Nach drei Waschschritten mit PBS/0,2% Tween20 wurden zum Nachweis der spezifisch gebundenen Antikörperkonstrukte die entsprechenden Zweit-Antikörper, Peroxidase-gekoppelt, eingesetzt (ein anti-Maus bzw. anti-human Fcyl für die ganzen Antikörper, ein anti-His Tag-Antikörper für scFv). Zum Nachweis des gebundenen sekundären Antikörpers erfolgte eine Farbreaktion mit TMB (3,3',5,5'-Tetramethylbenzidine). Nach ca. 15 Minuten wurde die Reaktion durch Zugabe von 2,5N H₂SO₄ abgestoppt. Die Messung erfolgte mit einem Mikrotiterplattenphotometer mit Filter 450nm im Dual-mode gegen einen Referenzfilter 630nm. Repräsentative Ergebnisse sind in den Abbildungen 4 bis 8 dargestellt.

### 5.1. Bindung an die glykosylierte PDTRP Region innerhalb einer MUC1 Tandem Repeat Sequenz

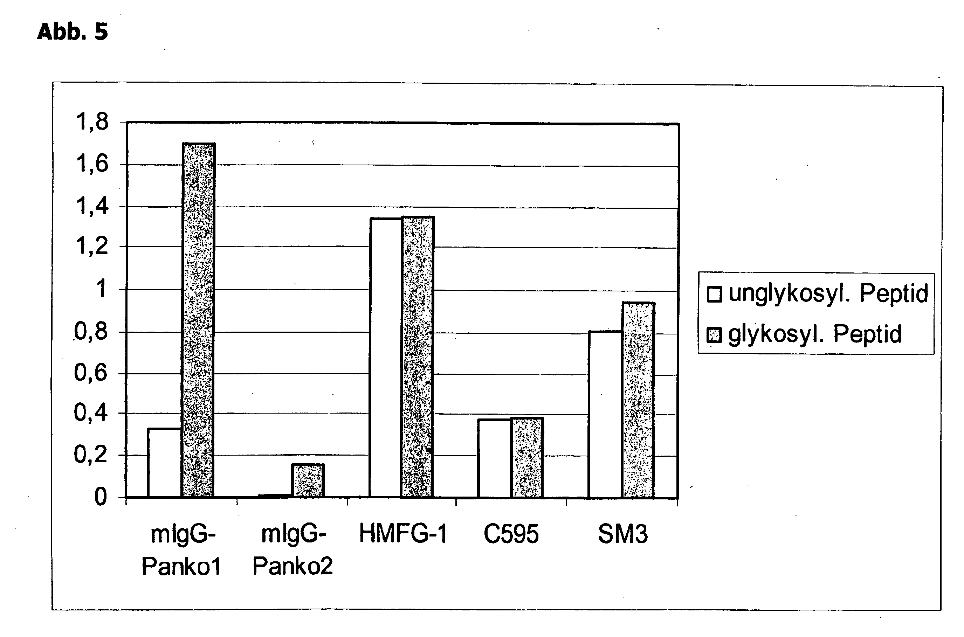

Abbildung 4 zeigt die Bindung der erfindungsgemäßen Erkennungsmoleküle bevorzugt an das glykosylierte MUC1 Peptid. Es sind zwei Erkennungsmoleküle mit variierenden Loop-Sequenzen im IgG-Format verglichen. Die Antikörperkonstrukte mIgG-Panko1 (SEQ ID NO. 60 und SEQ ID NO. 62) und mIgG-Panko2 (SEQ ID NO. 61 und SEQ ID NO. 63) binden hochspezifisch an das 30-mer, bevorzugt an das glykosylierte Peptid und nur schwach oder gar nicht an die nicht-glykosylierte Peptidsequenz. Im Vergleich zu mIgG-Panko1 und mIgG-Panko2 ist in Abbildung 5 die Bindung der MUC1-spezifischen Antikörper HMFG-1, C595 und SM3 an das nicht-glykosylierte und das glykosylierte 30-mer dargestellt (repräsentatives Beispiel). Die drei Antikörper HMFG-1, C595 und SM3 zeigen keine oder nur eine schwache Glykosylierungsabhängigkeit mit einem Faktor (Verhältnis glykosyliertes/nicht-glykosyliertes Peptid) für alle drei Antikörper von < 1,2. Die Antikörper dienen zur Abgrenzung und sind nicht Teil der erfindungsgemäßen Erkennungsmoleküle. Dagegen ergibt sich unter gleichen Bedingungen für den mIgG-Panko1 ein Faktor von > 4,5 (in Abb. 5: 5,1) und für den mIgG-Panko2 ein Faktor von > 20 (in Abb. 5: 22,1).

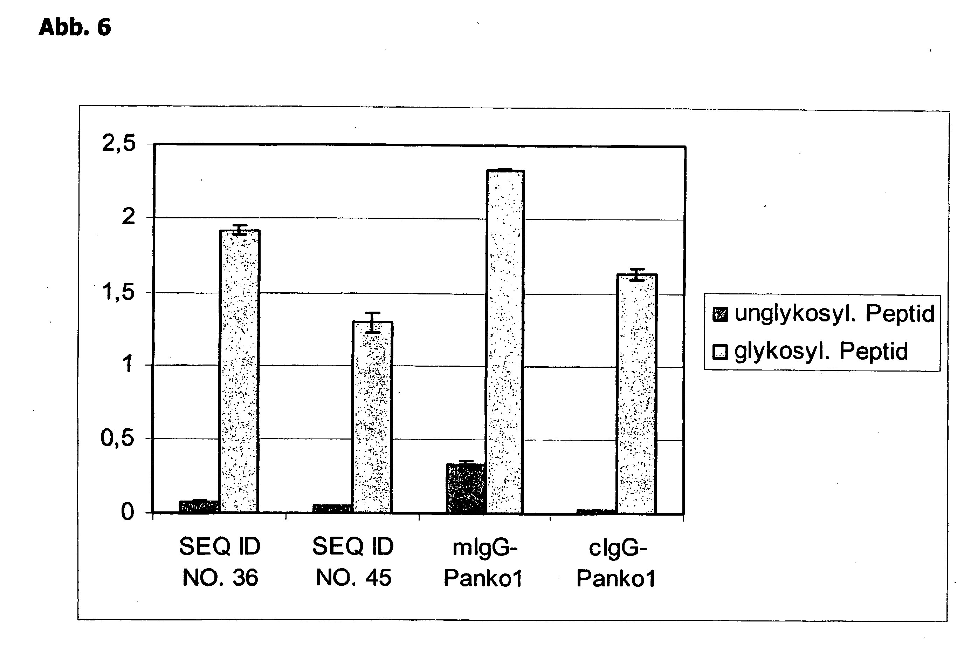

In Abbildung 6 sind verschiedene Formate der erfindungsgemäßen Erkennungsmoleküle in ihrer spezifischen Bindung bevorzugt an das glykosylierte MUC1-Peptid dargestellt.

### 5.2. Bindung an multiple nicht-glykosylierte MUC1 Tandem Repeats

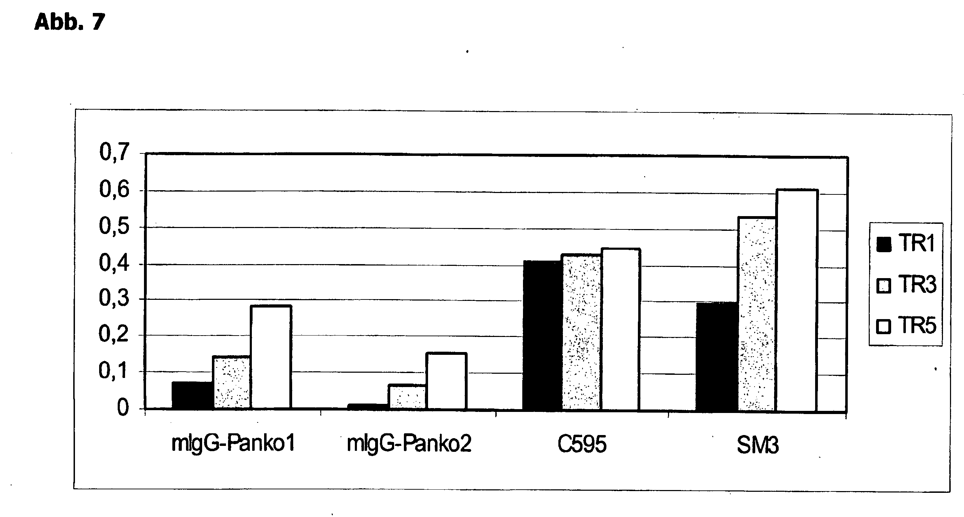

Abbildung 7 zeigt als repräsentatives Beispiel die Bindungsabhängigkeit der erfindungsgemäßen Erkennungsmoleküle mIgG-Panko1 und mIgG-Panko2 von der Anzahl der nicht-glykosylierten Tandem Repeats im Vergleich zu den MUC1-spezifischen Antikörpern C595 und SM3. Das Verhältnis des Signals mit dem TR5 Peptid zu dem mit dem TR1 Peptid stellt einen Faktor dar, der den Grad der Bindungsabhängigkeit von der Anzahl der Repeats wiederspiegelt. Für den mIgG-Panko1 und den mIgG-Panko2 ergeben sich Faktoren von > 3 (in Abb. 7: 3,9) bzw. > 8 (in Abb.7: 12,7), wohingegen die MUC1-spezifischen Antikörper unter gleichen Bedingungen C595 (Faktor 1,1) und SM3 (Faktor 2,1) keine oder nur eine geringe Abhängigkeit zeigen.

### 5.3. Bindung an multiple glykosylierte MUC1 Tandem Repeats

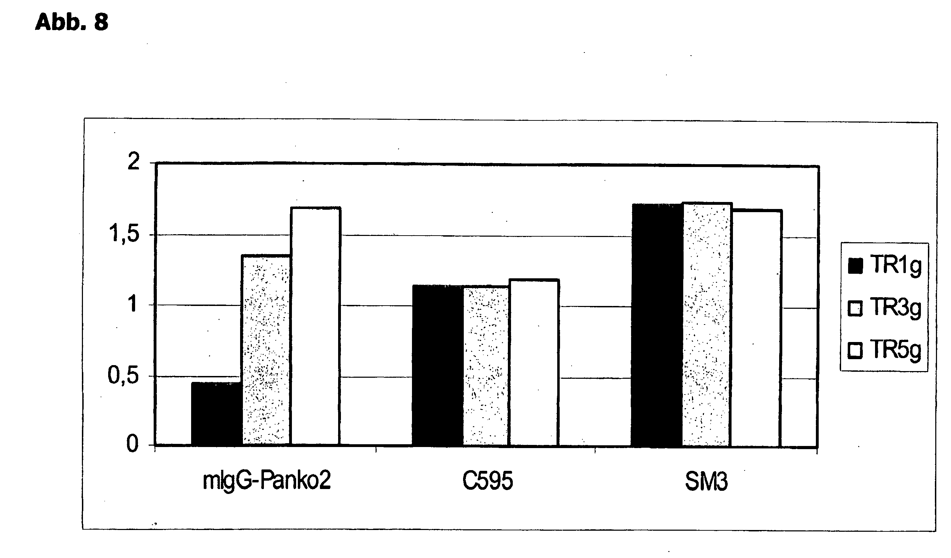

Abbildung 8 zeigt als repräsentatives Beispiel die Bindungsabhängigkeit des erfindungsgemäßen Erkennungsmoleküls mIgG-Panko2 von der Anzahl der Tandem Repeats (multiple glykosylierte PDTR-Regionen) im Vergleich zu den MUC1-spezifischen Antikörpern C595 und SM3. Der mIgG-Panko2 zeigt eine zusätzliche Abhängigkeit von der Anzahl glykosylierter Tandem Repeats, wohingegen die Bindung der MUC1-spezifischen Antikörper C595 und SM3 unabhängig von einer Erhöhung der Repeat-Anzahl ist.

### 6. Immunhistologische und immunzytologische Färbungen mit Erkennungsmolekülen, die das glykosylierte MUC1 Tumorepitop spezifisch erkennen

Für die immunhistologischen. Färbungen wurden Gefrierschnitte entsprechender Gewelaeproben luftgetrocknet und mit 10% Formaldehyd in PBS 15 min fixiert. Zur Reduktion der endogenen Peroxidase-Aktivität wurden die Schnitte mit 3% Wasserstoffperoxid in PBS behandelt und nach Blockierung unspezifischer Bindungsstellen mit Kaninchenserum mit einem erfindungsgemäßen MUC1-spezifischen Erkennungsmoleküls in Form eines Primärantikörpers inkubiert. Anschließend wurden die Präparate mit einem entsprechenden Sekundärantikörper (anti-Maus IgG POD gekoppelt) inkubiert. Die Farbreaktion erfolgte unter Verwendung des Peroxidase-Substrats Diaminobenzidin und die Gegenfärbung mit Hämatoxylin.

Das beispielhafte erfindungsgemäße Erkennungsmolekül mIgG-Panko2 reagiert nur mit wenigen Strukturen im Normalgewebe. Diese befinden sich hauptsächlich in immunpriveligierten Regionen des Körpers, beispielsweise auf der apikalen Oberfläche von Zellen bestimmter Drüsengänge, und sind somit in für einen Antikörper in vivo nicht oder nur sehr schlecht zugänglichen Bereichen (Tabelle 3). Darüber hinaus sind die Färbungen im normalen Brustgewebe im Vergleich zum Tumorgewebe schwach und befinden sich nur an der apikalen Membran.

**Tabelle 3: Reaktion von humanem Normalgewebe mit dem MUC1 spezifischen Antikörper mIgG-Panko2.**

| **Gewebetyp** | | **Reaktivität** |
|---|---|---|
| Epidermis | | |
| | Stratum basale | +/- |
| | Stratum spinosum | - |
| | Stratum granulosum | - |
| | Stratum corneum | - |
| | nicht epidermale Zellen | - |
| Magen | | |
| | Foveola Epithelium | - |
| | Fundusdrüsen | - |
| | Korpusdrüsen | - |
| Dünndarm | | |
| | Mucosa | - |
| Colon | | |
| | Mucosa | - |
| Brust | | |
| | Drüsengänge | + |
| | Azini | + |
| | myoepitheliale Zellen | - |
| Milz | | |
| | Trabeculae lienis | - |
| | Reti'kularzellen | - |
| | Lymphozyten | - |
| | Makrophagen | - |
| Endothelium | | - |
| Prostata | | + |
| Leber | | |
| | Hepatozyten | - |
| | Gallenwege | + |
| Niere | | |
| | Glomeruli | - |
| | Kapselepithelium | - |
| | Tubuli proximales | - |
| | Tubuli distalis | -/+ |
| | Sammelrohr | -/+ |
| Lymphknoten | | |
| | Lymphozyten | - |
| | Makrophagen | - |
| | Retikularzellen | - |
| Gallenblase | | |
| | Mucosa | + |
| Gehirn | | |
| | Neuronen | - |
| | Gliazellen | - |
| | Meninges | - |
| | Ependymzellen | - |
| Nebenniere | | |
| | Nebennierenrinde | - |
| | Nebennierenmark | - |
| Thymus | | |
| | Epitheliale Retikularzellen | - |
| | Hassall-Körperchen | -/+ |
| | Lymphozyten | - |
| | Makrophagen | - |
| Blase | | |
| | Urothelium | + |
| Herz | | |
| | Endocardium | - |
| | Myocardium | - |
| | Mesothelium | - |
| Bauchspeicheldrüse | | |
| | Drüsengänge | + |
| | Azini | + |
| | Langerhans' sche Inseln | - |
| Bindegewebe | | - |
| Synovialgewebe | | - |
| Muskelgewebe | | |
| | glatter Muskel | - |
| | Skelettmuskel | - |

Die beanspruchten Erkennungsmoleküle reagieren positiv mit einer Vielzahl von Tumoren. Die Daten in Tabelle 4 zeigen, dass die MUC1 spezifischen Erkennungsmoleküle einen großen Prozentsatz an Tumorpatienten einer Indikation erkennen, der von Indikation zu Indikation unterschiedlich ist. In Dickdarmadenomen korreliert die Färbung mit dem Grad der Dysplasie. Colonadenome reagieren nicht oder nur sehr schwach mit mIgG-Panko2, wohingegen Colonkarzinome positiv sind. Normales Pankreasgewebe ist nur schwach positiv, Pankreaskarzinome zeigen dagegen eine sehr starke Reaktion mit dem erfindungsgemäßen Erkennungsmolekül mIgG-Panko2.

**Tabelle 4: Reaktion von humanem Tumorgewebe mit mIgG-Panko2.**

| | **Gewebetyp** | | **% positive Fälle** |
|---|---|---|---|
| Colon | | | |
| | Adenome | | |
| | | tubulär | 25 |
| | | tubulär-villös | 25 |
| | | villös | 33 |
| | Transitorische Mucosa | | 30 |
| | Karzinome | | |
| | | Adenokarzinom | 90 |
| | | Papilläres Adenokarzinom | 67 |
| | | Muköses Adenokarzinom | 67 |
| | | Muzin-produzierendes | |
| | | Adenokarzinom | 100 |
| | | Siegelringzellkarzinom | 100 |
| | Lebermetastasen | | 81 |
| Magenkarzinome | | | 90 |
| Leberkarzinome | | | |
| | gut differenziert | | 14 |
| | mäßig differenziert | | 42 |
| | schwach differenziert | | 50 |
| Pankreaskarzinome | | | 100 |
| Mammakarzinome | | | |
| | Primärkarzinome | | 87 |
| | Metastasen | | 91 |
| Nierenzellkarzinome | | | 100 |

Für die immunzytologischen Färbungen wurde die Immunfluoreszenz eingesetzt. Dafür wurden die entsprechenden Zellen auf Objektträger angetrocknet und mit 5% Formaldehyd 10 min, fixiert. Nach Blockierung unspezifischer Bindungsstellen mit BSA (1% in PBS) wurden die Zellen mit dem erfindungsgemäßen Erkennungsmolekül in. Form von Primärantikörpern inkubiert. Anschließend wurde 3 mal mit PBS gewaschen und mit dem entsprechenden fluoreszenz-markierten Sekundärantikörper (anti-Maus IgG-FITC oder anti-human Fab-Cy2, Dianova) inkubiert. Nach mehrmaligem Waschen mit PBS wurden die Zellen in Mowiol eingebettet.

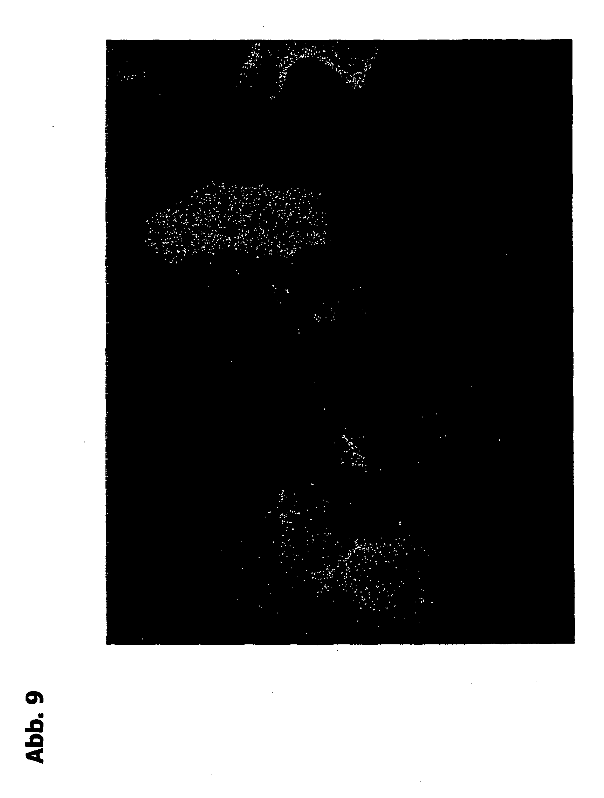

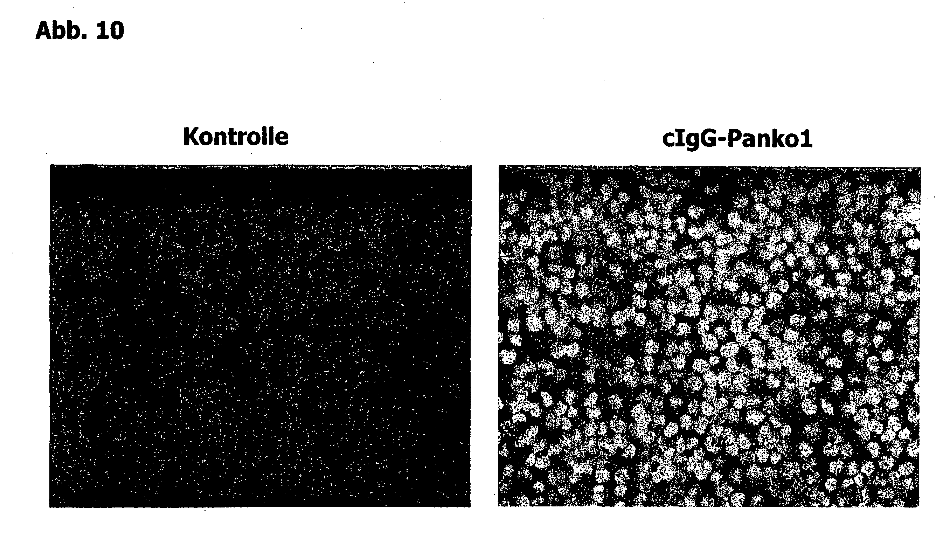

Es wurden verschiedene Zelllinien mit MUC1 spezifischen Erkennungsmolekülen in der Immunfluoreszenz getestet. Eine Reihe von Tumorzelllinien reagieren positiv (Tab. 5 und die Abb. 9 und 10).

**Tabelle 5: Reaktivität verschiedener Zelllinien mit dem MUC1 spezifischen Antikörper mIgG-Panko2.**

| **Zelllinien** | **Reaktivität** |
|---|---|
| ZR-75-1 | Positiv |
| T47D | Positiv |
| U266 | (positiv) |
| LN78 | Positiv |
| HT29 | Negativ |
| HCT15 | (positiv) |
| HepG2 | Negativ |
| K562 | Positiv |
| MCF-7 | Positiv |
| HEK293 | (positiv) |

Abbildung 9 zeigt beispielhaft eine Fluoreszenzmarkierung von T47D Zellen, einer Mammakarzinom-Zelllinie, mit mIgG-Panko2. Abbildung 10 zeigt beispielhaft eine Fluoreszenzmarkierung von K562 Zellen mit cIgG-Panko1.

Mit Hilfe der Immunhistologie und der Immunfluoreszenzmarkierung wurde ebenfalls die Neuraminidase-abhängigkeit des glykosylierten MUC1-Tumorepitops untersucht. Hierfür wurden die Schnitte bzw. die Zellen mit Neuraminidase (0,02 U/ml PBS + 0,01 M Ca²⁺) eine Stunde bei Raumtemperatur vorinkubiert, anschließend mit PBS gewaschen und dann wie oben markiert. Wie in den Tabellen 6 und 7 dargestellt ist die Bindung der erfindungsgemäßen Erkennungsmoleküle an das glykosylierte MUC1-Tumorepitop im Sinne der Erfindung Neuraminidase-unabhängig oder wird durch Neuraminidase-behandlung verstärkt.

**Tabelle 6: Immunhistologische Färbungen von humanem Gewebe mit dem mIgG-Panko2 vor und nach Neuraminidase-Behandlung.**

| | **Reaktivität unbehandelt** | **Reaktivität nach Neuraminidase-behandlung** |
|---|---|---|
| normales Colongewebe | - | - |
| Colonadenome | + | + |
| Colonkarzinome | ++ | ++ |
| Mammakarzinome | ++ | +++ |

**Tabelle 7: Immunfluoreszenzmarkierung von zwei Tumorzelllienien mit dem mIgG-Panko2 vor und nach Neuraminidase-Behandlung.**

| | **Reaktivität unbehandelt** | **Reaktivität nach Neuraminidase-behandlung** |
|---|---|---|
| K562 | + | ++ |
| ZR-75-1 | +++ | ++++ |

### 7. Chalatisierung und radioaktive Markierung von Antikörpern und Antikörperfragmenten, die das glykosylierte MUC1 Tumorepitop spezifisch erkennen

Durch Konjugation wurde an die Antikörper mIgG-Panko2 und cIgG-Panko1 bzw. an die scFv-Formate mit den Sequenzen SEQ ID NO. 36 und 45 ein Chelator kovalent gebunden, der die Bindung eines Radiometalls ermöglicht. Als Chelatoren kamen die kommerziellen Produkte der Firma Macrocyclics (Dallas, USA), p-isothiocyanatobenzyl-diethylenetriaminepentaacetic acid (p-SCN-Bz-DTPA) und p-isothiocyanatobenzyl -1,4,7,10-tetraazacyclododecane-1,4,7,10-tetraacetic acid (p-SCN-Bz-DOTA) zum Einsatz. Beide Chelatoren eignen sich zur Kopplung an Antikörper zu deren Radioaktivmarkierung [Brechbiel et al., 1986; Kozak et al., 1989; Stimmel et al., 1995].

Die Konjugation erfolgte durch Reaktion der Isothiocyanatgruppe des Chelators mit einer freien ε-Aminogruppe der Aminosäure Lysin am Antikörper. Es entsteht eine kovalente N-C-Bindung zwischen Chelator und Antikörper.

Der gereinigte Antikörper bzw. das gereinigte Antikörperfragment muß zunächst in Kopplungspuffer pH 8,7 umgepuffert werden. Hierzu wurde eine Ultrafiltration in einer Filtrationshülse (Centriprep YM-50 bzw. YM-10; Millipore) durchgeführt. Dies erfolgte durch mehrmalige Verdünnung mit 10-fachem Volumen und Filtration durch eine Membran mit definierter Porengröße durch Zentrifugation. Dadurch wurde PBS durch den alkalischen Kopplungspuffer (0,05 M Natrium-Carbonat, 0,15 M Natriumchlorid, pH 8,7) ersetzt.

Die Chelatisierung wurde mit den bifunktionellen Chelatoren p-SCN-Bz-DTPA- bzw. p-SCN-Bz-DOTA durchgeführt. Zur Chelatisierungsreaktion wurden Protein (1-10 mg/ml) in Kopplungspuffer und eine Lösung des Chelators von 1 mg/ml in 2% DMSO/Wasser so gemischt, dass ein molarer Überschuß des Chelators gewährleistet war. Es folgte eine Inkubation der Mischung von 1h bei 37°C. Anschließend wurde nicht gebundener Chelator durch Ultrafiltration im gleichen Gefäß (Centriprep YM-50 bzw. YM-10; Millipore) abgetrennt und wie oben beschrieben in den zur Radioaktivmarkierung notwendigen Beladungspuffer auf pH 4,2 umgepuffert (0,15 M Natriumacetat, 0,15 M Natriumclorid, pH 4,2). Die Proteinkonzentration während und nach diesem Schritt wurde wieder auf 1-10 mg/ml mit Hilfe einer UV-Messung bei 280nm eingestellt.

Es waren Bedingungen für die Chelatisierungsreaktion zu finden, die eine Radiomarkierung des Antikörpers erlauben, ohne dessen Bioaktivität wesentlich zu mindern.

Der chelatisierte Antikörper wurde mit einem Radiometall beladen, wodurch der Radio-Antikörper erzeugt wurde. Zur Beladung wurden die Isotope ¹¹¹Indium und ⁹⁰Yttrium verwendet. Beide haben chemisch und physikochemisch vergleichbare Eigenschaften und werden durch den Chelator als dreiwertige Ionen gebunden (¹¹¹In³⁺, ⁹⁰Y³⁺). Der mit ¹¹¹Indium markierte Antikörper ist ein γ-Strahler und wird in der Klinik zur individuellen Dosisfindung für den Patienten verwendet, während ⁹⁰Yttrium ein β-Strahler ist, der therapeutisch zum Einsatz kommt. Die Halbwertzeiten betragen für ¹¹¹In 67 Stunden und für ⁹⁰Y 64 Stunden.

Zur Beladung wurde ¹¹¹Indiumchlorid der Firma NEN (Perkin Elmer, Belgien) verwendet. Die Lieferung des Radiometalls erfolgt in salzsaurer Lösung. Diese ¹¹¹InCl₃ Lösung wurde zunächst kurzzeitig auf eine HCl-Konzentration von 1M gebracht. Anschließend wurde mit 0,05M HCl auf eine spezifische Aktivität von 80-320mCi/ml verdünnt und davon ein Aliquot zum Einbau in den chelatisierten Antikörper verwendet, wobei das zugegebene Volumen HCl-saurer¹¹¹InCl₃-Lösung gleich dem Volumen der vorgelegten Antikörperlösung im Kopplungspuffer pH 4,2 sein sollte, um die pH-Stabilität zu gewährleisten. Inkubationsdauer war 1h bei 37°C mit gelegentlichem vorsichtigem Mischen.

Im Anschluß daran wurde der Filtereinsatz wieder in die Filtrationshülse eingesetzt und wie oben beschrieben in Phosphatpuffer pH 7,2 mit physiologischem Gehalt an Kochsalz umgepuffert. Dabei erfolgte eine Trennung von hochmolekularem radioaktiv markiertem Antikörper und ungebundenem ¹¹¹InCl₃. Die Quantifizierung des Einbaus von ¹¹¹In in den chelatisierten Antikörper erfolgte dünnschichtchromatographisch. Die Einbaurate des Radiometalls lag bei 80-99% der eingesetzten Radioaktivität.

### 8. zellbindungsversuche und Scatchard Analyse von radioaktiv-markierten Erkennungsmolekülen, die das glykosylierte MUC1 Tumorepitop spezifisch erkennen

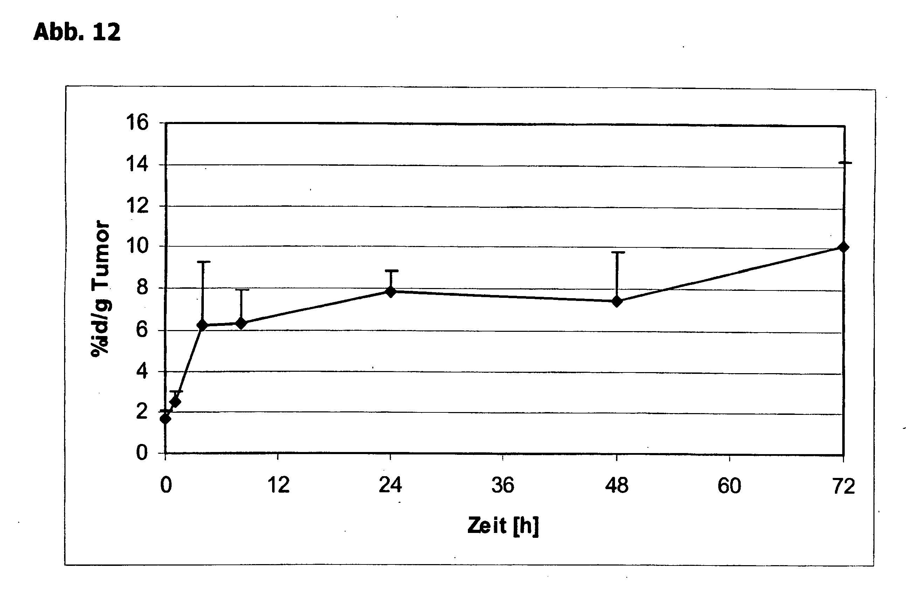

Verschiedene Tumorzelllinien wurden zur Testung der Zellbindungsfähigkeit der radiomarkierten Erkennungsmoleküle herangezogen. Dabei wurde jeweils in Dublettbestimmungen eine bestimmte Anzahl Zellen in einem 1,5 ml Gefäß vorgelegt und mit steigenden Mengen Antikörper inkubiert. Nach dem Waschen wurde anhand der Zählrate bestimmt, wieviel Antikörper gebunden hat. Dieser Wert wurde als Verhältnis gebunden/nichtgebunden gegen die Menge gebunden in ein Diagramm aufgetragen und im linearen Bereich der Kurve die Steigung ermittelt und der Abzissen-Schnittpunkt bestimmt (Scatchard Analyse s.u.). Pro Ansatz werden 1x10⁶ Zellen benötigt. Nach Vorinkubation der Zellen für eine Stunde auf Eis wurde die benötigte Menge Zellen in Reaktionsgefäße vorgelegt, zentrifugiert (5min 1000xg, 25°C) und der Überstand abgenommen. Dann wurde mit PBS/0,1% Tween20/1% BSA aufgefüllt bis zum Volumen 200µl abzüglich der noch zuzugebenen Menge Erkennungsmolekül. Anschließend wurde das entsprechende Erkennungsmolekül zum Endvolumen 200 µl zugegeben (ca. 40 bis 500 ng, je nach Erkennungsmolekül) und die Ansätze für eine Stunde bei 4-8°C inkubiert. Nach Zentrifugation (5min, 1000xg, 25°C) wurde der Überstand abgenommen und das Zellpellet in 500µl PBST/1%BSA vorsichtig resuspendiert. Nach nochmaligem Waschen wurde das Zellpellet im Gafäß am gamma-Counter gemessen. Von den Ausgangslösungen definierter Konzentration wurden die spezifischen Zählraten bestimmt, der Wert in cpm/ng wurde der Relativierung der Messwerte von gebundenem Antikörper zugrunde gelegt. Die Abzissenwerte sind in gebundene Moleküle Antikörper pro Zelle (r) aufgetragen. Der jeweilige Ordinatenwert ist der Quotient aus Bindung (r) zu freier Bindung, wobei die freie Bindung die Differenz aus Gesamtmenge und gebundener Menge Antikörper (in Abbildung 12 Angaben in M) darstellt. Der Abzissen-Schnittpunkt bezeichnet die Anzahl der Bindungsplätse/Zelle. Aus der Steigung der Geraden ergibt sich die Assoziationskonstante Kass in [M⁻¹].

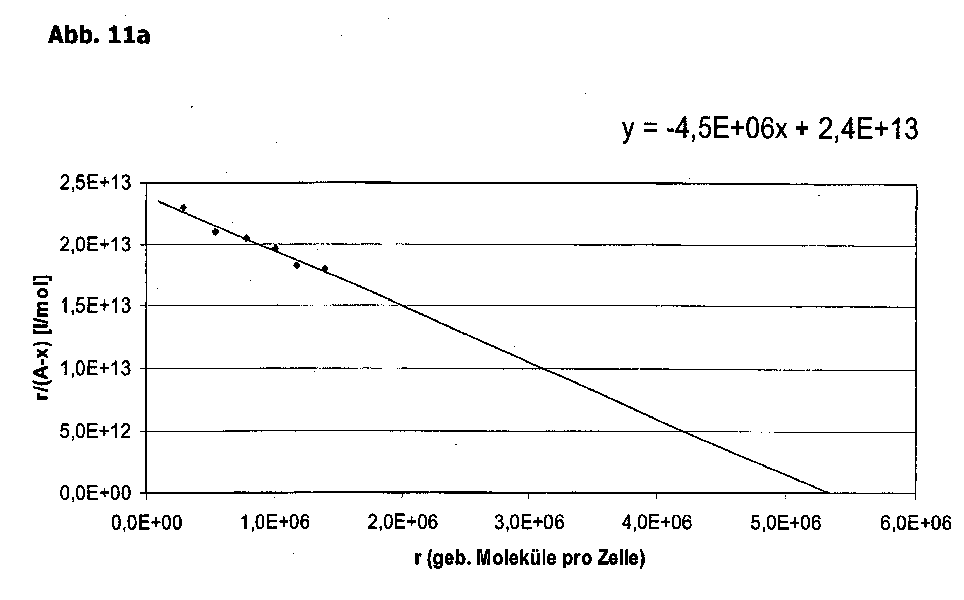

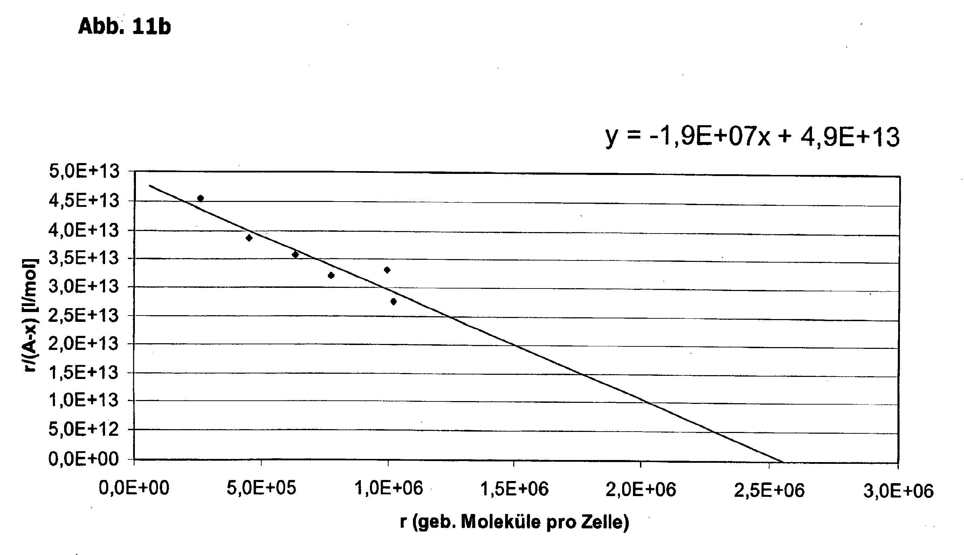

In Abbildung 11 sind beispielhaft die Scatchard-Analysen der Bindung der radioaktivmarkierten Erkennungsmoleküle im scFv-Format mit den Sequenzen SEQ ID NO. 36 (monovalenter scFv, Abb. 11a) und 45 (Multibody, Abb. 11b) an T47D Zellen dargestellt, wobei sich unter diesen Bedingungen für den monovalenten scFv eine Kass von 4,5 x 10⁶ M⁻¹ und 5,3 x 10⁶ Bindungsplätze/Zelle sowie für den Multibody eine Kass von 1,9 x 10⁷ M⁻¹ bzw. 2,6 x 10⁶ Bindungsplätze/Zelle ermitteln lassen.
In Tabelle 8 sind die Assoziationskonstanten und die Anzahl der Zellbindungsplätze verschiedener Antikörper und Antikörperformate an K562 Zellen zusammengefaßt und mit dem HMFG-1, der unter gleichen Bedingungen eingesetzt und getestet wurde, verglichen.

**Tabelle 8: Zellbindungstest und Scatchard-Analyse mit radioaktivmarkierten Erkennungsmolekülen an K562 Zellen.**

| **Antikörper** | **Kass [M-1]** | **Anzahl Bindungsplätze/Zelle** |
|---|---|---|
| mIgG-Panko2 | 9,5 x 10⁸ | 1,0 x 10⁹ |
| HMFG-1 | 2,7 x 10⁸ | 6,3 x 10⁴ |
| cIgG-Pankol | 1,2 x 10⁸ | 1,9 x 10⁵ |
| cIgG-Panko2 | 6,1 x 10" | 1,1 x 10⁵ |
| SEQ ID NO. 36 | 1,8 x 10⁷ | 5,5 x 10⁵ |
| SEQ ID NO. 46 | 4,9 x 10⁷ | 4,8 x 10⁵ |

### 9. Biodistribution der radioaktiv-markierten Erkennungs-moleküle, die das glyhosylierte MUC1-Tumorepitop spezifisch erkennen, in einem in vivo Tumormodell

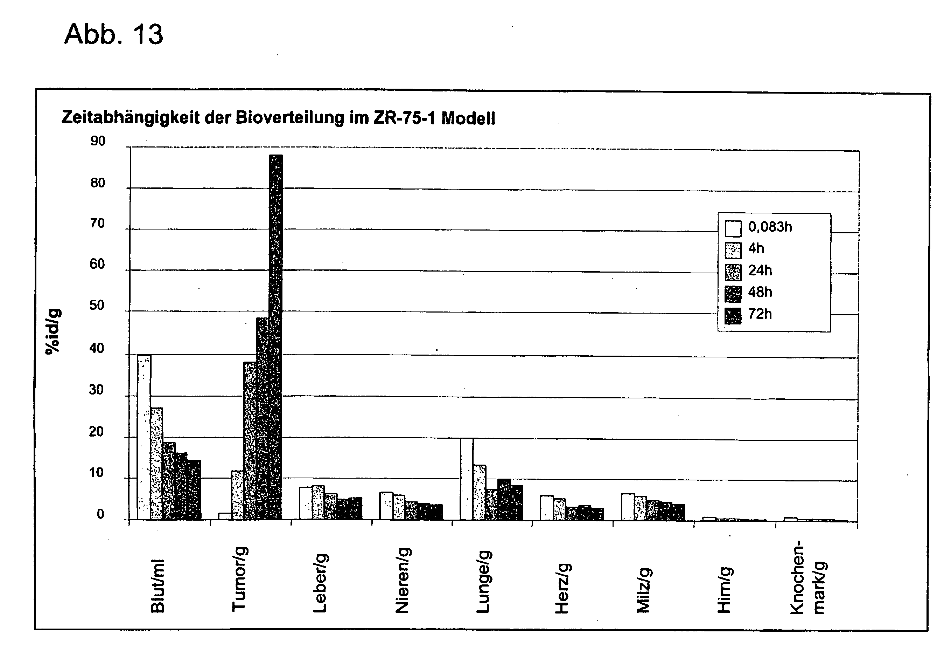

Als Tumormodell wurde humanes Colonkarzinomgewebe (Xenograft #5841) auf Nacktmäuse (Ncr: nu/nu, weiblich) subkutan transplantiert. Als ein weiteres Tumormodell wurden 10⁷ MUC1-positive ZR-75-1 Brustkrebszellen subkutan in Nacktmäuse (Ncr: nu/nu, weiblich) injiziert. Nach ca. 3-4 Wochen ist der Tumor unter der Haut tastbar. Den Tumor-tragenden Mäusen (pro Zeitpunkt n=5) wurden jeweils 5 µg ¹¹¹In-markierter mIgG-Panko2 in 200 µl i.v. in die Schwanzvene verabreicht. Nach 5min, 1h, 4h, 8h, 24h, 48h und 72h wurden die Mäuse getötet und die Verteilung der Radioaktivität im Tumor, im Serum und den Organen ermittelt. Die spezifische Anreicherung des mIgG-Panko2 im Tumor in %ID/g Tumor (bezogen auf die injizierte Dosis und das Tumorgeweicht) im Gewebetransplantat-Modell ist in Abbildung 12 dargestellt. Im Gegensatz dazu findet in den Organen und im Serum eine Abreicherung der Radioaktivität im angegebenen Zeitverlauf statt. In Abbildung 13 sind die Ergebnisse mit dem ZR-75-1-Modell zusammengefaßt. Die spezifische Aufnahme des radioaktiv-markierten mIgG-Panko2 im Tumor erreicht nach 72 h über 85%ID/g Tumor, wobei die Anreicherung in den Organen und im Serum sehr niedrig und vergleichbar mit gesunden Mäusen ist.

### 10. Nachweis des Tumor-spezifischen, sekretorischen MUC1 im Sandwich-ELISA mit Erkennungsmolekülen, die das glykosylierte MUC1 Tumorepitop spezifisch erkennen

Tumor-spezifisches, sekretorisches MUC1 kann im Sandwich-ELISA nachgewiesen werden. Dabei dient ein MUC1 spezifischer Antikörper als Fänger-Antikörper von MUC1 und ein erfindungsgemäßes Erkennungsmolekül zum Nachweis des Tumor-spezifischen MUC1. Um Core-1 positives, sekretorisches MUC1 nachzuweisen, dient beispielsweise ein erfindungsgemäßes Erkennungmolekül als Fänger und ein Core-1 spezifischer Antikörper zum Nachweis des Core-1 Antigens. Ein dritter Enzym- oder Fluoreszenz-gekoppelter Antikörper muss zur Detektion des Zweitantikörpers eingesetzt werden. Als Beispiel wurden die Überstände von drei Tumorzelllinien analysiert (K562, ZR-75-1 und T47D). Die Ergebnisse sind in Tabelle 9 dargestellt. 10⁵ Zellen pro ml Zellkulturmedium wurden ausgesät, 4 Tage ohne Mediumwechsel kultiviert, anschließend ein Aliquot abgenommen und der Zellkulturüberstand durch Zentrifugation vom Zellpellet getrennt. 50 µl dieser Überstände wurden unverdünnt im ELISA eingesetzt. Der Sandwich-ELISA wurde durchgeführt, indem die Mikrotiterplatte mit dem Fänger-Antikörper (HMFG-1 oder mIgG-Panko2, 1 µg/ml) in PBS über Nacht bei 4 °C beschichtet wurde. - Es wurden drei verschiedenen Konzentrationen des Antikörpers für die Beschichtung getestet (1 µg/ml, 2 µg/ml und 4 µg/ml). Die Beschichtung mit 1 µg/ml erwies sich im Sandwich-ELISA als am empfindlichsten. - Anschließend wurden die beschichteten Platten zweimal mit PBS gewaschen und 1,5 h in 5% BSA, 0,05% Tween 20 in PBS bei Raumtemperatur blockiert. Der Blockierungspuffer wurde entfernt, die Platten erneut einmal mit 0,1% Tween 20 in PBS (Waschpuffer) gewaschen, die Proben dazugegeben und 1,5 h bei Raumtemperatur inkubiert. Als Negativkontrollen wurden das Zellkulturmedium oder 2% BSA in Waschpuffer (Verdünnungspuffer für Zweitantikörper) verwendet. Eine Positivkontrolle stand nicht zur Verfügung. Für den MUC1-Core-1-Sandwich-ELISA erfolgte nach dreimaligem Waschen die Neuraminidase-Behandlung in den dafür vorgesehenen Wells. Zu diesem Zweck wurde die Neuraminidase-Lösung (DADE Behring, Deutschland) in Immidazolpuffer (0,68 g Immidazol, 0,19 g CaCl₂ und 0,4 g NaCl in 100 ml H₂O, pH 6,8) 1:5 verdünnt und 50 µl/well 30 min bei 37 °C inkubiert. Als Kontrolle wurde der Imidazolpuffer ohne Neuraminidase-Lösung in einem entsprechenden Well inkubiert. Anschließend wurden die Wells dreimal gewaschen und der biotinylierte mIgG-Panko2 (EZ-Link Sulfo-NHS-LC-Biotin, Pierce) bzw. ein anti-Core-1 Antikörper (mIgM-Karo4) zum Nachweis des MUC1 bzw. des Core-1 tragenden MUC1 in 2% BSA in Waschpuffer dazugegeben und eine weitere Stunde bei Rauintemperatur inkubiert. Nach erneutem dreimaligen Waschen folgte die Zugabe von Peroxidase-gekoppeltem Strepavidin bzw. eines anti-IgM(µ) Antikörpers Peroxidase-gekoppelt (Dianova) 1:300 bzw. 1:5000 verdünnt in 2% BSA in Waschpuffer und eine Inkubation von 1 h bei Raumtemperatur. Abschließend wurden die Platten zweimal in Waschpuffer und einmal in PBS gewaschen. Die Färbereaktion erfolgte in 25 mM Zitronensäure, Phosphatpuffer pH 5,0 mit 0,04% H₂O₂ und 0,4 mg/ml o-Phenylendiamin (Sigma) im Dunkeln bei Raumtemperatur. Die Farbreaktion wurde durch Zugabe von 2,5 N Schwefelsäure (Endkonzentration 0,07 N) gestoppt und im ELISA-Reader bei 492 nm mit einem Referenzfilter von 620 nm vermessen.

**Tabelle 9: Analyse von Tumor-spezifischem, sekretorischem MUC1 in Kulturüberständen zweier Zelllinien und Detektion von Core-1 vor und nach Neuraminidase Behandlung im Sandwich ELISA.**

| **Fänger Ab** | **HMFG-1** | **mIgG-Panko2** | |
|---|---|---|---|
| **Nachweis-Ab Zelllinie** | **mIgG-Panko2** | **anti-core-1 (mIgM-Karo4)** | |
| | | **- Neuraminidase** | **+ Neureminidase** |
| K562 | + | - | + |
| ZR-75-1 | +++ | - | + |
| T47D | ++ | - | +++ |

### 11. Nachweis von MUC1 im Serum von Colonkarzinom-Patienten durch Erkennungsmoleküle, die das glykosylierte MUC1 Tumorepitop spezifisch erkennen

Zum Nachweis von löslichem MUC1 in Patienten-Seren wurde ein Sandwich-ELISA wie oben beschrieben verwendet. Als Fänger-Antikörper wurde der anti-MUC1 Antikörper 115D8 (Fänger im kommmerziellen CA 15-3 Test) oder der mIgG-Panko2 (1 µg/ml PBS) verwendet. Nach dem Blocken mit 2% BSA/PBS wurden die Seren aufgetragen. Es wurden 24 verschiedene Colonkarzinom-Patientenseren in verschiedenen Verdünnungen (unverdünnt bis 1:32 verdünnt) untersucht. Als Standard dienten Verdünnungen eines definierten Serums eines Mammakarzinom-Patienten (152 U/ml, Enzymun-Test CA 15-3, Boehringer Mannheim). Als Grenzwert wurden 23 U/ml (in der Literatur Durchschnittswert für Normalseren) festgelegt. Zum Nachweis des gebundenen MUC1 wurde der mIgG-Panko2 mit zwei anderen anti-MUC1 Antikörpern, dem DF3 (Nachweisantikörper im CA 15-3 Test) und dem HMFG-1, verglichen. Alle drei Antikörper wurden biotinyliert (EZ-Link Sulfo-NHS-LC-Biotin, Pierce) eingesetzt und nach dreimaligem Waschen mit PBS + 0,1% Tween20 mit Streptavidin-POD gekoppelt (1:300 in 0,2% BSA/PBS) und TMB als Peroxidase-Substrat (vgl. oben) nachgewiesen.

In Tabelle 10 sind die Daten zusammengefaßt. Löslichem MUC1 im Serum von Colonkarzinom-Patienten wird mit dem erfindungsgemäßen Erkennungsmolekül mIgG-Panko2 deutlich weniger gebunden als mit den bekannten anti-MUC1 Antikörpern DF3 und HMFG-1.

**Tabelle 10: Nachweis von Serum-MUC1 im ELISA unter Verwendung verschiedener anti-MUC1 Antikörper.**

| **Fänger-antikörper** | **Nachweis-antikörper** | **Proben über Grenzvert/Gesamtproben** | **%** |
|---|---|---|---|
| 115D8 | DF3 | 20/24 | 83 |
| 115D8 | HMFG-1 | 19/24 | 79 |
| 115D8 | mIgG-Panko2 | 8/24 | 33 |
| mIgG-Panko2 | mIgG-Panko2 | 8/24 | 33 |
| | | 1/12 | 8 |

### 12. Tumortherapie zur Reduktion von MUC1-positiven Tumoren mit radioaktiv-markierten Erkennungsmolekülen, die das glykosylierten MUC1-Tumorepitop spezifisch erkennen, in einem in vivo Tumormodell

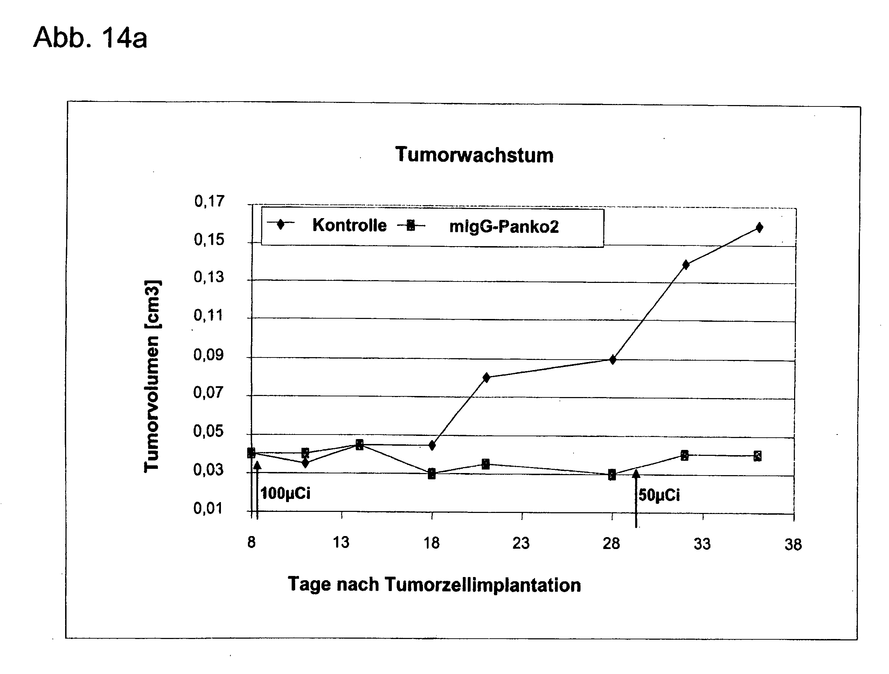

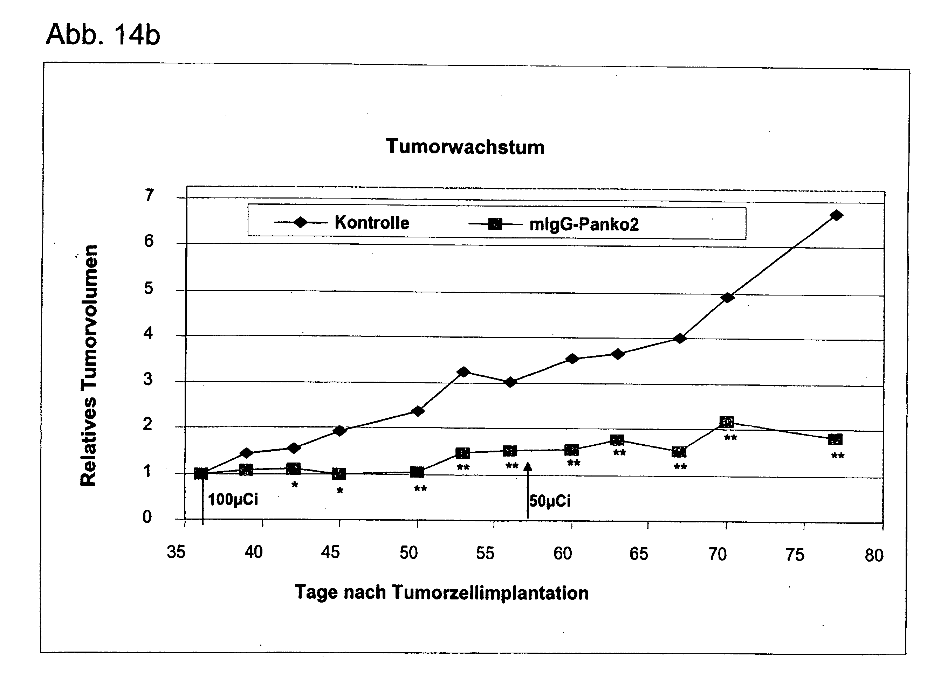

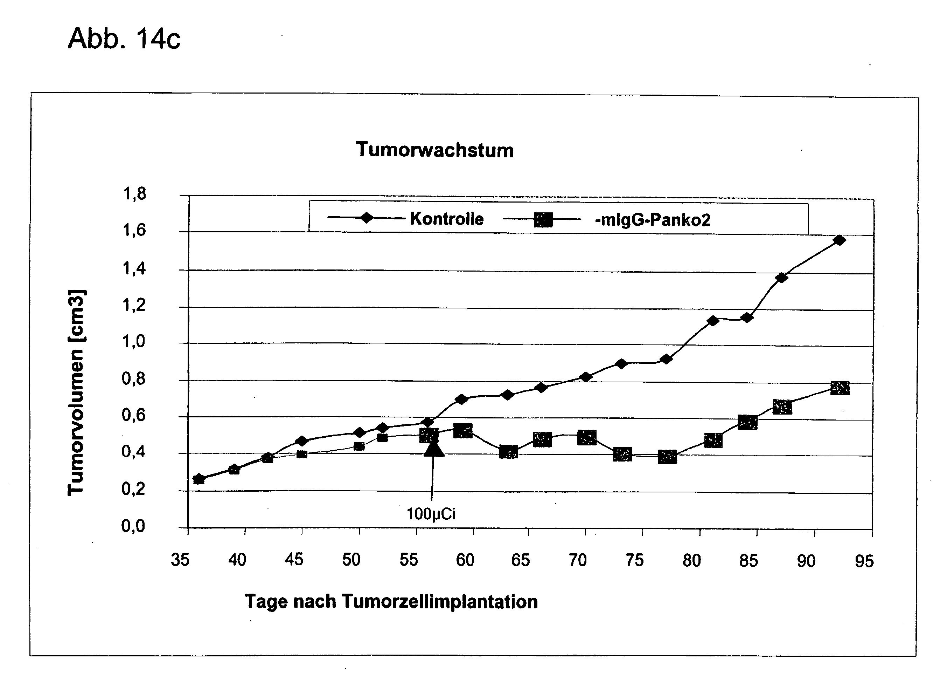

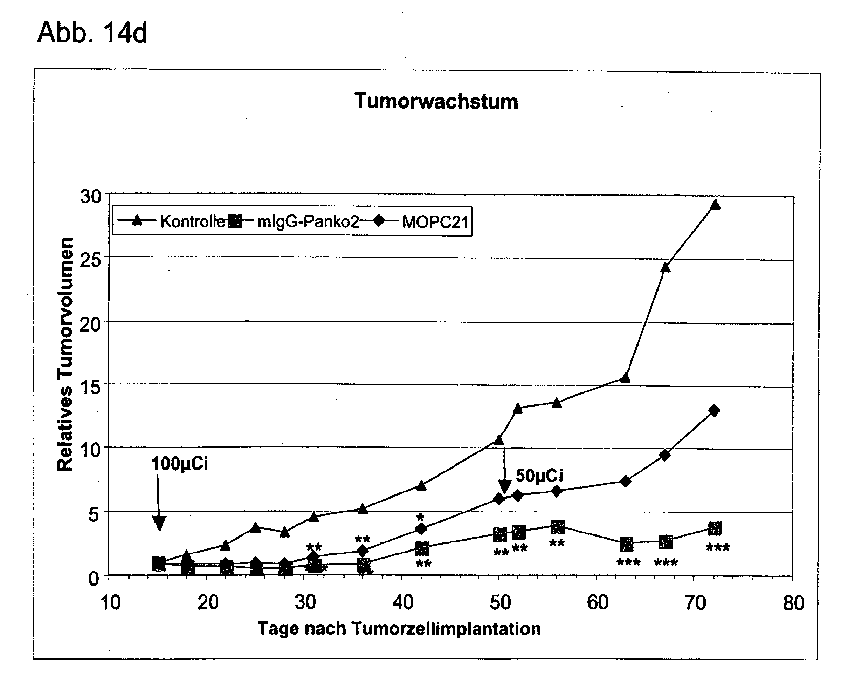

Das therapeutische Potential des mIgG-Panko2 wurde im ZR-75-1-Tumormodell (siehe Beispiel 9) untersucht. Die chelatisierten Erkennungsmoleküle (siehe Beispiel 7) wurden hierfür mit ⁹⁰Yttrium (ein β-Strahler zur Zerstörung der Tumorzellen) beladen (pH 4,5, 37°C, 30 min; vergleiche Einbau von ¹¹¹Indium) und in der Dünnschichtchromatographie die Stabilität kontrolliert.
Eine Woche bis acht Wochen nach der subkutanen Injektion der ZR-75-1 Zellen (je nach gewünschter Tumorgröße als Modelle zur Behandlung solider mittlerer (ca. 0,3 cm³) oder großer Tumore (> 0,5 cm³) oder zur Behandlung der minimalen Resterkrankung(< 0,05 cm³)) wurden den Tumor-tragenden Mäusen 200 µl i.v. in die Schwanzvene verabreicht. Die Injektionslösung enthielt den ⁹⁰Y-markierten mIgG-Panko2 (100 µCi pro Dosis; spezifische Aktivität 3 mCi/mg Antikörper) in Ca/Mg-PBS mit 4% fötalem Kälberserum zum Schutz vor Radiolyse. Kontrollgruppen erhielten die gleiche Injektion ohne radioaktiv-markiertem Erkennungsmolekül bzw. einen radioaktiv-markierten Kontrollantikörper (⁹⁰Y-MOPC21).
Körpergewicht und Tumorgröße wurden zweimal wöchentlich gemessen und verglichen. Das relative Tumorwachstum wurde unter Berücksichtigung der jeweiligen Tumorgröße zu Beginn der Behandlung bestimmt. Eine zweite Injektion (50 µCi pro Dosis) wurde ca. drei Wochen nach der ersten Behandlung injiziert. Diese Art der Behandlung führte in allen untersuchten Tieren zu einem starken Anti-Tumor-Effekt. Bei der Behandlung von kleinen (< 0,05 cm³) oder mittleren Tumoren (ca. 0,3 cm³) konnte damit das Tumorwachstum über den gesamten Beobachtungszeitraum (6 Wochen) komplett inhibiert werden (Abb. 14a und 14b). In Tieren mit sehr großen Tumoren (> 0,5 cm³) zu Beginn der Behandlung wurde das Tumorwachstum für ungefähr drei Wochen unterdrückt (Abb. 14c). Eine zweite Injektion konnte aufgrund der nicht mehr zumutbaren Tumorgröße in der Kontrollgruppe nicht erfolgen.
Die Behandlung mit einem irrelevanten ⁹⁰Y-markierten Antikörper (MOPC21) in gleicher Dosierung führt nur zu einer geringen Reduzierung des Tumorwachstums verglichen mit unbehandelten Tieren (Abb. 14d), was auf die unspezifische Bestrahlung durch eine relativ langsame Abreicherung des Antikörpers aus dem Serum zurückgeführt werden kann.
Außer einer geringen Myelotoxizität zeigte die Behandlung mit dem ⁹⁰Y-markierten mIgG-Panko2 keine Nebenwirkungen.

### 13. Nachweis der Antikörper-abhängigen Zellzytotoxizität der Erkennungsmoleküle, die das glykosylierte MUC1-Tumorepitop spezifisch erkennen, in einem in vitro Modell

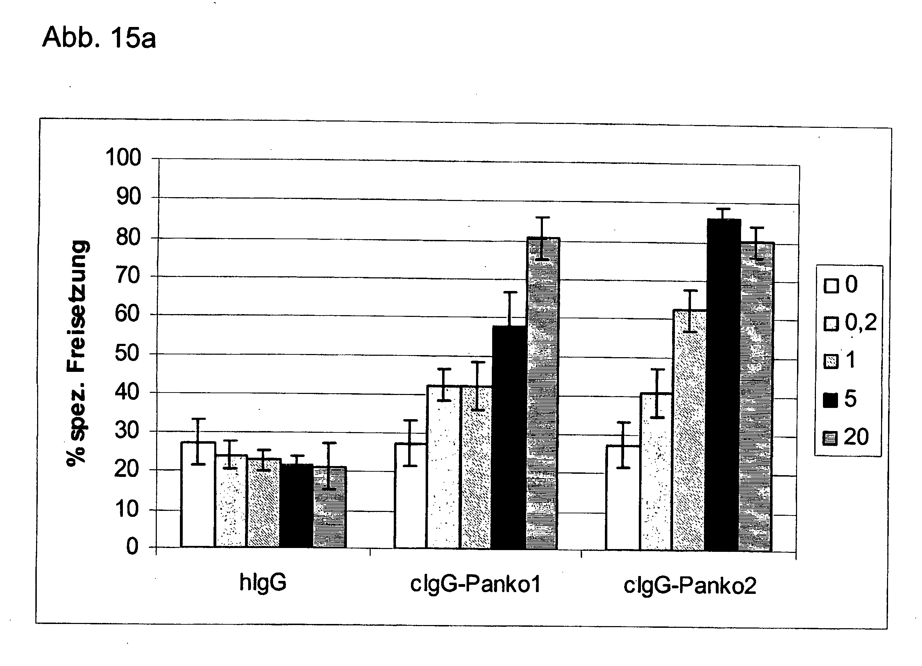

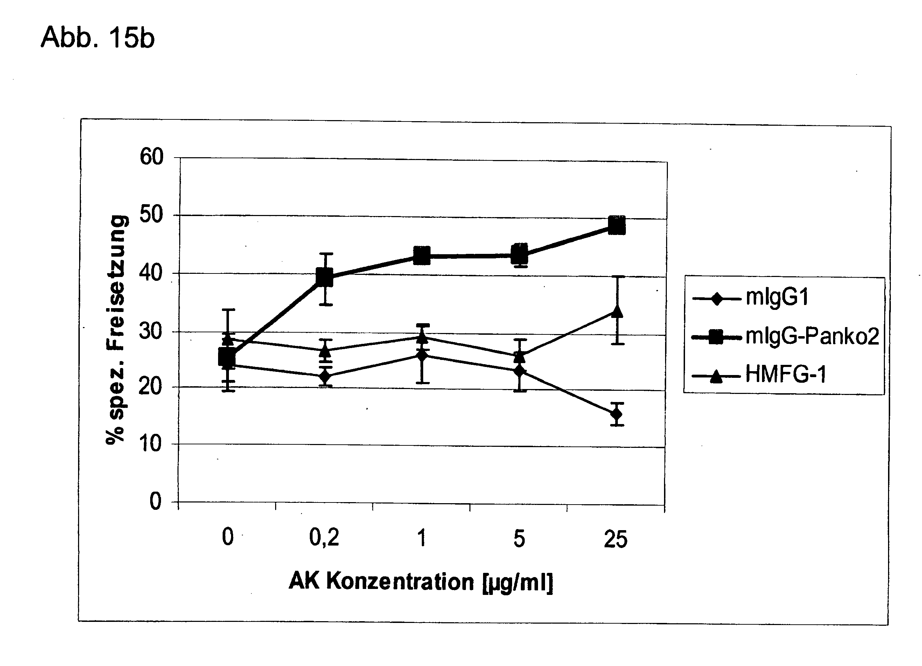

Die Antikörper-abhängigen Zellzytotoxizität (ADCC) der erfindungsgemäßen Erkennungsmoleküle wurde in einem Europium-Freisetzungstest untersucht. Die Zielzellen (ZR-75-1 oder MCF-7; 5 x 10⁶) wurden für 10 Minuten bei 4°C in 800 µl Europiumpuffer (50 mM HEPES, pH 7.4, 93 mM NaCL, 5 mM KC1, 2 mM MgCl₂, 10 mM Diethylentriamin-pentaessigsäure, 2 mM Europium(III)-Azetat) inkubiert, in einem Multiporator (Eppendorf) elektroporiert (710 V, 1 Puls, 30 µs) und anschließend weitere 10 min auf Eis inkubiert. Dann wurden die Zellen 5 mal in RPMI 1640/5% FKS gewaschen und in einer 96-Loch-Rundbodenplatte (Nunc) ausgesät (5 x 10³ in 100 µl pro Loch). Nach der Zugabe von 20 µl des mIgG-Panko2, des HMPG-1, des cIgG-Pankol oder des cIgG-Panko2 in verschiedenen Konzentrationen (0,2 bis 25 µg/ml Endkonzentration in 200 µl Inkubationsvolumen) bzw. der entsprechenden Kontrollen (Medium, Isotypkontrolle hIgG) wurden als Effektorzellen humane periphere Blutzellen (80 µl/Loch, Präparation siehe Beispiel 14)) zugegeben, wobei ein Verhältnis Effektorzelle/Zielzelle von 5 bis 100 zu 1 eingesetzt wurde. Zur Bestimmung der spontanen Freisetzung wurden 80 µl RPMI/FKS ohne Effektorzellen zugegeben. Die maximale Freisetzung wurde nach kompletter Lyse der Zielzellen mit Ethanol bestimmt. Nach 4 bis 20-stündiger Inkubation im Brutschrank bei 37°C wurde die Platte bei 500xg 5 Minuten zentrifugiert und jeweils 20 µl Probe in 200 µl pro Loch Enhancement-Lösung (PerkinElmer Wallac) pipettiert. Nach einer Inkubation von 15 Minuten bei Raumtemperatur wurde die Fluoreszenz bestimmt (Victor² Fluorometer, PerkinElmer Wallac). Die spezifische Zytotoxizität ergibt sich aus der Gleichung (experimentelle, Lyse - spontane Lyse)/(maximale Lyse - spontane Lyse).
Die chimären IgG des Panko1 und Panko2 zeigen eine hohe spezifische Lyse der MUC1 positiven Zielzellen (Abb. 15a). Der murine IgG des Panko2 zeigt eine etwas geringere Zytotoxizität als die chimären Erkennungsmoleküle, aber einen deutlich höheren Effekt als der murine anti-MUC1-Antikörper HMFG-1, für den nur in der höchsten getesteten Konzentration eine schwache Lyse zu beobachten ist (Abb. 15b).

### 14. Analyse der Bindung von Erkennungsmolekülen, die das glykosylierte MDC1-Tumorepitop spezifisch erkennen, an humane Blutzellen

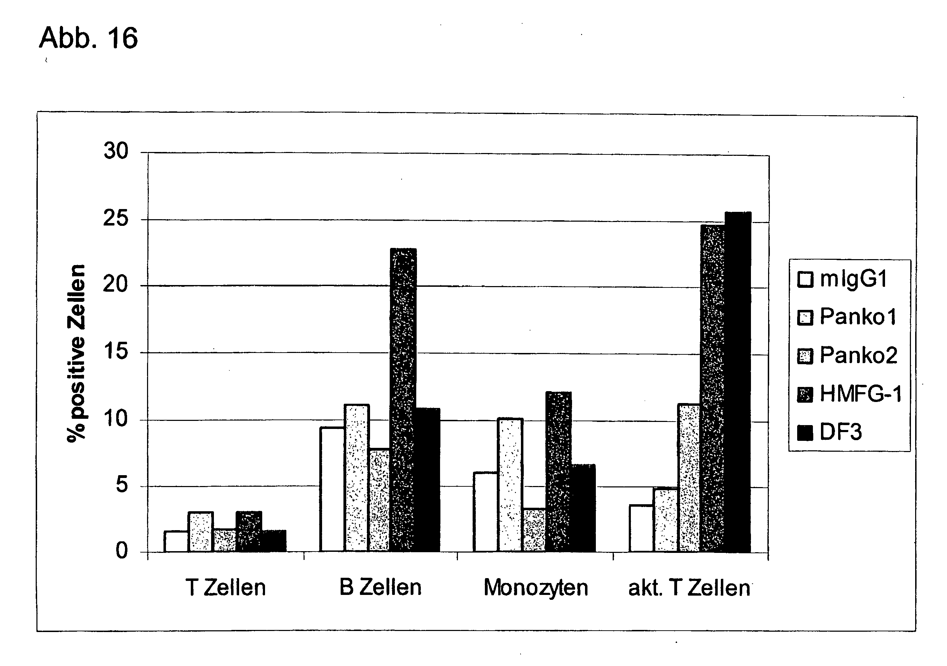

Eine Expression von MUC1 auf hämatopoetischen Zellen konnte durch verschiedene anti-MUC1 Antikörper nachgewiesen werden. Allerdings lassen unterschiedliche Ergebnisse mit verschiedenen anti-MUC1 Antikörpern darauf schließen, daß die Erkennung von nicht-epithelialem MUC1 stark von der Feinspezifität des jeweiligen Antikörpers abhängig ist und ein variierendes Glykosylierungsmuster des MUC1 auf den Zellen vorliegt. Die Bindung der erfindungsgemäßen Erkennungsmoleküle an humane Blutzellen wurde in der Durchflußzytometrie untersucht.
Humane periphere Blutzellen wurden aus dem Blut von gesunden Spendern mittels Dichte-Zentrifugation gewonnen (Ficoll-Hypaque). Die Zellschicht wurde abgenommen und 3 mal mit RPMI 1640/5% FKS gewaschen. Die Zellen wurden entweder frisch verwendet oder kryokonserviert und vor der Verwendung in der Zellfärbung oder dem Einsatz als Effektorzellen im ADCC-Test (siehe Beispiel 13) in RPMI 1640/10% FKS aufgetaut. Zur Stimulierung humaner T-Zellen wurden die isolierten Blutzellen in RPMI/FKS + 1 µg/ml PHA + 60 U/ml hIL-2 3 bis 8 Tage im Brutschrank inkubiert. Die Stimulierung wurde in der Durchflußzytometrie durch Nachweis des Aktivierungsmarkers CD25 kontrolliert.
Die isolierten Blutzellen wurden mit mIgG-Panko1, mIgG-Panko2, HMFG-1 oder DF3 und als Kontrolle mit einer mIgG1-Isotypkontrolle für eine Stunde auf Eis inkubiert. Nach dem Waschen mit PBS wurden die Zellen mit Cy3-konjugiertem Ziege anti-Maus Ig (dianova) und/oder mit einem CD-Marker spezifischen (CD3, CD14, CD19), FITC-markierten Antikörper inkubiert (30 min, 4°C, im Dunkeln). Nach dem Waschen wurden die Zellen in der Durchflußzytometrie analysiert. Keiner der getesteten Antikörper bindet an nicht-aktivierte T-Zellen. Nach PHA-Stimulierung wird die MUC1-Expression auf humanen T-Zellen hochreguliert. Dieses MUC1 wird stark durch den HMFG-1 und den DF3 detektiert und nur gering durch den Panko2. Der Panko1 zeigt keine Bindung an PHA-stimulierte Blutzellen (Abb. 16). Panko2 und DF3 erkennen weder das MUC1 auf Monozyten noch auf B-Zellen. Im Gegensatz dazu binden HMFG-1 und Panko1 schwach an Monozyten und der HMFG-1 auch an B-Zellen (Abb. 16), wobei die detektierten Oberflächendichten deutlich niedriger sind als die auf aktivierten T-Zellen.

### Legende zu den Abbildungen

| | |
|---|---|
| **Abb. 1****:** | **Vektor zur Klonierung und bakteriellen Expression von single chain Antikörperfragmenten.** |
| **Abb. 2****:** | **Klonierungsschema zur Herstellung von single chain Antikörperfragmenten mit unterschiedlicher Linkerlänge.** |
| **Abb. 3****:** | **Vektorsystem zur Klonierung und eukaryontischen Expression von chimären Antikörpern im IgG1 oder IgM-Format.** |
| **Abb. 4****:** | **Bindung der erfindungsgemäßen Erkennungsmoleküle mIgG-Panko1 und mIgG-Panko2 an glykosyliertes und nicht-glykosyliertes MUC1 Peptid im ELISA.** Als Antigene wurde das nicht-glykosylierte 30-mer mit der Sequenz APPAHGVTSAPDTRPAPGSTAPPAHGVTS und das glykosylierte 30-mer mit der Sequenz APPAHGVTSAPDT[GalNAcα]RPAPGSTAPPAHGVTSA verwendet und in PBS an die Platte gebunden. Die Antikörper mIgG-Panko1 und mIgG-Panko2 wurden in einer Konzentration von 0,5 µg/ml im ELISA eingesetzt. |
| **Abb. 5****:** | **Vergleich der spezifischen Bindung der anti-MUC1 Antikörper HMFG-1, C595 und SM3 mit mIgG-Panko1 und mIgG-Panko2 an glykosyliestes und nicht-glykosyliertes MUC1 Peptid im ELISA.** Als Antigene wurde das nicht-glykosylierte 30-mer mit der Sequenz APPAHGVTSAPDTRPAPGSTAPPAHGVTS und das glykosylierte 30-mer mit der Sequenz APPAHGVTSAPDT[GalNAcα]RPAPGSTAPPAHGVTSA verwendet und in H₂O an die Platte angetrocknet. Die Antikörper wurden in einer Konzentration von 10 µg/ml im ELISA eingesetzt. |
| **Abb. 6****:** | **Spezifische Bindung verschiedener bevorzugter Formate erfindungsgemäßer Erkennungsmoleküle im ELISA, beispielhaft am nicht-glykosylierten und glykosylierten 30-mer MUC1 Peptid.** Als Antigene wurde das nicht-glykosylierte 30-mer mit der Sequenz APPAHGVTSAPDTRPAPGSTAPPAHGVTS und das glykosylierte 30-mer mit der Sequenz APPAHGVTSAPDT[GalNAcα]RPAPGSTAPPAHGVTSA verwendet und in PBS an die Platte gebunden. Die beiden scFv Formate SEQ ID NO. 36 und 45 wurden mit 0,5 µg/ml, der murine IgG mit 0,1 µg/ml und der chimäre IgG mit 0,01 µg/ml eingsetzt. Da für die verschiedenen Formate unterschiedliche Sekundärantikörper verwendet werden, sind die ELISA Daten nur qualitativ zu bewerten. |
| **Abb. 7****:** | **Abhängigkeit der Bindung der erfindungsgemäßen Erkennungsmoleküle mIgG-Panko1 und mIgG-Panko2 von der Anzahl der Tandem Repeats im nicht-glykosylierten MUC1 Peptiden im Vergleich zu den MUC1-spezifischen Antikörpern SM3 und C595 im ELISA.** Als Antigene wurde eine Serie von glykosylierten Mull-peptiden unterschiedlicher Länge der Sequenz [VTSAPDTRPAPGSTAPPAHG]ₙ, wobei n = 1, 3 und 5 (TR1, TR3 und TR5) ist, verwendet und in H₂O an die Platte angetrocknet. Die Antikörper wurden in einer Konzentration von 10 µg/ml eingesetzt. |
| **Abb. 8****:** | **Abhängigkeit der Bindung des erfindungsgemäßen Erkennungsmoleküls mIgG-Panko2 von der Anzahl der Tandem Repeats (multiple glykosylierte PDTR-Regionen) im Vergleich zu den MUC1-spezifischen Antikörpern SM3 und C595 im ELISA.** Als Antigene wurde eine Serie von glykosylierten MUC1-Peptiden unterschiedlicher Länge der Sequenz A[HGVTSAPDT(GalNAcα)RPAPGSTAPPA]ₙ, wobei n = 1, 3 und 5 (TR1g, TR3g und TR5g) ist, verwendet und in H₂O an die Platte angetrocknet. Die Antikörper wurden in einer Konzentration von 10 µg/ml eingesetzt. |
| **Abb. 9****:** | **Fluoreszenzmarkierung von Zellen der Tumorzelllinie T47D (Mammakarzinom) mit dem MUC1 spezifischen Erkennungsmolekül mIgG-Panko2.** |
| **Abb. 10****:** | **Fluoressensmarkierung von Zellen der Tumorzelllinie K562 (erythroide Leukämie) mit** |
| | **dem MUC1 spezifischen Erkennungsmolekül cIgG-Panko1.** |
| **Abb. 11****:** | **Scatchard-Diagramm zur Analyse der Zellbindung radioaktiv markierter MUC1-spezifischer Erkennungsmoleküle.** Beispielhaft sind hier die Bindungsdaten der beiden scFv Formate SEQ ID NO. 36 (a) und SEQ ID NO. 45 (b) dargestellt. r: gebundene Moleküle pro Zelle, A: eingesetzte Konzentration des radioaktiv markierten Erkennungsmoleküls [M], x: an die Zellen gebundener Anteil [M]. Die Differenz A-x stellt die Konzentration der freien Erkennungsmoleküle im Ansatz dar. Oben ist die entsprechende Geradengleichung angegeben, wobei der Geradenanstieg die Assoziationskonstante wiedergibt. |
| **Abb. 12****:** | **Spezifische Anreicherung des radioaktiv-markierten Erkennungsmoleküls mIgG-Panko2** im **Tumor in einem Maus-Xenotransplantat-Modell.** Den Tumor-tragenden Mäusen (pro Zeitpunkt n=5) wurden jeweils 5 µg ¹¹¹In-markierter mIgG-Panko2 i.v. verabreicht. Nach den angegebenen Zeitpunkten wurden die Mäuse getötet und die Anreicherung im Tumor, bezogen auf die injizierte Dosis und das Tumorgewicht (%ID/g), bestimmt. |
| **Abb. 13****:** | **Hohe spezifische Anreicherung des radioaktiv-markierten Erkennungsmoleküls mIgG-Panko2** im **Tumor in einem Maus-ZR-75-1-Tumorzell-Modell.** |
| | Den Tumor-tragenden Mäusen (pro Zeitpunkt n=6) wurden jeweils 5 µg ¹¹¹In-markierter mIgG-Panko2 i.v. verabreicht. Nach den angegebenen Zeitpunkten wurden die Mäuse getötet und die Anreicherung im Tumor, im Serum und den Organen, bezogen auf die injizierte Dosis und das Tumor- bzw. Organgewicht (%ID/g), bestimmt. |
| **Abb. 14****:** | **Inhibition des Tumorwachstums in Tumor-tragenden Mäusen nach Behandlung mit dem radioaktiv-markierten Erkennungsmolekül mIgG-Panko2.** Am 8. Tag (14a; kleine Tumore: < 0,05 cm³), am 36. Tag (14b; mittlere Tumore: ca. 0,3 cm³) bzw. am 57 Tag (14c; große Tumore: > 0,5 cm³) nach der subkutanen Injektion der ZR-75-1 Zellen wurden den Tumor-tragenden Mäusen 200 µl i.v. in die Schwanzvene verabreicht. Die Injektionslösung enthielt den ⁹⁰Y-markierten mIgG-Panko2 (100 µCi pro Dosis; spezifische Aktivität 3 mCi/mg Antikörper) in Ca/Mg-PBS mit 4% fötalem Kälberserum zum Schutz vor Radiolyse. Kontrollgruppen erhielten die gleiche Injektion ohne radioaktiv-markiertem Erkennungsmolekül. Bei kleinen und mittleren Tumoren erfolgte eine zweite Injektion (50 µCi) ca. 3 Wochen später. Abb. 14d zeigt die Behandlung Tumor-tragender Mäuse mit dem ⁹⁰Y-mIgG-Panko2 im Vergleich zu einem irrelevanten radioaktiv-markierten Kontrollantikörper ⁹⁰Y-MOPC21. |
| **Abb. 15****:** | **Spezifische Ermittlung der Antikörper-abhängigen Zellzytotoxizität durch die erfindungsgemäßen Erkennungsmoleküle Panko1 und Panko2**. Die Antikörper würden in einer Konzentration von 0,2 bis 25 µg/ml eingsetzt. (a) Die cIgG-Formate des Panko1 und Panko2 zeigen eine hohe spezifische Lyse der MUC1 positiven ZR-75-1 Zellen. (b) Der mIgG-Panko2 vermittelt ebenfalls eine spezifische Tumorzelllyse und zeigt einen deutlich höheren Effekt als der murine anti-MUC1-Antikörper HMFG-1, für den nur in der höchsten getesteten Konzentration (25 µg/ml) eine schwache Lyse zu beobachten ist. |
| **Abb. 16****:** | **Analyse der Bindung der erfindungsgemäßen Erkennungsmoleküle an humane Blutzellen.** Humane periphere Blutzellen wurden aus dem Blut von gesunden Spendern mittels Dichte-Zentrifugation gewonnen. Zur Stimulierung humaner T-Zellen wurden die isolierten Blutzellen in RPMI/FKS + 1 µg/ml PHA + 60 U/ml hIL-2 3 bis 8 Tage im Brutschrank inkubiert. Die isolierten Blutzellen wurden mit mIgG-Panko1, mIgG-Panko2, HMFG-1, DF3 oder mIgG1 (Kontrolle) und mit Cy3-konjugiertem Ziege anti-Maus Ig (dianova) und/oder mit einem CD-Marker spezifischen (CD3, CD14, CD19) FITC-markierten Antikörper gefärbt. Nach dem Waschen wurden die Zellen in der Durchflußzytometrie analysiert. |
| | Die Erkennungsmoleküle Panko1 und Panko2 zeigen keine oder nur eine geringe Bindung an humane Blutzellen. Im Gegensatz dazu binden die MUC1-spezifischen Antikörper HMFG-1 und DF3 stark an PHA-stimulierte T-Zellen und der HMFG-1 auch an B-Zellen und Monozyten. |

### Referenzen

Boel E et al. J Immunol Methods 239: 153-66 (2000).
Brechbiel MW et al. Inorg Chem 25: 2772-2781 (1986).
Chothia C et al. Science 233, 755-758 (1986).
Chothia C et al. Nature 342, 877-883 (1989).
Chothia C et al. J Mol Biol 227, 799-817 (1992).
Chothia C & Lesk AM. J Mol Biol 196, 901-917 (1987).
Dai J et al. Tumor Biol 19 (suppl 1): 100-110 (1998).
Herrera AM et al. Biochem Biophys Res Com 273: 557-559 (2000).
Hinoda Y et al. Gastroenterol Jpn 27: 390-395 (1992).
Jensen KB et al. Biochem Biophys Res Com 298: 566-573 (2002).
Kozak RW et al. Cancer Res 49: 2639-2644 (1989).
Liao et al. Biotechnol Bioeng 73, 313-323 (2000).
Martin ACR & Thornton JM. J Mol Biol 263, 800-815 (1996).
Nuttall SD et al. Proteins 36, 217-227 (1999).
Nygren PA & Uhlen M. Cur Opin Struc Biol 7: 463-469 (1997).
Rooman MJ et al. Protein Eng 3, 23-27 (1989).
Skerra A. J Mol Recog 13, 167-187 (2000).
Stimmel JB et al. Bioconjug Chem 6: 219-225 (1995).
Tonye Libyh M. et al. Blood 90(10): 3978-83 (1997).
US 5,506,343
US 5,683,674
US 5,804,187
US 6,315,997
WO 0244217
WO 9320841
Wu S & Cygler M. J Mol Biol 229, 597-601 (1993).

### Sequenzliste

### CDR Sequenzen

| | |
|---|---|
| SEQ ID NO. 1 | DAWMD |
| SEQ ID NO. 2 | NYWMN |
| | |
| SEQ ID NO. 3 | EIRSKANNHATYYAESVKG |
| SEQ ID NO. 4 | EIRLKSNNYTTHYAESVKG |
| | |
| SEQ ID NO. 5 | GGYGFDY |
| SEQ ID NO. 6 | HYYFDY |
| | |
| SEQ ID NO. 7 | RSSQSIVHSNGNTYLE |
| SEQ ID NO. 8 | RSSKSLLHSNGITYFF |
| | |
| SEQ ID NO. 9 | KVSNRFS |
| SEQ ID NO. 10 | QMSNLAS |
| | |
| SEQ ID NO. 11 | FQGSHVPLT |
| SEQ ID NO. 12 | AQNLELPPT |

### CDR Sequenzen (canonical structure variants)

| | |
|---|---|
| SEQ ID NO. 13 | NYWVN |
| SEQ ID NO. 14 | NYWIN |
| SEQ ID NO. 15 | NYWYN |
| SEQ ID NO. 16 | NYWWN |
| SEQ ID NO. 17 | DAWID |
| SEQ ID NO. 18 | DAWVD |
| SEQ ID NO. 19 | DAWYD |
| SEQ ID NO. 20 | DAWWD |
| | |
| SEQ ID D10. 21 | EIRSKANNYATYYAESVKG |
| SEQ ID NO. 22 | EIRLKSNKYTTHYAESVKG |
| SEQ ID NO. 23 | EIRLKSNSYTTHYAESVKG |
| | |
| SEQ ID NO. 24 | RPSQSIVHSNGNTYLE |
| SEQ ID NO. 25 | RSSQSIVHSNGNTYFE |
| SEQ ID NO. 26 | RPSQSIVHSNGNTYFE |
| SEQ ID NO. 27 | RPSKSLLHSNGITYFF |
| SEQ ID NO. 28 | RSSKSLLHSNGITYLF |
| SEQ ID NO. 29 | RPSKSLLHSNGITYLF |
| | |
| SEQ ID NO. 30 | FQGSHPPLT |
| SEQ ID NO. 31 | AQNLEPPPT |

### variable schwere Ketten

SEQ ID NO. 32
SEQ ID NO. 33

### variable leichte Ketten

SEQ ID NO. 34
SEQ ID NO. 35

### VH/VL Paarungen

| | |
|---|---|
| Panko1 | SEQ ID NO. 32 |
| | SEQ ID NO. 34 |
| | |
| Panko2 | SEQ ID NO. 33 |
| | SEQ ID NO. 35 |

### verschiedene single chain Fv Formate

SEQ ID NO. 36
SEQ ID NO. 37
SEQ ID NO. 38
SEQ ID NO. 39
SEQ ID NO. 40
SEQ ID NO. 41
SEQ ID NO. 42
SEQ ID NO. 43
SEQ ID NO. 44
SEQ ID NO. 45
SEQ ID NO. 46
SEQ ID NO. 47
SEQ ID NO. 48
SEQ ID NO. 49
SEQ ID NO. 50
SEQ ID NO. 51
SEQ TD NO. 52
SEQ ID NO. 53
SEQ ID NO. 54
SEQ ID NO. 55
SEQ ID NO. 56
SEQ ID NO. 57
SEQ ID NO. 58
SEQ ID NO. 59

### Murine Antikörper

### leichte Kette eines murinen IgG1 (kappa Kette)

SEQ ID NO. 60
SEQ ID NO. 61

### schwere Kette muriner IgG1

SEQ ID NO. 62
SEQ ID NO. 63

### murine Antikörper

mIgG-Panko1 SEQ ID NO. 60
   SEQ ID NO. 62
mIgG-Panko2 SEQ ID NO. 61
   SEQ ID NO. 63

### Chimäre Antikörper (Maus/Mensch)

### schwere Kette chimärer IgG1 (gamma Kette)

SEQ ID NO. 64
SEQ ID NO. 65

### schwere Kette eines chimären IgM (mu Kette)

SEQ ID NO. 66
SEQ ID NO. 67

### leichte Kette eines chimären IgG1 oder IgM (kappa Kette)

SEQ ID NO. 68
SEQ ID NO. 69

### chimäre Antikörper

| | |
|---|---|
| cIgG-Panko1 | SEQ ID NO. 64 |
| | SEQ ID NO. 68 |
| | |
| cIgG-Panko2 | SEQ ID NO. 65 |
| | SEQ ID NO. 69 |
| | |
| cIgM-Panko1 | SEQ ID NO. 66 |
| | SEQ ID NO. 68 |
| | |
| cIgM-Pankol | SEQ ID NO. 67 |
| | SEQ ID NO. 69 |

## Patentansprüche

1. Erkennungsmolekül, **dadurch gekennzeichnet, dass** es folgende Aminosäuresequenzen umfasst:
(i) eine Aminosäuresequenz, die eine Homologie von mindestens 60%, vorzugsweise 70%, bevorzugt 80%, ganz besonders bevorzugt 90% zu der Aminosäuresequenz SEQ ID NO:1 oder 2 hat; und
(ii) eine Aminosäuresequenz, die eine Homologie von mindestens 60%, vorzugsweise 70%, bevorzugt 80%, ganz besonders bevorzugt 90% zu der Aminosäuresequenz SEQ ID NO:3 oder 4 hat; und
(iii) eine Aminosäuresequenz, die eine Homologie von mindestens 60%, vorzugsweise 70%, bevorzugt 80%, ganz besonders bevorzugt 90% zu der Aminosäuresequenz SEQ ID NO:5 oder 6 hat,
und
das glykosylierte MUC1-Tumorepitop spezifisch in der Weise bindet, dass die Stärke der Bindung im Vergleich zur Bindung an das nicht-glykosylierte Peptid gleicher Länge und Peptidsequenz mindestens um den Faktor 20 erhöht ist.

2. Erkennungsmolekül nach Anspruch 1, **dadurch gekennzeichnet, dass** es weiterhin folgende Aminosäuresequenzen umfasst:
(i) eine Aminosäuresequenz, die eine Homologie von mindestens 60%, vorzugsweise70%, bevorzugt 80%, ganz besonders bevorzugt 90% zu der Aminosäuresequenz SEQ ID NO:7 oder 8 hat; und
(ii) eine Aminosäuresequenz, die eine Homologie von mindestens 60%, vorzugsweise70%, bevorzugt 80%, ganz besonders bevorzugt 90% zu der Aminosäuresequenz SEQ ID NO:9 oder 10 hat; und
(iii) eine Aminosäuresequenz, die eine Homologie von mindestens 60%, vorzugsweise70%, bevorzugt 80%, ganz besonders bevorzugt 90% zu der Aminosäuresequenz SEQ ID NO:11 oder 12 hat,
und
das glykosylierte MUC1-Tumorepitop spezifisch in der Weise bindet, dass die Stärke der Bindung im Vergleich zur Bindung an das nicht-glykosylierte Peptid gleicher Länge und Peptidsequenz mindestens um den Faktor 20 erhöht ist.

3. Erkennungsmolekül nach Anspruch 1 oder 2, **dadurch gekennzeichnet, dass** mindestens eine Aminosäure mindestens einer Sequenz gemäß SEQ ID NO:1 bis 12 durch eine Aminosäure mit analogen physikochemischen Eigenschaften ersetzt ist und dass das Erkennungsmolekül das glykosylierte MUC1-Tumorepitop spezifisch in der Weise bindet, dass die Stärke der Bindung im Vergleich zur Bindung an das nicht-glykosylierte Peptid gleicher Länge und Peptidsequenz mindestens um den Faktor 20 erhöht ist.

4. Erkennungsmolekül nach einem der Ansprüche 1 bis 3, **dadurch gekennzeichnet, dass** die Sequenz SEQ ID NO:1 oder 2 durch eine äquivalente kanonische Strukturvariante gemäß SEQ ID NOs:13 bis 20 ersetzt ist und/oder mindestens eine Sequenz der Sequenzen SEQ ID NO:3 oder 4 durch eine äquivalente kanonische Strukturvariante gemäß SEQ ID NOs:21 bis 23 ersetzt ist und/oder mindestens eine Sequenz gemäß SEQ ID NO:7 oder 8 durch eine äquivalente kanonische Strukturvariante gemäß SEQ ID NO:24 bis 29 ersetzt ist und/oder mindestens eine Sequenz der Sequenzen SEQ ID NO:11 oder 12 durch eine äquivalente kanonische Strukturvariante gemäß SEQ ID NO:30 oder 31 ersetzt ist und das Erkennungsmolekül das glykosylierte MUC1-Tumorepitop spezifisch in der Weise bindet, dass die Stärke der Bindung im Vergleich zur Bindung an das nicht-glykosylierte Peptid gleicher Länge und Peptidsequenz mindestens um den Faktor 20 erhöht ist.

5. Erkennungsmolekül nach einem der Ansprüche 1 bis 4, **dadurch gekennzeichnet, dass** die variable schwere Kette VH und die variable leichte Kette VL auf verschiedenen Polypeptidketten liegen.

6. Erkennungsmolekül nach einem der Ansprüche 1 bis 4, **dadurch gekennzeichnet, dass** die variable schwere Kette VH und die variable leichte Kette VL miteinander in einem Fusionsprotein direkt verbunden sind.

7. Erkennungsmolekül nach einem der Ansprüche 1 bis 6, **dadurch gekennzeichnet, dass** es ein Immunglobulin der Isotypen IgG, IgM, IgA, IgE, IgD und/oder deren Subklassen umfasst.

8. Erkennungsmolekül, fusioniert, chemisch gekoppelt, kovalent oder nicht-kovalent assoziiert mit einem oder mehreren Antikörpern oder Antikörperfragmenten mit anderer Spezifität, single chain Antikörperfragment, Multibody, Fab-Fragment, Fusionsprotein aus einem Antikörperfragment mit Peptiden oder Proteinen abgeleitet von einem Erkennungsmolekül nach einem der Ansprüche 1 bis 6, wobei das Erkennungsmolekül das glykosylierte MUC1-Tumorepitop spezifisch in der Weise bindet, dass die Stärke der Bindung im Vergleich zur Bindung an das nicht-glykosylierte Peptid gleicher Länge und Peptidsequenz mindestens um den Faktor 20 erhöht ist.

9. Konstrukt, umfassend die Erkennungsmoleküle nach einem der Ansprüche 1 bis 8, **dadurch gekennzeichnet, dass** die Erkennungsmoleküle mit Zusatzsequenzen und/oder Strukturen fusioniert, chemisch gekoppelt oder nicht-kovalent assoziiert sind.

10. Konstrukt nach Anspruch 9, **dadurch gekennzeichnet, dass** die Erkennungsmoleküle mit
(i) Immunglobulindomänen verschiedener Spezies,
(ii) Enzymmolekülen,
(iii) Interaktionsdomänen,
(iv) Domänen zur Stabilisierung,
(v) Signalsequenzen,
(vi) Fluoreszenzfarbstoffen,
(vii) Toxinen,
(viii) katalytischen Antikörpern,
(ix) zytolytischen Komponenten,
(x) Immunmodulatoren,
(xi) Immuneffektoren,
(xii) MHC-Klasse I oder Klassell Antigenen,
(xiii) Chelatoren zur radioaktiven Markierung,
(xiv) Radioisotopen,
(xv) Liposomen,
(xvi) Transmembrandomänen,
(xvii) Viren und/oder
(xviii) Zellen
fusioniert, chemisch gekoppelt, kovalent oder nicht-kovalent assoziiert sind.

11. Nukleinsäuremolekül, umfassend Nukleinsäuresequenzen, die die Aminosäuresequenz von mindestens einem Erkennungsmolekül nach einem der Ansprüche 1 bis 8 oder ein Konstrukt nach Anspruch 9 oder 10 kodiert.

12. Nukleinsäuremolekül nach Anspruch 11, wobei die schwere Kette durch einen ersten Vektor und die leichte Kette eines Erkennungsmoleküls nach einem der Ansprüche 1 bis 8 oder nach einem Konstrukt nach Anspruch 9 oder 10 durch einen zweiten Vektor kodiert wird.

13. Expressionskassette oder Vektor, umfassend ein Nukleinsäuremolekül nach Anspruch 11 oder 12 und einen Promotor, der operativ mit der Nukleinsäure verknüpft ist.

14. Wirtszelle, umfassend mindestens einen Vektor oder eine Expressionskassette nach Anspruch 13.

15. Zusammensetzung umfassend
(i) mindestens ein Erkennungsmolekül nach einem der Ansprüche 1 bis 8;
(ii) mindestens ein Konstrukt nach Anspruch 9 oder 10; und/oder
(iii) mindestens ein Nukleinsäuremolekül nach Anspruch 11 oder 12.

16. Zusammensetzung nach Anspruch 15, **dadurch gekennzeichnet, dass** die Zusammensetzung eine pharmazeutische Zusammensetzung, gegebenenfalls mit einem pharmazeutisch verträglichen Träger und/oder Adjuvans, ist.

## Claims

1. Recognition molecule, **characterized in that** it comprises the following amino acid sequences:
(i) an amino acid sequence having a homology of at least 60% , preferably 70%, more preferably 80%, most preferably 90% to the amino acid sequence SEQ ID NO: 1 or 2; and
(ii) an amino acid sequence having a homology of at least 60% , preferably 70 %, more preferably 80 %, most preferably 90% to the amino acid sequence SEQ ID NO: 3 or 4; and
(iii) an amino acid sequence having a homology of at least 60% , preferably 70 %, more preferably 80 %, most preferably 90% to the amino acid sequence SEQ ID NO: 5 or 6;
and
specifically binds the glycosylated MUC1 tumor epitope such that the strength of the bond is increased by at least a factor of 20 in comparison with the bond to the non-glycosylated peptide of identical length and identical peptide sequence.

2. The recognition molecule according to claim 1, **characterized in that** it further comprises the following amino acid sequences:
(i) an amino acid sequence having a homology of at least 60% , preferably 70%, more preferably 80%, most preferably 90% to the amino acid sequence SEQ ID NO: 7 or 8; and
(ii) an amino acid sequence having a homology of at least 60% , preferably 70%, more preferably 80%, most preferably 90% to the amino acid sequence SEQ ID NO: 9 or 10; and
(iii) an amino acid sequence having a homology of at least 60% , preferably 70%, more preferably 80%, most preferably 90% to the amino acid sequence SEQ ID NO: 11 or 12;
and
specifically binds the glycosylated MUC1 tumor epitope such that the strength of the bond is increased by at least a factor of 20 in comparison with the bond to the non-glycosylated peptide of identical length and identical peptide sequence.

3. The recognition molecule according to claim 1 or 2, **characterized in that** at least one amino acid of at least one sequence of SEQ ID NO: 1 to 12 is substituted by an amino acid with analogue physicochemical properties, and that the recognition molecule specifically binds the glycosylated MUC1 tumor epitope such that the strength of the bond is increased by at least a factor of 20 in comparison with the bond to the non-glycosylated peptide of identical length and identical peptide sequence.

4. The recognition molecule according to any one of claims 1 to 3, **characterized in that** the sequence SEQ ID NO: 1 or 2 is substituted by an equivalent canonical structure variant of SEQ ID NOs: 13 to 20 and/or that at least one sequence of sequences SEQ ID NO: 3 or 4 is substituted by an equivalent canonical structure variant of SEQ ID NOs: 21 to 23 and/or that at least one sequence of SEQ ID NO: 7 or 8 is substituted by an equivalent canonical structure variant of SEQ ID NO: 24 to 29 and/or that at least one sequence of sequences SEQ ID NO: 11 or 12 is substituted by an equivalent canonical structure variant of SEQ ID NO : 30 or 31, and that the recognition molecule specifically binds the glycosylated MUC1 tumor epitope such that the strength of the bond is increased by at least a factor of 20 in comparison with the bond to the non-glycosylated peptide of identical length and identical peptide sequence.

5. The recognition molecule according to any one of claims 1 to 4, **characterized in that** the variable heavy chain VH and the variable light chain VL are on different polypeptide chains.

6. The recognition molecule according to any one of claims 1 to 4, **characterized in that** the variable heavy chain VH and the variable light chain VL are directly linked in a fusion protein.

7. The recognition molecule according to any one of claims 1 to 6, **characterized in that** it comprises an immunoglobulin of the isotypes IgG, IgM, IgA, IgE, IgD and/or subclasses thereof.

8. Recognition molecule that is fused, chemically coupled, covalently or non-covalently associated with one or more antibodies or antibody fragments having a different specificity, single chain antibody fragment, multibody, Fab-fragment, fusion protein of an antibody fragment with peptides or proteins derived from a recognition molecule according to any one of claims 1 to 6, wherein the recognition molecule specifically binds the glycosylated MUC1 tumor epitope such that the strength of the bond is increased by at least a factor of 20 in comparison with the bond to the non-glycosylated peptide of identical length and identical peptide sequence.

9. Construct comprising the recognition molecules according to any one of claims 1 to 8, **characterized in that** the recognition molecules is fused, chemically coupled or non-covalently associated with accessory sequences and/or structures.

10. The construct according to claim 9, **characterized in that** the recognition molecules are fused, chemically coupled, covalently or non-covalently associated with
(i) immunoglobulin domains from different species,
(ii) enzyme molecules
(iii) interaction domains,
(iv) domains for stabilization,
(v) signal sequences,
(vi) fluorescent dyes,
(vii) toxins,
(viii) catalytic antibodies,
(ix) cytolytic components,
(x) immunomodulators,
(xi) immunoeffectors,
(xii) MHC class I or class II antigens,
(xiii) chelating agents for radiolabeling,
(xiv) radioisotopes,
(xv) liposomes,
(xvi) transmembrane domains,
(xvii) viruses and/or
(xviii) cells.

11. Nucleic acid molecule comprising nucleic acid sequences encoding the amino acid sequence of at least one recognition molecule according to any one of claims 1 to 8, or a construct according to claim 9 or 10.

12. Nucleic acid molecule according to claim 11, wherein the heavy chain is encoded by a first vector and the light chain of a recognition molecule according to any one of claims 1 to 8 or a construct according to claim 9 or 10 is encoded by a second vector.

13. Expression cassette or vector comprising a nucleic acid molecule according to claim 11 or 12, and a promoter which is operatively linked to the nucleic acid.

14. A host cell comprising at least one vector or expression cassette according to claim 13.

15. A composition comprising
(i) at least one recognition molecule according to any one of claims 1 to 8;
(ii) at least one construct according to claim 9 or 10, and/or
(iii) at least one nucleic acid molecule according to claim 11 or 12

16. A composition according to claim 15, **characterized in that** the composition is a pharmaceutical composition, optionally with a pharmaceutically acceptable carrier and/or adjuvant.

## Revendications

1. Molécule de reconnaissance, **caractérisée en ce qu'**elle comprend les séquences d'acides aminés suivantes :
(i) une séquence d'acides aminés qui a une homologie d'au moins 60 %, de préférence 70 %, préférablement 80 %, de manière tout particulièrement préférée 90 % à la séquence d'acides aminés SEQ ID N° : 1 ou 2 ; et
(ii) une séquence d'acides aminés qui a une homologie d'au moins 60 %, de préférence 70 %, préférablement 80 %, de manière tout particulièrement préférée 90 % à la séquence d'acides aminés SEQ ID N° : 3 ou 4 ; et
(iii) une séquence d'acides aminés qui a une homologie d'au moins 60 %, de préférence 70 %, préférablement 80 %, de manière tout particulièrement préférée 90 % à la séquence d'acides aminés SEQ ID N° : 5 ou 6,
et
se fixe spécifiquement au déterminant antigénique tumoral MUC1 glycosylé de telle sorte que l'intensité de la fixation par rapport à la fixation au peptide non glycosylé de même longueur et séquence peptidique est augmentée au moins du facteur 20.

2. Molécule de reconnaissance selon la revendication 1, **caractérisée en ce qu'**elle comprend en outre les séquences d'acides aminés suivantes :
(i) une séquence d'acides aminés qui a une homologie d'au moins 60 %, de préférence 70 %, préférablement 80 %, de manière tout particulièrement préférée 90 % à la séquence d'acides aminés SEQ ID N° : 7 ou 8 ; et
(ii) une séquence d'acides aminés qui a une homologie d'au moins 60 %, de préférence 70 %, préférablement 80 %, de manière tout particulièrement préférée 90 % à la séquence d'acides aminés SEQ ID N° : 9 ou 10 ; et
(iii) une séquence d'acides aminés qui a une homologie d'au moins 60 %, de préférence 70 %, préférablement 80 %, de manière tout particulièrement préférée 90 % à la séquence d'acides aminés SEQ ID N° : 11 ou 12,
et
se fixe spécifiquement au déterminant antigénique tumoral MUC1 glycosylé de telle sorte que l'intensité de la fixation par rapport à la fixation au peptide non glycosylé de même longueur et séquence peptidique est augmentée au moins du facteur 20.

3. Molécule de reconnaissance selon la revendication 1 ou 2, **caractérisée en ce qu'**au moins un acide aminé d'au moins une séquence selon SEQ ID N° : 1 à 12 est remplacé par un acide aminé avec des propriétés physicochimiques analogues et que la molécule de reconnaissance se fixe spécifiquement au déterminant antigénique tumoral MUC1 glycosylé de telle sorte que l'intensité de la fixation par rapport à la fixation au peptide non glycosylé de même longueur et séquence peptidique est augmentée au moins du facteur 20.

4. Molécule de reconnaissance selon l'une quelconque des revendications 1 à 3, **caractérisée en ce que** la séquence SEQ ID N° : 1 ou 2 est remplacée par une variante structurelle canonique équivalente selon SEQ ID N° : 13 à 20 et/ou au moins une séquence des séquences SEQ ID N° : 3 ou 4 est remplacée par une variante structurelle canonique équivalente selon SEQ ID N° : 21 à 23 et/ou au moins une séquence selon SEQ ID N° : 7 ou 8 est remplacée par une variante structurelle canonique équivalente selon SEQ ID N° : 24 à 29 et/ou au moins une séquence des séquences SEQ ID N° : 11 ou 12 est remplacée par une variante structurelle canonique équivalente selon SEQ ID N° : 30 ou 31 et la molécule de reconnaissance se fixe spécifiquement au déterminant antigénique tumoral MUC1 glycosylé de telle sorte que l'intensité de la fixation par rapport à la fixation au peptide non glycosylé de même longueur et séquence peptidique est augmentée au moins du facteur 20.

5. Molécule de reconnaissance selon l'une quelconque des revendications 1 à 4, **caractérisée en ce que** la chaîne lourde variable VH et la chaîne légère variable VL se situent sur différentes chaînes polypeptidiques.

6. Molécule de reconnaissance selon l'une quelconque des revendications 1 à 4, **caractérisée en ce que** la chaîne lourde variable VH et la chaîne légère variable VL sont reliées directement ensemble dans une protéine de fusion.

7. Molécule de reconnaissance selon l'une quelconque des revendications 1 à 6, **caractérisée en ce qu'**elle comprend une immunoglobuline des isotypes IgG, IgM, IgA, IgE, IgD et/ou de leurs sous-classes.

8. Molécule de reconnaissance fusionnée, couplée chimiquement, associée par covalence ou non covalence à un ou plusieurs anticorps ou fragments d'anticorps avec une autre spécificité, fragment d'anticorps à chaîne unique, multicorps, fragment Fab, protéine de fusion provenant d'un fragment d'anticorps avec des peptides ou protéines dérivée d'une molécule de reconnaissance selon l'une quelconque des revendications 1 à 6, dans laquelle la molécule de reconnaissance se fixe spécifiquement au déterminant antigénique tumoral MUC1 glycosylé de telle sorte que l'intensité de la fixation par rapport à la fixation au peptide non glycosylé de même longueur et séquence peptidique est augmentée au moins du facteur 20.

9. Hybride comprenant les molécules de reconnaissance selon l'une quelconque des revendications 1 à 8, **caractérisé en ce que** les molécules de reconnaissance sont fusionnées, couplées chimiquement ou associées par non covalence à des séquences supplémentaires et/ou structures.

10. Hybride selon la revendication 9, **caractérisé en ce que** les molécules de reconnaissance sont fusionnées, couplées chimiquement, associées par covalence ou non covalence à
(i) des domaines d'immunoglobuline d'espèce différente,
(ii) des molécules enzymatiques,
(iii) des domaines d'interaction,
(iv) des domaines de stabilisation,
(v) des séquences de signal,
(vi) des colorants fluorescents,
(vii) des toxines,
(viii) des anticorps catalytiques,
(ix) des composants cytolytiques,
(x) des immunomodulateurs,
(xi) des immunoeffecteurs,
(xii) des antigènes du CMH de classe I ou classe II,
(xiii) des chélateurs pour le marquage radioactif,
(xiv) des radio-isotopes,
(xv) des liposomes,
(xvi) des domaines transmembranaires,
(xvii) des virus et/ou
(xviii) des cellules.

11. Molécule d'acide nucléique comprenant des séquences d'acide nucléique qui code pour la séquence d'acides aminés d'au moins une molécule de reconnaissance selon l'une quelconque des revendications 1 à 8 ou un hybride selon la revendication 9 ou 10.

12. Molécule d'acide nucléique selon la revendication 11, dans laquelle la chaîne lourde est codée par un premier vecteur et la chaîne légère d'une molécule de reconnaissance selon l'une quelconque des revendications 1 à 8 ou selon un hybride selon la revendication 9 ou 10 par un second vecteur.

13. Cassette d'expression ou vecteur comprenant une molécule d'acide nucléique selon la revendication 11 ou 12 et un promoteur qui est relié de manière opérationnelle à l'acide nucléique.

14. Cellule souche comprenant au moins un vecteur ou une cassette d'expression selon la revendication 13.

15. Composition comprenant
(i) au moins une molécule de reconnaissance selon l'une quelconque des revendications 1 à 8 ;
(ii) au moins un hybride selon la revendication 9 ou 10 ; et/ou
(iii) au moins une molécule d'acide nucléique selon la revendication 11 ou 12.

16. Composition selon la revendication 15, **caractérisée en ce que** la composition est une composition pharmaceutique, éventuellement avec un support et/ou adjuvant pharmaceutiquement compatible.
